# EUROPEAN PATENT SPECIFICATION

(11) **EP 3 294 323 B1**
(45) Date of publication and mention of the grant of the patent: **13.10.2021**
(21) Application number: 16797013.6
(22) Date of filing: 13.05.2016
(51) Int. Cl.: A61K 38/47, C12N 7/00, C12N 15/86, A61K 31/675, A61K 45/06, A61K 48/00, A61P 25/28

(54) **ADENO-ASSOCIATED VIRUS FOR THERAPEUTIC DELIVERY TO CENTRAL NERVOUS SYSTEM**
ADENO-ASSOZIIERTES VIRUS FÜR DIE FREISETZUNG EINES THERAPEUTIKUMS IM ZENTRALNERVENSYSTEM
VIRUS ADÉNO-ASSOCIÉ POUR UNE ADMINISTRATION THÉRAPEUTIQUE AU SYSTÈME NERVEUX CENTRAL

(30) Priority: 15.05.2015 US 201562162174 P; 06.11.2015 US 201562252055 P; 01.03.2016 US 201662301980 P; 03.05.2016 US 201662331156 P
(43) Date of publication of application: 21.03.2018
(73) Proprietor: McIvor, R. Scott, St. Louis Park, MN 55416 (US); Belur, Lalitha R., St. Paul, MN 55116 (US); Kozarsky, Karen, Bala Cynwyd, PA 19004 (US)
(72) Inventor: McIvor, R. Scott, St. Louis Park, MN 55416 (US); Belur, Lalitha R., St. Paul, MN 55116 (US); Kozarsky, Karen, Bala Cynwyd, PA 19004 (US)
(74) Representative: Forresters IP LLP
(86) International application number: PCT/US2016/032392
(87) International publication number: WO 2016/187017

(56) References cited:
- WO-A1-2014/186579
- WO-A2-2015/013148
- US-A1- 2012 009 268
- US-A1- 2012 288 489
- US-A1- 2013 039 888
- US-A1- 2014 088 179
- FU ET AL.: 'Neurological correction of lysosomal storage in a mucopolysaccharidosis IIIB mouse model by adeno-associated virus-mediated gene delivery' MOLECULAR THERAPY vol. 5, no. 1, 01 January 2002, pages 42 - 49, XP055117423

## Description

### Background

The mucopolysaccharidoses (MPSs) are a group of 11 storage diseases caused by disruptions in glycosaminoglycan (GAG) catabolism, leading to their accumulation in lysosomes (Muenzer, 2004; Munoz-Rojas et al., 2008). Manifestations of varying severity include organomegaly, skeletal dysplasias, cardiac and pulmonary obstruction and neurological deterioration. For MPS I, deficiency of iduronidase (IDUA), severity ranges from mild (Scheie syndrome) to moderate (Hurler-Scheie) to severe (Hurler syndrome), with the latter resulting in neurologic deficiency and death by age 15 (Muenzer, 2004; Munoz-Rojas et al., 2008). Therapies for MPSs have been for the most part palliative. However, there are some of the MPS diseases, including Hurler syndrome, for which allogeneic hematopoietic stem cell transplantation (HSCT) has exhibited efficacy (Krivit, 2004; Orchard et al., 2007; Peters et al., 2003). Additionally, for more and more of the MPS diseases, enzyme replacement therapy (ERT) is becoming available (Brady, 2006). In general, HSCT and ERT result in the clearing of storage materials and improved peripheral conditions, although some problems persist after treatment (skeletal, cardiac, corneal clouding). The primary challenge in these cellular and enzyme therapies is effectiveness in addressing neurological manifestations, as peripherally administered enzyme does not penetrate the blood-brain barrier and HSCT has been found to be of benefit for some, but not all, MPS's.

MPS I has been one of the most extensively studied of the MPS diseases for development of cellular and molecular therapies. The effectiveness of allogeneic HSCT is most likely the result of metabolic cross-correction, whereby the missing enzyme is released from donor-derived cells and subsequently taken up by host cells and trafficked to lysosomes, where the enzyme contributes to lysosomal metabolism (Fratantoni et al., 1968). Clearing of GAG storage materials is subsequently observed in peripheral organs such as liver and spleen, there is relief from cardiopulmonary obstruction and improvement in corneal clouding (Orchard et al., 2007). Of particular importance is the effect of allogeneic stem cell transplantation on the emergence of neurologic manifestations in the MPS diseases. In this regard, there is evidence for several MPS diseases that individuals engrafted with allogeneic stem cells face an improved outcome in comparison with untransplanted patients (Bjoraker et al., 2006; Krivit, 2004; Orchard et al., 2007; Peters et al., 2003). A central hypothesis explaining the neurologic benefit of allogeneic hematopoietic stem cell transplant is the penetration of donor-derived hematopoietic cells (most likely microglia) (Hess et al., 2004; Unger et al., 1993) into the central nervous system, where the missing enzyme is expressed by engrafted cells from which point the enzyme diffuses into CNS tissues and participates in clearing of storage materials. The level of enzyme provided to CNS tissues is thus limited to that amount expressed and released from donor-derived cells engrafting in the brain. While such engraftment is of great benefit for MPS I, recipients nonetheless continue to exhibit below normal IQ and impaired neurocognitive capability (Ziegler and Shapiro, 2007).

The phenomenon of metabolic cross correction also explains the effectiveness of ERT for several lysosomal storage diseases (Brady, 2006), most notably MPS I. However, due to the requirement for penetration of the blood-brain barrier (BBB) by the enzyme missing in the particular lysosomal storage disease (LSD) in order to effectively reach the CNS, effectiveness of enzyme therapy for neurologic manifestations of lysosomal storage disease (LSD) has not been observed (Brady, 2006). Enzymes are almost always too large and generally too charged to effectively cross the BBB. This has prompted investigations into invasive intrathecal enzyme administration (Dickson et al., 2007), for which effectiveness has been demonstrated in a canine model of MPS I (Kakkis et al., 2004) and for which human clinical trials are beginning for MPS I (Pastores, 2008; Munoz-Rojas et al., 2008). Key disadvantages of enzyme therapy include its great expense (> $200,000 per year) and the requirement for repeated infusions of recombinant protein. Current clinical trials of intrathecal IDUA administration are designed to inject the enzyme only once every three months, so the effectiveness of this dosing regimen remains uncertain. WO2014/186579 also discloses the idea to use AAV9 as a vector for treating different types of lysosomal storage diseases. However, WO 2014/186579 does not mention neurocognition in the context of gene therapy.

### Summary of the Invention

The invention is defined in the claims.

The AAV vectors employed in the methods of the invention are useful to deliver genes to the CNS. In one embodiment, the invention provides for intranasal delivery to the CNS of therapeutic proteins via AAV, e.g., to prevent, inhibit or treat neurocognitive dysfunction or neurological disease. As described herein, the intranasal delivery of the vector led to transduction of the forebrain (olfactory bulb) and expression of therapeutic protein. The protein diffused to all areas of the brain. Thus, the use of intranasal delivery AAV vectors to express, e.g., a secreted protein, allows for the treatment of many different neurologic disorders, e.g., MPS I, MPS II, MP SIII, other metabolic diseases, including Parkinson's disease and Alzheimer's disease, and the like. For example, assay of extracts from all microdissected parts of the brain shows widespread distribution throughout the brain of alpha-L-iduronidase delivered by the rAAV.

In one embodiment, rAAV is delivered to a mammal intrathecally (IT), endovascularly (IV), cerebroventricularly (ICV) or intranasally (IN) to prevent, inhibit or treat neurocognitive dysfunction or neurological disease. In one embodiment, the intranasal administration results in non-invasive direct administration to CNS with metabolic cross-correction. In one embodiment, the mammal is subjected to immunosuppression. In one embodiment, the mammal is subjected to tolerization.

In one aspect of the disclosure, the disease to be prevented, inhibited or treated with a particular gene includes, MPS I (IDUA), MPS II (IDS), MPS IIIA (Heparan-N-sulfatase;sulfaminidase), MPS IIIB (alpha-N-acetyl-glucosaminidase), MPS IIIC (Acetyl-CoA:alpha -N-acetyl-glucosaminide acetyltransferase), MPS IIID (N-acetylglucosamine 6-sulfatase), MPS VII (beta-glucoronidase), Gaucher (acid beta-glucosidase), Alpha-mannosidosis (alpha-mannosidase), Beta-mannosidosis (beta-mannosidase), Alpha-fucosidosis (alpha-fucosidase), Sialidosis (alpha-sialidase) , Galactosialidosis (Cathepsin A), Aspartylglucosaminuria (aspartylglucosaminidase), GM1-gangliosidosis (beta-galactosidase), Tay-Sachs (beta-hexosaminidase subunit alpha), Sandhoff (beta-hexosaminidase subunit beta), GM2-gangliosidosis/variant AB (GM2 activator protein), Krabbe (galactocerebrosidase), Metachromatic leukodystrophy (arylsulfatase A), and other neurologic disorders including Alzheimer's disease (expression of an antibody, such as an antibody to beta-amyloid, or an enzyme that attacks the plaques and fibrils associated with Alzheimer's), or Alzheimer's and Parkinson's diseases (expression of neuroprotective proteins including GDNF or Neurturin).

Thus, methods of preventing, inhibiting, and/or treating, for example one or more symptoms associated with, a disease of the central nervous system (CNS) in a mammal in need thereof are described. The methods involve delivering to the CNS of a mammal in need of treatment a composition comprising an effective amount of a recombinant adeno-associated virus (rAAV) vector comprising an open reading frame encoding a gene product, e.g., a therapeutic gene product. Target gene products that may be encoded by an rAAV vector include, alpha-L-iduronidase, iduronate-2-sulfatase, heparan sulfate sulfatase, N-acetyl-alpha-D-glucosaminidase, beta-hexosaminidase, alpha-galactosidase, beta-galactosidase, beta-glucuronidase or glucocerebrosidase, as well as those disclosed hereinabove. Diseases that may be prevented, inhibited or treated using the methods disclosed herein include, mucopolysaccharidosis type I disorder, a mucopolysaccharidosis type II disorder, or a mucopolysaccharidosis type VII disorder, as well as the disorders listed above. The AAV vector can be administered in a variety of ways to ensure that it is delivered to the CNS/brain, and that the transgene is successfully transduced in the subject's CNS/brain. Routes of delivery to the CNS/brain include intrathecal administration, intracranial administration, e.g., intracerebroventricular administration, or lateral cerebroventricular administration, intranasal administration, endovascular administration, and intraparenchymal administration.

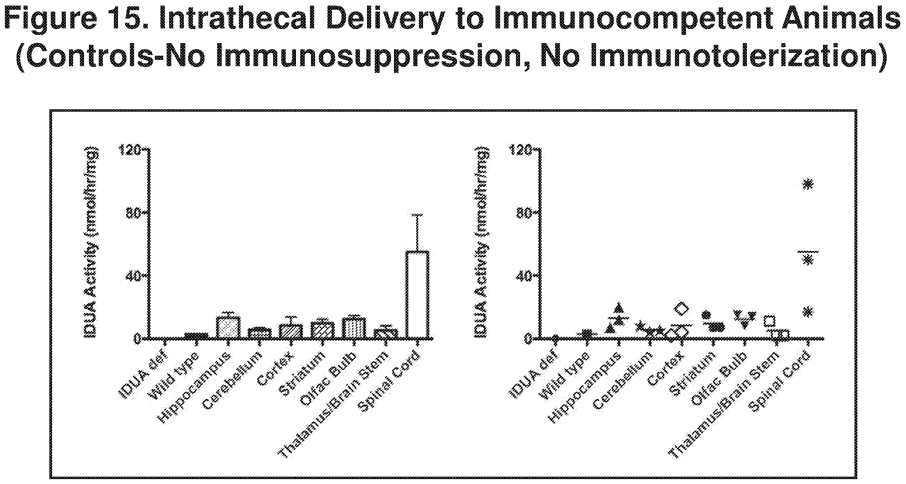

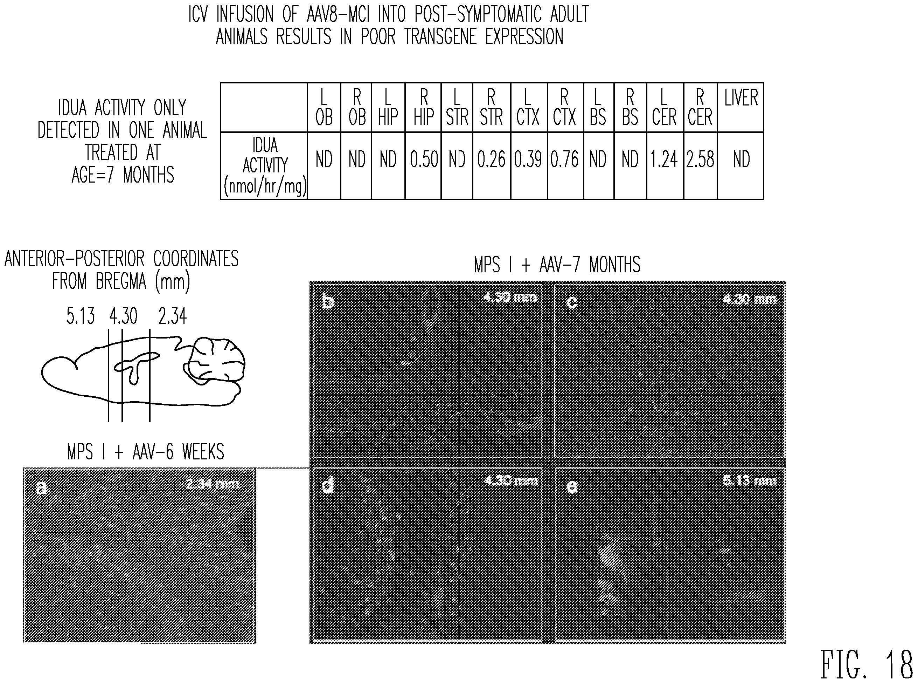

In one aspect of the disclosure, the methods involve delivering to the CNS of an adult mammal in need of treatment a composition comprising an effective amount of a rAAV serotype 9 (rAAV9) vector comprising an open reading frame encoding a gene. In one aspect of the disclosure, the methods involve delivering to the CNS of an adult mammal in need of treatment a composition comprising an effective amount of a rAAV9 vector comprising an open reading frame encoding IDUA. These methods are based, in part, on the discovery that an AAV9 vector can efficiently transduce the therapeutic transgene in the brain/CNS of adult subjects, restoring enzyme levels to wild type levels (see Figure 15, *infra*). The results achieved using AAV9 are surprising in view of previous work which demonstrated that intravascular delivery of AAV9 in adult mice does not achieve widespread direct neuronal targeting (see Foust et al., 2009), as well as additional data showing that direct injection of AAV8-IDUA into the CNS of adult IDUA-deficient mice resulted in poor transgene expression (Figure 18). The examples described herein use a pre-clinical model for the treatment of MPS1, an inherited metabolic disorder caused by deficiency of the lysosomal enzyme alpha-L-iduronidase (IDUA). The examples demonstrate that direct application of AAV9-IDUA into the CNS of immunocompetent adult IDUA-deficient mice resulted in IDUA enzyme expression and activity that is the same or higher than IDUA enzyme expression and activity in wild-type adult mice (see Figure 15, *infra*).

The examples also demonstrate that co-therapy to induce immunosuppression or immunotolerization, or treatment of immunodeficient animals, can achieve even higher levels of IDUA enzyme expression and activity. Patients with genotypes that promote an immune response that neutralizes enzyme activity (see, e.g., Barbier et al., 2013) are treated with an immunosuppressant in addition to the rAAV vector comprising an open reading frame encoding a gene product, such as IDUA.

Neonatal IDUA^{-/-} mice are immunologically naïve. Administration of AAV8-IDUA to neonatal IDUA^{- /-}mice resulted in IDUA expression (Wolf et al., 2011), thus tolerizing the animals to IDUA. As described herein, the applicability of AAV-mediated gene transfer to adult (immunocompetent) mice by direct infusion of AAV to the central nervous system was shown using different routes of administration. For example, AAV9-IDUA was administered by direct injection into the lateral ventricles of adult IDUA-deficient mice that were either immunocompetent, immunodeficient (NODSCID/IDUA-/-), immunosuppressed with cyclophosphamide (CP), or immunotolerized by weekly injection of human iduronidase protein (Aldurazyme) starting at birth. CP immunosuppressed animals were also administered AAV9-IDUA by intranasal infusion, by intrathecal injection, and by endovascular infusion with and without mannitol to disrupt the blood-brain barrier. Animals were sacrificed at 8 weeks after vector administration, and brains were harvested and microdissected for evaluation of IDUA enzymatic activity, tissue glycosaminoglycans, and IDUA vector sequences in comparison with normal and affected control mice. Results from these studies show that numerous routes for AAV vector administration directly to the CNS may be employed, e.g., so as to achieve higher levels of protein delivery and/or enzyme activity in the CNS. In addition, although the brain is an immunoprivileged site, administration of an immunosuppressant or immunotolerization may increase the activity found in the brain after AAV administration. Higher levels of expression per administration and/or less invasive routes of administration are clinically more palatable to patients.

Thus, the invention includes the use of recombinant AAV (rAAV) vectors that encode a gene product with therapeutic effects when expressed in the CNS of a mammal. The mammal is an immunocompetent mammal with a disease or disorder of the CNS (a neurologic disease). An "immunocompetent" mammal as used herein is a mammal of an age where both cellular and humoral immune responses are elicited after exposure to an antigenic stimulus, by upregulation of Th1 functions or IFN-γ production in response to polyclonal stimuli, in contrast to a neonate which has innate immunity and immunity derived from the mother, e.g., during gestation or via lactation. An adult mammal that does not have an immunodeficiency disease is an example of an immunocompetent mammal. For example, an immunocompetent human is typically at least 1, 2, 3, 4, 5 or 6 months of age, and includes adult humans without an immunodeficiency disease. In one embodiment, the AAV is administered intrathecally. In one embodiment, the AAV is administered intracranially (e.g., intracerebroventricularly). In one embodiment, the AAV is administered intranasally, with or without a permeation enhancer. In one embodiment, the AAV is administered endovascularly, e.g., carotid artery administration, with or without a permeation enhancer. In one embodiment, the mammal that is administered the AAV is immunodeficient or is subjected to immunotolerization or immune suppression, e.g., to induce higher levels of therapeutic protein expression relative to a corresponding mammal that is administered the AAV but not subjected to immunotolerization or immune suppression. In one embodiment, an immune suppressive agent is administered to induce immune suppression. In one embodiment, the mammal that is administered the AAV is not subjected to immunotolerization or immune suppression (e.g., administration of the AAV alone provides for the therapeutic effect).

In one embodiment, the invention provides a method to augment secreted protein in the central nervous system of a mammal having neurological disease, which may include a neurocognitive dysfunction. The method includes intranasally administering to the mammal a composition comprising an effective amount of a recombinant adeno-associated virus (rAAV) vector comprising an open reading frame encoding the secreted protein, the expression of which in the mammal decreases neuropathology and/or enhances neurocognition throughout the brain relative to a mammal with the disease or dysfunction but not administered the rAAV. In one embodiment, the encoded protein comprises a neuroprotective protein, e.g., GDNF or Neurturin. In one embodiment, the encoded protein comprises an antibody, e.g., one that binds beta-amyloid. In one embodiment, the protein is an enzyme that cleaves plaque or fibrils associated with Alzheimer's disease. In one embodiment, the mammal is not treated with an immunosuppressant. In another embodiment, for example, in subjects that may generate an immune response that neutralizes activity of the therapeutic protein, the mammal is treated with an immunosuppressant, e.g., a glucocorticoid, cytostatic agents including an alkylating agent, an anti-metabolite, a cytotoxic antibiotic, an antibody, or an agent active on immunophilin, such as a nitrogen mustard, nitrosourea, platinum compound, methotrexate, azathioprine, mercaptopurine, fluorouracil, dactinomycin, an anthracycline, mitomycin C, bleomycin, mithramycin, IL-2 receptor- (CD25-) or CD3-directed antibodies, anti-IL-2 antibodies, ciclosporin, tacrolimus, sirolimus, IFN-β, IFN-γ, an opioid, or TNF-α (tumor necrosis factor-alpha) binding agent. In one embodiment, the rAAV and the immune suppressant are co-administered or the immune suppressant is administered after the rAAV. In one embodiment, the immune suppressant is intrathecally administered. In one embodiment, the immune suppressant is intracerebroventricularly administered. In one aspect of the disclosure, the rAAV vector is a rAAV1, rAAV3, rAAV4, rAAV5, rAA rh10, or rAAV9 vector. In one embodiment, prior to administration of the composition the mammal is immunotolerized.

In one embodiment, the invention provides a method to prevent, inhibit or treat neurological disease, which may include neurocognitive dysfunction in a mammal. The method includes intranasally administering to the mammal a composition comprising an effective amount of a recombinant adeno-associated virus (rAAV) vector comprising an open reading frame encoding a protein, the expression of which in the mammal prevents, inhibits or treats neuropathology and/or neurocognitive dysfunction. In one embodiment, the encoded protein comprises a neuroprotective protein, e.g., GDNF or Neurturin. In one embodiment, the encoded protein comprises an antibody, e.g., one that binds beta-amyloid. In one embodiment, the protein is an enzyme that cleaves plaque or fibrils associated with Alzheimer's disease. In one embodiment, the mammal is not treated with an immunosuppressant. In another embodiment, for example, in subjects that may generate an immune response that neutralizes activity of the therapeutic protein, the mammal is treated with an immunosuppressant, e.g., a glucocorticoid, cytostatic agents including an alkylating agent, an anti-metabolite, a cytotoxic antibiotic, an antibody, or an agent active on immunophilin, such as a nitrogen mustard, nitrosourea, platinum compound, methotrexate, azathioprine, mercaptopurine, fluorouracil, dactinomycin, an anthracycline, mitomycin C, bleomycin, mithramycin, IL-2 receptor- (CD25-) or CD3-directed antibodies, anti-IL-2 antibodies, ciclosporin, tacrolimus, sirolimus, IFN-β, IFN-γ, an opioid, or TNF-α (tumor necrosis factor-alpha) binding agent. In one embodiment, the rAAV and the immune suppressant are co-administered or the immune suppressant is administered after the rAAV. In one embodiment, the immune suppressant is intrathecally administered. In one embodiment, the immune suppressant is intracerebroventricularly administered. In one aspect of the disclosure, the rAAV vector is a rAAV1, rAAV3, rAAV4, rAAV5, rAAVrh10, or rAAV9 vector. In one embodiment, prior to administration of the composition the mammal is immunotolerized. In one aspect of the disclosure, the mammal has Alzheimer's disease or Parkinson's disease.

In one aspect of the disclosure, the invention provides a method to provide for cross-correction of a secreted protein in the central nervous system in a mammal having a neurological disease, which may include neurocognitive dysfunction. The method includes: intranasally, intrathecally, intracerebrovascularly or intravenously administering to the mammal an effective amount of a composition comprising an effective amount of a recombinant adeno-associated virus (rAAV) vector comprising an open reading frame encoding the secreted protein, the expression of which in the mammal provides for cross-correction. In one aspect of the disclosure, the encoded protein comprises a neuroprotective protein, e.g., GDNF or Neurturin. In one aspect of the disclosure, the encoded protein comprises an antibody, e.g., one that binds beta-amyloid. In one aspect of the disclosure, the protein is an enzyme that cleaves plaque or fibrils associated with Alzheimer's disease. In one aspect of the disclosure, the mammal is not treated with an immunosuppressant. In one aspect of the disclosure, for example, in subjects that may generate an immune response that neutralizes activity of the therapeutic protein, the mammal is treated with an immunosuppressant, e.g., a glucocorticoid, cytostatic agents including an alkylating agent, an anti-metabolite, a cytotoxic antibiotic, an antibody, or an agent active on immunophilin, such as a nitrogen mustard, nitrosourea, platinum compound, methotrexate, azathioprine, mercaptopurine, fluorouracil, dactinomycin, an anthracycline, mitomycin C, bleomycin, mithramycin, IL-2 receptor- (CD25-) or CD3-directed antibodies, anti-IL-2 antibodies, ciclosporin, tacrolimus, sirolimus, IFN-β, IFN-γ, an opioid, or TNF-α (tumor necrosis factor-alpha) binding agent. In one aspect of the disclosure, the rAAV and the immune suppressant are co-administered or the immune suppressant is administered after the rAAV. In one aspect of the disclosure, the immune suppressant is intrathecally administered. In one aspect of the disclosure, the immune suppressant is intracerebroventricularly administered. In one aspect of the disclosure, the rAAV vector is a rAAV1, rAAV3, rAAV4, rAAV5, rAAVrh10, or rAAV9 vector. In one aspect of the disclosure, prior to administration of the composition the mammal is immunotolerized.

The invention provides a method to prevent, inhibit or treat neurocognitive dysfunction associated with a disease or disorder of the central nervous system in a mammal in need thereof. The method includes intrathecally, e.g., to the lumbar region, or intracerebroventricularly, e.g., to the lateral ventricle, administering to the mammal a composition comprising an effective amount of a rAAV vector comprising an open reading frame encoding a gene product, the expression of which in the central nervous system of the mammal prevents, inhibits or treats the neurocognitive dysfunction. In one aspect of the disclosure, the gene product is a lysosomal storage enzyme. In one aspect of the disclosure, the mammal is an immunocompetent adult. In one aspect of the disclosure, the rAAV vector is an AAV1, AAV2, AAV3, AAV4, AAV5, AAV6, AAV7, AAV8, AAVrh10, or AAV9 vector. In one aspect of the disclosure, the mammal is a human. In one aspect of the disclosure, multiple doses are administered. In one aspect of the disclosure, the composition is administered weekly, monthly or two or more months apart.

In one aspect of the disclosure, the method includes intrathecally, e.g., to the lumbar region, administering to a mammal a composition comprising an effective amount of a rAAV vector comprising an open reading frame encoding a gene product, the expression of which in the central nervous system of the mammal prevents, inhibits or treats neurocognitive dysfunction, and optionally administering a permeation enhancer. In one aspect of the disclosure, the permeation enhancer is administered before the composition. In one aspect of the disclosure, the composition comprises a permeation enhancer. In one aspect of the disclosure, the permeation enhancer is administered after the composition. In one aspect of the disclosure, the gene product is a lysosomal storage enzyme. In one aspect of the disclosure, the mammal is an immunocompetent adult. In one aspect of the disclosure, the rAAV vector is an AAV-1, AAV-3, AAV-4, AAV-5, AAV-6, AAV-7, AAV-8, AAV rh10, or AAV-9 vector. In one aspect of the disclosure, the mammal is a human. In one aspect of the disclosure, multiple doses are administered. In one aspect of the disclosure, the composition is administered weekly, monthly or two or more months apart. In one aspect of the disclosure, the mammal that is intrathecally administered the AAV is not subjected to immunotolerization or immune suppression (e.g., administration of the AAV alone provides for the therapeutic effect). In one aspect of the disclosure, the mammal that is intrathecally administered the AAV is immunodeficient or is subjected to immunotolerization or immune suppression, e.g., to induce higher levels of therapeutic protein expression relative to a corresponding mammal that is intrathecally administered the AAV but not subjected to immunotolerization or immune suppression.

In one aspect of the disclosure, the method includes intracerebroventricularly, e.g., to the lateral ventricle, administering to an immunocompetent mammal a composition comprising an effective amount of a rAAV vector comprising an open reading frame encoding a gene product, the expression of which in the central nervous system of the mammal prevents, inhibits or treats neurocognitive dysfunction. In one aspect of the disclosure, the gene product is a lysosomal storage enzyme. In one aspect of the disclosure, the rAAV vector is an AAV1, AAV2, AAV3, AAV4, AAV5, AAV6, AAV7, AAV8, AAVrh 10, or AAV9 vector. In one aspect of the disclosure, the rAAV vector is not a rAAV5 vector. In one aspect of the disclosure, the mammal is a human. In one aspect of the disclosure, multiple doses are administered. In one aspect of the disclosure, the composition is administered weekly, monthly or two or more months apart. In one aspect of the disclosure, the mammal that is intracerebroventricularly administered the AAV is not subjected to immunotolerization or immune suppression (e.g., administration of the AAV alone provides for the therapeutic effect). In one aspect of the disclosure, the mammal that is intracerebroventricularly administered the AAV is immunodeficient or is subjected to immunotolerization or immune suppression, e.g., to induce higher levels of therapeutic protein expression relative to a corresponding mammal that is intracerebroventricularly administered the AAV but not subjected to immunotolerization or immune suppression In one aspect of the disclosure, the mammal is immunotolerized to the gene product before the composition comprising the AAV is administered.

Further provided is a method to prevent, inhibit or treat neurocognitive dysfunction associated with a disease or disorder of the central nervous system in a mammal in need thereof. The method includes endovascularly administering to the mammal a composition comprising an effective amount of a rAAV vector comprising an open reading frame encoding a gene product, the expression of which in the central nervous system of the mammal prevents, inhibits or treats the dysfunction, and an effective amount of a permeation enhancer. In one aspect of the disclosure, the composition comprises the permeation enhancer. In one aspect of the disclosure, the permeation enhancer comprises mannitol, sodium glycocholate, sodium taurocholate, sodium deoxycholate, sodium salicylate, sodium caprylate, sodium caprate, sodium lauryl sulfate, polyoxyethylene-9-laurel ether, or EDTA. In one aspect of the disclosure, the gene product is a lysosomal storage enzyme. In one aspect of the disclosure, the mammal is an immunocompetent adult. In one aspect of the disclosure, the rAAV vector is an AAV1, AAV2, AAV3, AAV4, AAV5, AAV6, AAV7, AAV8, AAVrh10, or AAV9 vector. In one aspect of the disclosure, the rAAV vector is not a rAAV5 vector. In one aspect of the disclosure, the mammal is a human. In one aspect of the disclosure, multiple doses are administered. In one aspect of the disclosure, the composition is administered weekly. In one aspect of the disclosure, the composition is administered weekly, monthly or two or more months apart. In one aspect of the disclosure, the mammal that is endovascularly administered the AAV is not subjected to immunotolerization or immune suppression (e.g., administration of the AAV provides for the therapeutic effect). In one aspect of the disclosure, the mammal that is endovascularly administered the AAV is immunodeficient or is subjected to immunotolerization or immune suppression, e.g., to induce higher levels of therapeutic protein expression relative to a corresponding mammal that is endovascularly administered the AAV but not subjected to immunotolerization or immune suppression.

In one aspect of the disclosure, the method includes intranasally administering to a mammal a composition comprising an effective amount of a rAAV9 vector comprising an open reading frame encoding a gene product, the expression of which in the central nervous system of the mammal prevents, inhibits or treats neurocognitive dysfunction, and optionally administering a permeation enhancer. In one aspect of the disclosure, intranasal delivery may be accomplished as described in U.S. Patent No. 8,609,088, the disclosure of which is incorporated by reference herein. In one aspect of the disclosure, the permeation enhancer is administered before the composition. In one aspect of the disclosure, the composition comprises a permeation enhancer. In one aspect of the disclosure, the permeation enhancer is administered after the composition. In one aspect of the disclosure, the gene product is a lysosomal storage enzyme. In one aspect of the disclosure, the mammal is an immunocompetent adult. In one aspect of the disclosure, the mammal is a human. In one aspect of the disclosure, multiple doses are administered. In one aspect of the disclosure, the composition is administered weekly, monthly or two or more months apart. In one aspect of the disclosure, the mammal that is intranasally administered the AAV is not subjected to immunotolerization or immune suppression. In one aspect of the disclosure, the mammal that is intranasally administered the AAV is subjected to immunotolerization or immune suppression, e.g., to induce higher levels of IDUA protein expression relative to a corresponding mammal that is intranasallly administered the AAV but not subjected to immunotolerization or immune suppression.

Also provided is a method to prevent, inhibit or treat neurocognitive dysfunction associated with a disease of the central nervous system in a mammal in need thereof. The method includes administering to the mammal a composition comprising an effective amount of a rAAV vector comprising an open reading frame encoding a gene product, the expression of which in the central nervous system of the mammal prevents, inhibits or treats, and an immune suppressant. In one embodiment, the immune suppressant comprises cyclophosphamide. In one embodiment, the immune suppressant comprises a glucocorticoid, cytostatic agents including an alkylating agent or an anti-metabolite such as methotrexate, azathioprine, mercaptopurine or a cytotoxic antibiotic, an antibody, or an agent active on immunophilin. In one embodiment, the immune suppressant comprises a nitrogen mustard, nitrosourea, a platinum compound, methotrexate, azathioprine, mercaptopurine, fluorouracil, dactinomycin, an anthracyclin, mitomycin C, bleomycin, mithramycin, IL2-receptor- (CD25-) or CD3-directed antibodies, anti-IL-2 antibodies, cyclosporin, tacrolimus, sirolimus, IFN-β, IFN-γ, an opioid, or TNF-α (tumor necrosis factor-alpha) binding agents such as infliximab (Remicade), etanercept (Enbrel), or adalimumab (Humira). In one embodiment, the rAAV and the immune suppressant are co-administered. In one embodiment, the rAAV is administered before and optionally after the immune suppressant. In one embodiment, the immune suppressant is administered before the rAAV. In one embodiment, the rAAV and the immune suppressant are intrathecally administered. In one embodiment, the rAAV and the immune suppressant are intracerebroventricularly administered. In one embodiment, the rAAV is intrathecally administered and the immune suppressant is intravenously administered. In one aspect of the disclosure, the gene product is a lysosomal storage enzyme. In one embodiment, the mammal is an adult. In one aspect of the disclosure, the rAAV vector is an AAV1, AAV2, AAV3, AAV4, AAV5, AAV6, AAV7, AAV8, AAVrh10, or AAV9 vector. In one embodiment, the mammal is a human. In one embodiment, multiple doses are administered. In one embodiment, the composition is administered weekly. In one embodiment, the composition is administered weekly, monthly or two or more months apart.

The invention also provides a method to prevent, inhibit or treat neurocognitive dysfunction associated with a disease of the central nervous system in a mammal in need thereof. A mammal immunotolerized to a gene product that is associated with the disease is administered a composition comprising an effective amount of a rAAV vector comprising an open reading frame encoding a gene product, the expression of which in the central nervous system of the mammal prevents, inhibits or treats the one or more symptoms. In one aspect of the disclosure, the gene product is a lysosomal storage enzyme. In one aspect of the disclosure, the mammal is an adult. In one aspect of the disclosure, the rAAV vector is an AAV1, AAV2, AAV3, AAV4, AAV5, AAV6, AAV7, AAV8, AAVrh10, or AAV9 vector. In one aspect of the disclosure, the mammal is a human. In one aspect of the disclosure, multiple doses are administered. In one aspect of the disclosure, the composition is administered weekly.

Gene products that may be encoded by rAAV vectors include, alpha-L-iduronidase, iduronate-2-sulfatase, heparan sulfate sulfatase, N-acetyl-alpha-D-glucosaminidase, beta-hexosaminidase, alpha-galactosidase, betagalactosidase, beta-glucuronidase, glucocerebrosidase, fibroblast growth factor-2 (FGF-2), brain derived growth factor (BDGF), neurturin, glial derived growth factor (GDGF), tyrosine hydroxylase, dopamine decarboxylase, or glutamic acid decarboxylase.

Diseases that may exhibit neurologic symptoms or neurocognitive dysfunction that may be prevented, inhibited or treated using the methods disclosed herein include, Adrenoleukodystrophy, Alzheimer disease, Amyotrophic lateral sclerosis, Angelman syndrome, Ataxia telangiectasia, Charcot-Marie-Tooth syndrome, Cockayne syndrome, Deafness, Duchenne muscular dystrophy, Epilepsy, Essential tremor, Fragile X syndrome, Friedreich's ataxia, Gaucher disease, Huntington disease, Lesch-Nyhan syndrome, Maple syrup urine disease, Menkes syndrome, Myotonic dystrophy, Narcolepsy, Neurofibromatosis, Niemann-Pick disease, Parkinson disease, Phenylketonuria, Prader-Willi syndrome, Refsum disease, Rett syndrome, Spinal muscular atrophy, Spinocerebellar ataxia, Tangier disease, Tay-Sachs disease, Tuberous sclerosis, Von Hippel-Lindau syndrome, Williams syndrome, Wilson's disease, or Zellweger syndrome. In one aspect of the disclosure, the disease is a lysosomal storage disease, e.g., a lack or deficiency in a lysosomal storage enzyme. Lysosomal storage diseases include, mucopolysaccharidosis (MPS) diseases, for instance, mucopolysaccharidosis type I, e.g., Hurler syndrome and the variants Scheie syndrome and Hurler-Scheie syndrome (a deficiency in alpha-L-iduronidase); Hunter syndrome (a deficiency of iduronate-2-sulfatase); mucopolysaccharidosis type III, e.g., Sanfilippo syndrome (A, B, C or D; a deficiency of heparan sulfate sulfatase, N-acetyl-alpha-D-glucosaminidase, acetyl CoA:alpha-glucosaminide N-acetyl transferase or N-acetylglucosamine-6-sulfate sulfatase); mucopolysaccharidosis type IV, e.g., Morquio syndrome (a deficiency of galactosamine-6-sulfate sulfatase or beta-galactosidase); mucopolysaccharidosis type VI, e.g., Maroteaux-Lamy syndrome (a deficiency of arylsulfatase B); mucopolysaccharidosis type II; mucopolysaccharidosis type III (A, B, C or D; a deficiency of heparan sulfate sulfatase, N-acetyl-alpha-D-glucosaminidase, acetyl CoA:alpha-glucosaminide N-acetyl transferase or N-acetylglucosamine-6-sulfate sulfatase); mucopolysaccharidosis type IV (A or B; a deficiency of galactosamine-6-sulfatase and beta-galatacosidase); mucopolysaccharidosis type VI (a deficiency of arylsulfatase B); mucopolysaccharidosis type VII (a deficiency in beta-glucuronidase); mucopolysaccharidosis type VIII (a deficiency of glucosamine-6-sulfate sulfatase); mucopolysaccharidosis type IX (a deficiency of hyaluronidase); Tay-Sachs disease (a deficiency in alpha subunit of beta-hexosaminidase); Sandhoff disease (a deficiency in both alpha and beta subunit of beta-hexosaminidase); GM1 gangliosidosis (type I or type II); Fabry disease (a deficiency in alpha galactosidase); metachromatic leukodystrophy (a deficiency of aryl sulfatase A); Pompe disease (a deficiency of acid maltase); fucosidosis (a deficiency of fucosidase); alpha-mannosidosis (a deficiency of alpha-mannosidase); beta-mannosidosis (a deficiency of beta-mannosidase), ceroid lipofuscinosis, and Gaucher disease (types I, II and III; a deficiency in glucocerebrosidase), as well as disorders such as Hermansky-Pudlak syndrome; Amaurotic idiocy; Tangier disease; aspartylglucosaminuria; congenital disorder of glycosylation, type la; Chediak-Higashi syndrome; macular dystrophy, corneal, 1; cystinosis, nephropathic; Fanconi-Bickel syndrome; Farber lipogranulomatosis; fibromatosis; geleophysic dysplasia; glycogen storage disease I; glycogen storage disease Ib; glycogen storage disease Ic; glycogen storage disease III; glycogen storage disease IV; glycogen storage disease V; glycogen storage disease VI; glycogen storage disease VII; glycogen storage disease 0; immunoosseous dysplasia, Schimke type; lipidosis; lipase b; mucolipidosis II; mucolipidosis II, including the variant form; mucolipidosis IV; neuraminidase deficiency with beta-galactosidase deficiency; mucolipidosis I; Niemann-Pick disease (a deficiency of sphingomyelinase); Niemann-Pick disease without sphingomyelinase deficiency (a deficiency of a npc1 gene encoding a cholesterol metabolizing enzyme); Refsum disease; Sea-blue histiocyte disease; infantile sialic acid storage disorder; sialuria; multiple sulfatase deficiency; triglyceride storage disease with impaired long-chain fatty acid oxidation; Winchester disease; Wolman disease (a deficiency of cholesterol ester hydrolase); Deoxyribonuclease I-like 1 disorder; arylsulfatase E disorder; ATPase, H+ transporting, lysosomal, subunit 1 disorder; glycogen storage disease IIb; Ras-associated protein rab9 disorder; chondrodysplasia punctata 1, X-linked recessive disorder; glycogen storage disease VIII; lysosome-associated membrane protein 2 disorder; Menkes syndrome; congenital disorder of glycosylation, type Ic; and sialuria. Replacement of less than 20%, e.g., less than 10% or about 1% to 5% levels of lysosomal storage enzyme found in nondiseased mammals, may prevent, inhibit or treat neurological symptoms such as neurological degeneration in mammals.

In one aspect of the disclosure, the methods described herein involve delivering to the CNS of an immunocompetent human in need of treatment a composition comprising an effective amount of a rAAV9 vector comprising an open reading frame encoding an IDUA. Routes of administration to the CNS/brain include intrathecal administration, intracranial administration, e.g., intracerebroventricular administration or lateral cerebroventricular administration, intranasal administration, endovascular administration, and intraparenchymal administration.

Other viral vectors may be employed in the methods of the invention, e.g., viral vectors such as retrovirus, lentivirus, adenovirus, semliki forest virus or herpes simplex virus vectors.

### Brief Description of the Figures

Figure 1. Experimental design for iduronidase-deficient mice administered AAV9-IDUA either intracerebroventricularly (ICV) or intrathecally. To prevent immune response, animals were either immunosuppressed with cyclophosphamide (CP), immunotolerized at birth by intravenous administration of human iduronidase protein (aldurazyme), or the injections were carried out in NOD-SCID immunodeficient mice that were also iduronidase deficient. Animals were sacrificed at the indicated time post-treatment, the brains were microdissected and extracts assayed for iduronidase activity.
Figure 2. IDUA activity in immunodeficient, IDUA deficient animals.
Figure 3. IDUA activity in immunosuppressed animals administered AAV vector by ICV route.
Figure 4. IDUA activity in immunosuppressed animals administered AAV vector by IT route.
Figure 5. IDUA activity in immunotolerized animals administered AAV vector ICV.
Figure 6. Compilation of all mean levels of IDUA activity for side-by-side comparison.
Figure 7. Data are grouped according to the area of the brain.
Figure 8. Assay for GAG storage material in the different sections of the brain for all four of the test groups.
Figure 9. Schematic of experimental design.
Figure 10. Intracranial infusion of AAV9-IDUA into immunodeficient MPS I mice. Adult animals were injected with 10¹¹ vector genomes and evaluated for iduronidase expression in the brain after 10 weeks. Enzyme activity levels in the brain were significantly higher than in the brains of wild type animals, and ranged from 30- to 300-fold higher than wild type.
Figure 11. Intracranial administration of AAV9-IDUA in immunocompetent, IDUA deficient mice. Adult animals were injected with 10¹¹ vector genomes, and immunosuppressed by weekly injection of cyclophosphamide (CP). CP injections were terminated at 6 weeks post vector injection due to poor health, and the animals were sacrificed at 8 weeks post-injection. Brains were microdissected and assayed for IDUA enzyme activity.
Figure 12. Intracranial infusion of AAV9-IDUA into immunotolerized MPS I mice. MPS 1 mice were tolerized with either a single dose of Aldurazyme at birth or multiple doses administered weekly, starting at birth. Mice were infused with vector at 4 months, and sacrificed at 11 weeks after injection. Brains were microdissected and analyzed for iduronidase expression. Enzyme activities ranged from an average of 10- to 1000-fold higher than wild type levels.
Figure 13. Intrathecal administration of AAV9-IDUA in immunocompetent, IDUA deficient animals. Adult MPS I mice were injected with AAV9-IDUA intrathecally, followed by a weekly immunosuppressive regimen of cyclophosphamide. Animals were sacrificed at 11 weeks post-injection, and then brains and spinal cords were analyzed for IDUA enzyme activity.

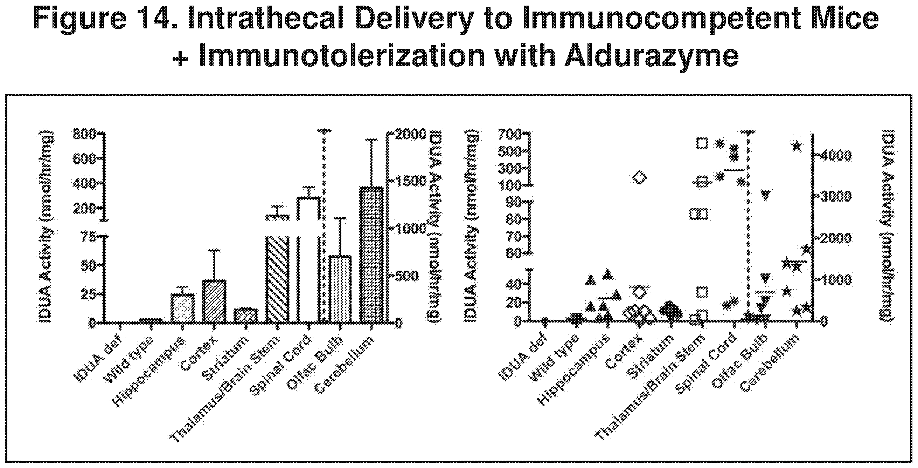

Figure 14. Intrathecal infusion of AAV9-IDUA in immunotolerized MPS I mice. IDUA deficient animals were tolerized at birth with a single dose of Aldurazyme or multiple doses administered weekly starting at birth. At 4 months of age animals were infused intrathecally with AAV9-IDUA vector, and at 10 weeks post-injection animals were sacrificed, brains microdissected and assayed for iduronidase activity. There was restoration of enzyme activity in all parts of the brain, with activities in the cerebellum ranging from 200- to 1500-fold higher than wild type levels. Levels of enzyme activity in the olfactory bulb and cerebellum (to the right of the dashed line) correspond to the right Y-axis.
Figure 15. Intrathecal infusion of AAV9-IDUA in immunocompetent MPS I animals. Control MPS I animals were injected with AAV9-IDUA vector, but were not immunosuppressed nor immunotolerized. Animals were sacrificed at 11 weeks after vector injection, and then their brains were assayed for iduronidase activity. Enzyme levels were restored to wild type levels in all parts of the brain, but were significantly lower than in animals that were either immunosuppressed or immunotolerized.
Figure 16. Normalization of glycosaminoglycan (GAG) levels following intracranial or intrathecal AAV9 infusion. AAV9-IDUA was injected intracranially or intrathecally into immunodeficient, immunosuppressed or immunotolerized MPS I mice as indicated. Animals were sacrificed 8-11 weeks after injection, then the brains were microdissected and analyzed for GAG levels. GAG storage was restored to wild type levels or close to wild type in all groups analyzed.

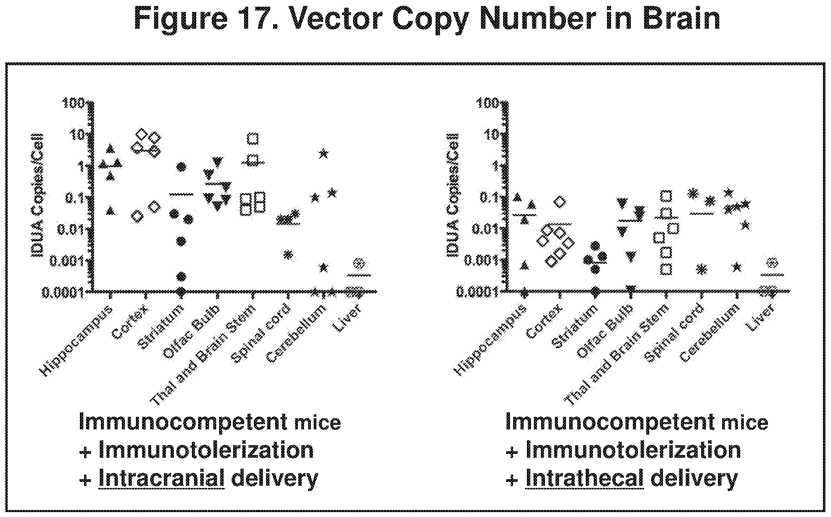

Figure 17. IDUA vector copies in brain. Microdissected brains were analyzed for IDUA vector sequences by QPCR. The copy numbers in intracranially and intrathecally injected mice correlate to the levels of enzyme activity depicted in Figures 11 and 13.
Figure 18. ICV infusion of AAV8-MCI into adult animals.
Figure 19. Intranasal administration of AAV9-IDUA in immunocompetent, IDUA deficient animals. Adult MPS I mice were infused with AAV9-IDUA intranasally, followed by a weekly immunosuppressive regimen of cyclophosphamide. Animals were sacrificed at 12 weeks post-injection and brains were analyzed for IDUA enzyme activity.
Figure 20. IDUA vector copies in brain. Microdissected brains were analyzed for IDUA vector sequences by QPCR. The copy numbers in intranasally injected mice correlate to the levels of enzyme in Figure 19.

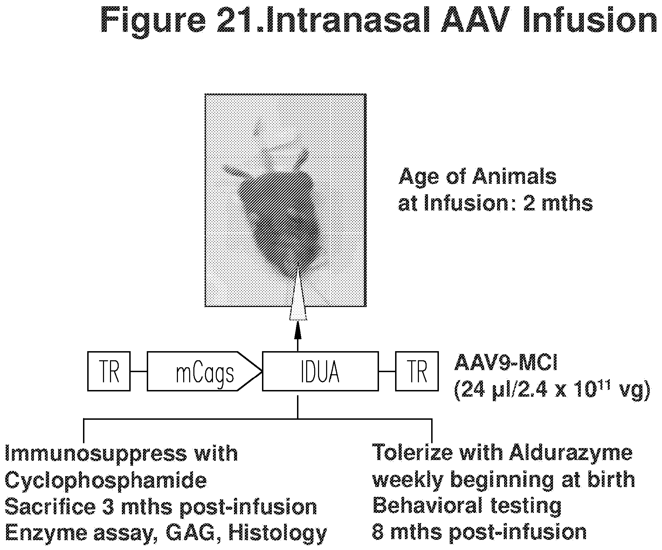

Figure 21. Protocol with immunosuppressant or tolerization using IN delivery of AAV9-IDUA.
Figure 22. Restoration of IDUA activity after IN delivery of AAV9-IDUA.
Figure 23. GAG activity after IN delivery of AAV9-IDUA.
Figure 24. IDUA immunofluorescence in brain after IN delivery of AAV9-IDUA.
Figure 25. GFP immunofluorescence in brain after IN delivery of AAV9-GFP.

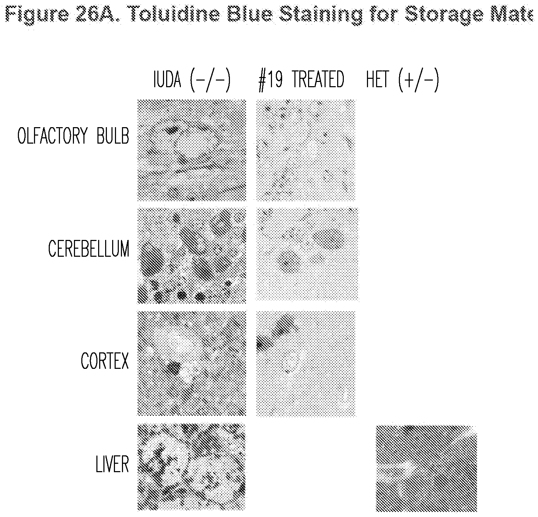

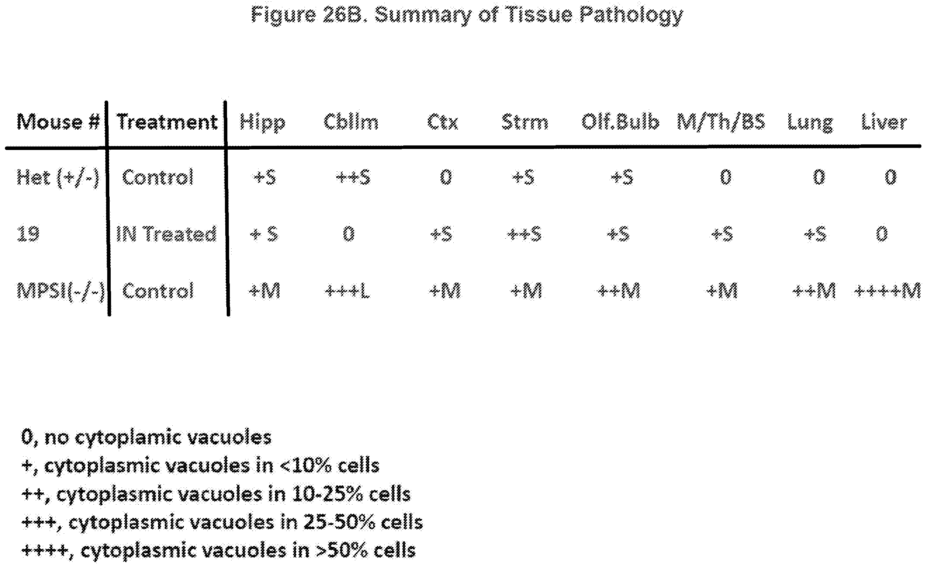

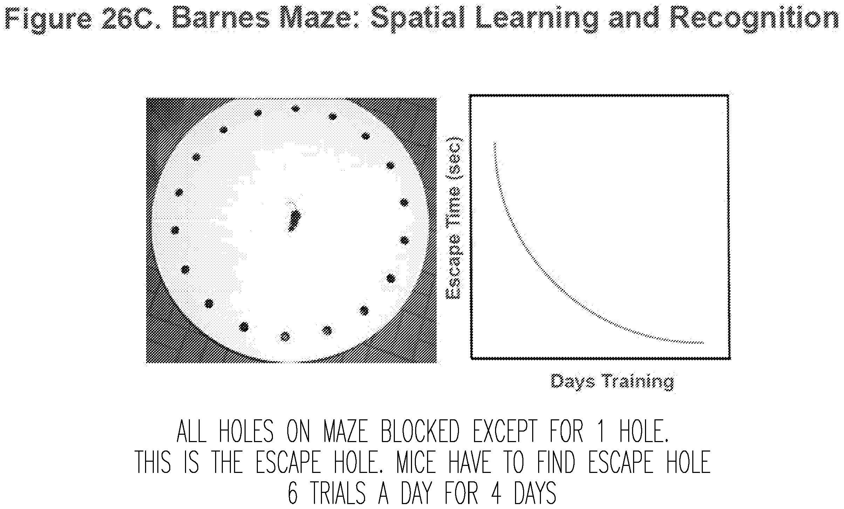

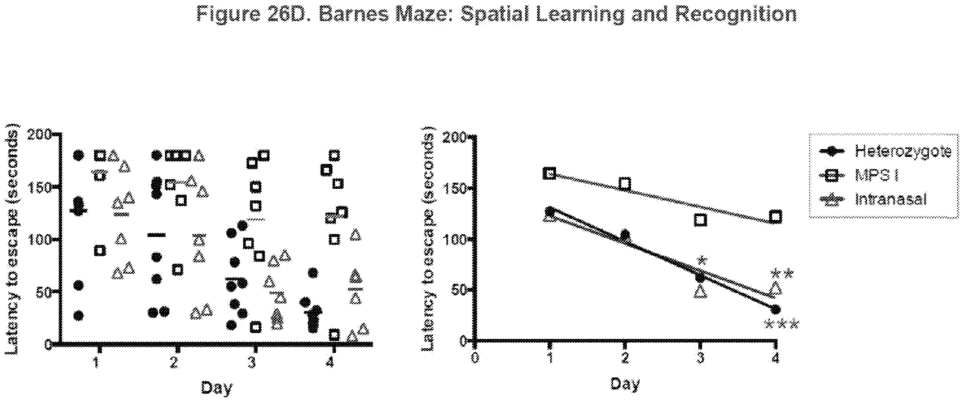

Figures 26A-D. A) Toluidine blue staining. B) Summary of tissue pathology in control heterozygous and homozygous MPS I mice and mice treated with IN delivery of IDUA AAV9-MCI. C) Barnes maze. D) Barnes maze data.
Figures 27A-B. A) Schematic of AAV9 vectors. B) Summary *of in vivo* testing groups for IT and IV delivery of AAV9.hIDS vectors.
Figure 28. IDS activity in plasma of mice administered AAV9-hIDS vectors via IT and IV routes.
Figure 29. CNS IDS activity after IT injection of AAV9-hIDS. For each group of mice, the data for the following tissues are presented left to right: spinal chord, thalamus/brain stem, cerebellum, cortex, hippocampus, and striatum.

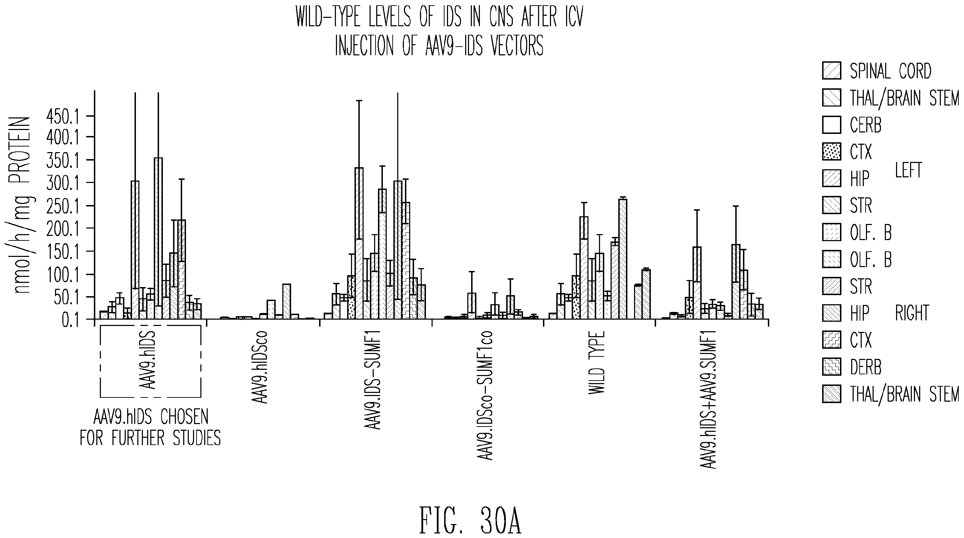

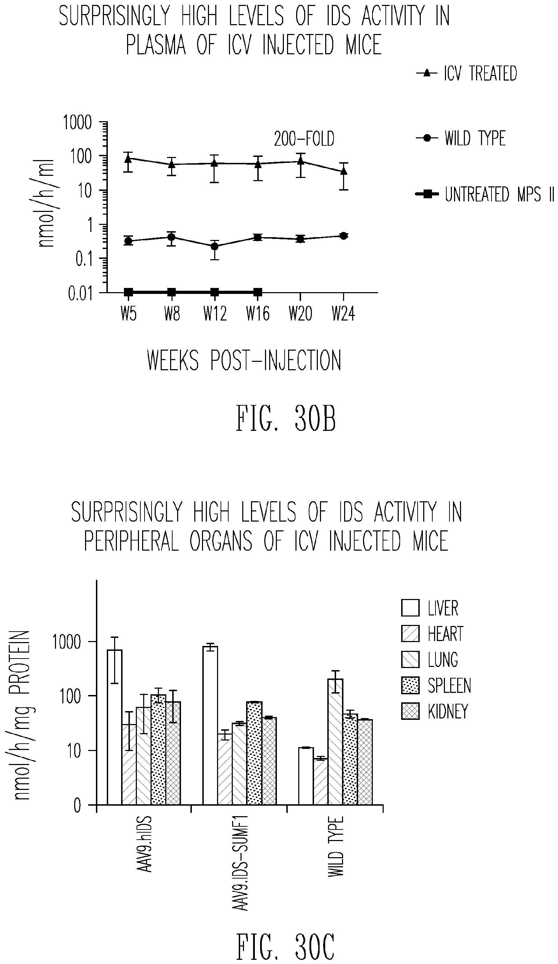

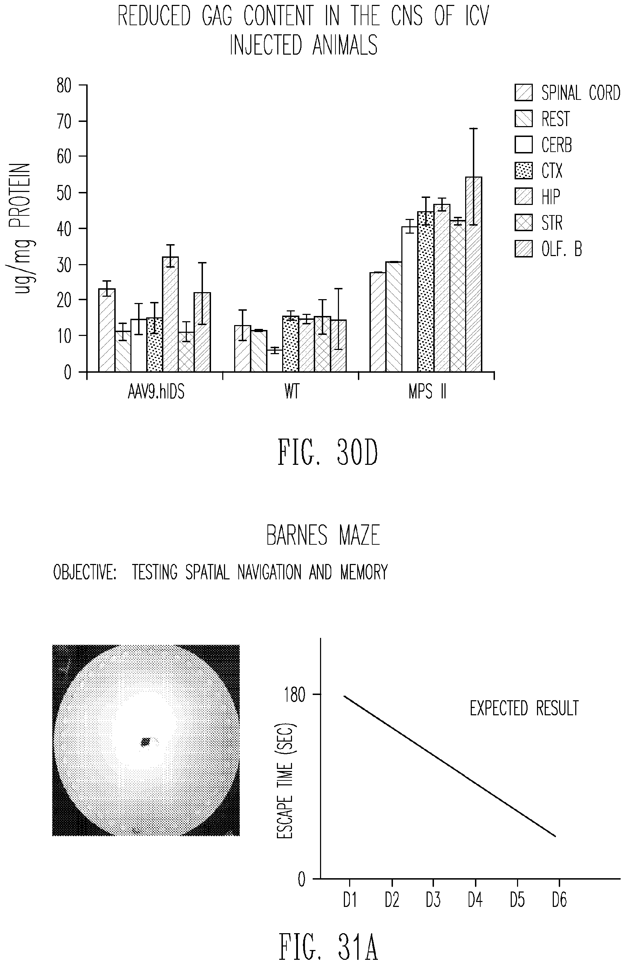

Figures 30A-D. A) CNS IDS Activity after ICV injection of AAV9-hIDS. For each group of mice, the data for the following tissues are presented left to right: spinal cord; the left side thalamus/brain stem, cerebellum, cortex, hippocampus, striatum, olfactory bulb, and the right side olfactory bulb, striatum, hippocampus, cortex, cerebellum, and thalamus/brain stem. B) IDS activity in plasma after ICV injection of AAV9-hIDS. C) IDS activity in peripheral organs after ICV injection of AAV9-hIDS. For each group of mice, the data for the following organs is presented left to right: liver, heart, lung, spleen and kidney. D) GAG content after ICV injection. For each group of mice, the data for the following tissues are presented left to right: spinal cord, rest, cerebellum, cortex, hippocampus, striatum, and olfactory bulb.
Figures 31A-B. A) Barnes maze. B) Performance on day 1 and day 4.
Figure 32. Comparison of neurologic function of wild-type and MPS II mice.
Figure 33. Experimental design for IV gene delivery using AAV9 or AAVrh10.
Figures 34A-C. Restoration of IDUA activity in plasma (A), peripheral tissues (B) and CNS (C) and after IV administration.
Figures 35A-C. GAG activity in urine (A), peripheral tissues (B) and CNS (C).
Figures 36A-F. IDUA immunofluorescence in tissue sections. A) Liver. B) Heart. C) Lung. D) Thalamus. E) Hippocampus. F) Cerebellum.
Figure 37. IDUA enzyme activity levels after IN infusion of AAV9-IDUA. High levels of IDUA enzyme activity were observed in olfactory bulb (10-100 fold higher than normal) and normalized (wt) levels in other parts of the brain.
Figure 38. Reduction of storage material in treated mice.
Figures 39A-D. IDUA (A), GFP (B); and olfactory bulb (C) immunofluorescence. (D) Co-staining with olfactory marker protein in olfactory bulb.
Figure 40. Improved neurocognitive function in IN treated mice.

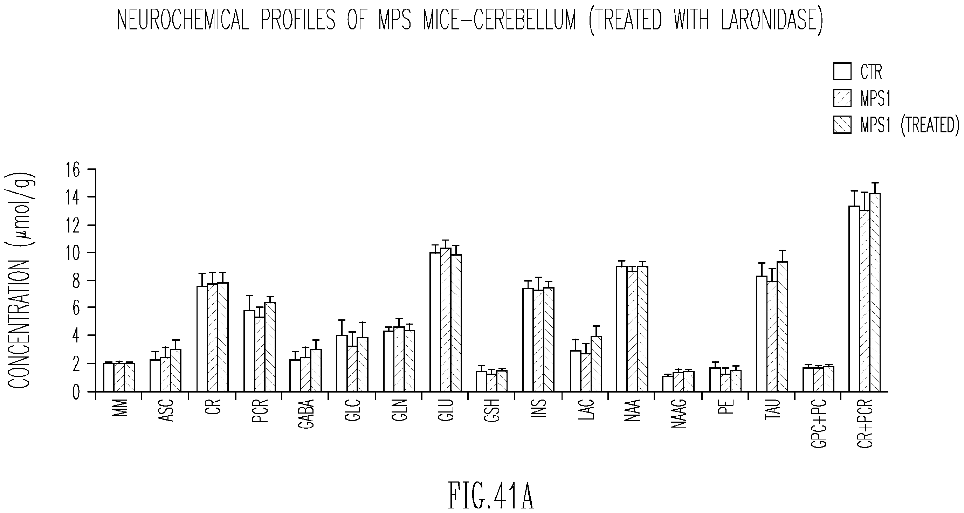

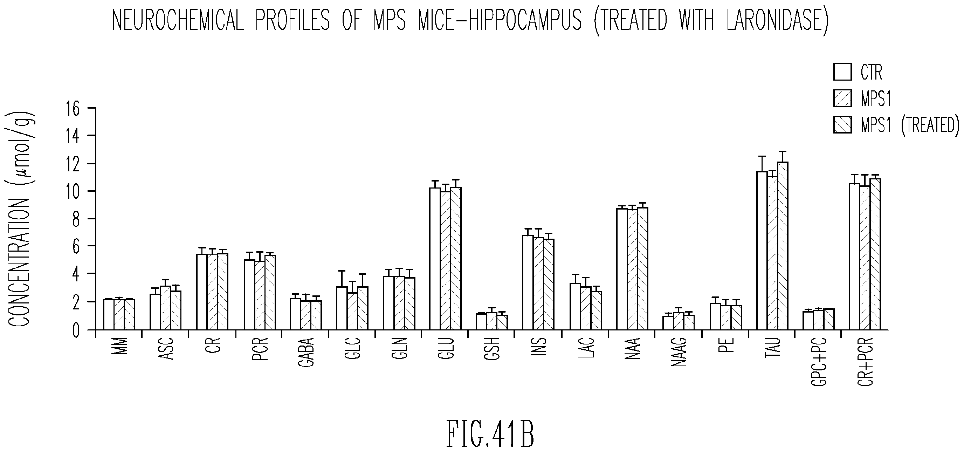

Figures 41A-B. Neurochemical profiles in cerebellum (A) and hippocampus (B). Control (CTR) is bar on left, MPS I (untreated) is middle bar and MPS I treated is bar on right, for each neurochemical profile.
Figure 42. Choroid plexus staining after IN delivery of AAVrh10-GFP.

### Detailed Description of the Invention

### Definitions

As used herein, "individual" (as in the subject of the treatment) means a mammal. Mammals include, for example, humans; non-human primates, e.g., apes and monkeys; and non-primates, e.g., dogs, cats, rats, mice, cattle, horses, sheep, and goats. Non-mammals include, for example, fish and birds.

The term "disease" or "disorder" are used interchangeably, and are used to refer to diseases or conditions wherein lack of or reduced amounts of a specific gene product, e.g., a lysosomal storage enzyme, plays a role in the disease such that a therapeutically beneficial effect can be achieved by supplementing, e.g., to at least 1% of normal levels.

"Substantially" as the term is used herein means completely or almost completely; for example, a composition that is "substantially free" of a component either has none of the component or contains such a trace amount that any relevant functional property of the composition is unaffected by the presence of the trace amount, or a compound is "substantially pure" is there are only negligible traces of impurities present.

"Treating" or "treatment" within the meaning herein refers to an alleviation of symptoms associated with a disorder or disease, "inhibiting" means inhibition of further progression or worsening of the symptoms associated with the disorder or disease, and "preventing" refers to prevention of the symptoms associated with the disorder or disease.

As used herein, an "effective amount" or a "therapeutically effective amount" of an agent of the invention e.g., a recombinant AAV encoding a gene product, refers to an amount of the agent that alleviates, in whole or in part, symptoms associated with the disorder or condition, or halts or slows further progression or worsening of those symptoms, or prevents or provides prophylaxis for the disorder or condition, e.g., an amount that is effective to prevent, inhibit or treat in the individual one or more neurological symptoms.

In particular, a "therapeutically effective amount" refers to an amount effective, at dosages and for periods of time necessary, to achieve the desired therapeutic result. A therapeutically effective amount is also one in which any toxic or detrimental effects of compounds of the invention are outweighed by the therapeutically beneficial effects.

A "vector" as used herein refers to a macromolecule or association of macromolecules that comprises or associates with a polynucleotide and which can be used to mediate delivery of the polynucleotide to a cell, either *in vitro* or *in vivo.* Illustrative vectors include, for example, plasmids, viral vectors, liposomes and other gene delivery vehicles. The polynucleotide to be delivered, sometimes referred to as a "target polynucleotide" or "transgene," may comprise a coding sequence of interest in gene therapy (such as a gene encoding a protein of therapeutic interest) and/or a selectable or detectable marker.

"AAV" is adeno-associated virus, and may be used to refer to the virus itself or derivatives thereof. The term covers all subtypes, serotypes and pseudotypes, and both naturally occurring and recombinant forms, except where required otherwise. As used herein, the term "serotype" refers to an AAV which is identified by and distinguished from other AAVs based on its binding properties, e.g., there are eleven serotypes of AAVs, AAV1-AAV11, including AAV2, AAV5, AAV8, AAV9 and AAVrh10, and the term encompasses pseudotypes with the same binding properties. Thus, for example, AAV9 serotypes include AAV with the binding properties of AAV9, e.g., a pseudotyped AAV comprising AAV9 capsid and a rAAV genome which is not derived or obtained from AAV9 or which genome is chimeric. The abbreviation "rAAV" refers to recombinant adeno-associated virus, also referred to as a recombinant AAV vector (or "rAAV vector").

An "AAV virus" refers to a viral particle composed of at least one AAV capsid protein and an encapsidated polynucleotide. If the particle comprises a heterologous polynucleotide (i.e., a polynucleotide other than a wild-type AAV genome such as a transgene to be delivered to a mammalian cell), it is typically referred to as "rAAV". An AAV "capsid protein" includes a capsid protein of a wild-type AAV, as well as modified forms of an AAV capsid protein which are structurally and or functionally capable of packaging a rAAV genome and bind to at least one specific cellular receptor which may be different than a receptor employed by wild type AAV. A modified AAV capsid protein includes a chimeric AAV capsid protein such as one having amino acid sequences from two or more serotypes of AAV, e.g., a capsid protein formed from a portion of the capsid protein from AAV9 fused or linked to a portion of the capsid protein from AAV-2, and a AAV capsid protein having a tag or other detectable non-AAV capsid peptide or protein fused or linked to the AAV capsid protein, e.g., a portion of an antibody molecule which binds a receptor other than the receptor for AAV9, such as the transferrin receptor, may be recombinantly fused to the AAV9 capsid protein.

A "pseudotyped" rAAV is an infectious virus having any combination of an AAV capsid protein and an AAV genome. Capsid proteins from any AAV serotype may be employed with a rAAV genome which is derived or obtainable from a wild-type AAV genome of a different serotype or which is a chimeric genome, i.e., formed from AAV DNA from two or more different serotypes, e.g., a chimeric genome having 2 inverted terminal repeats (ITRs), each ITR from a different serotype or chimeric ITRs. The use of chimeric genomes such as those comprising ITRs from two AAV serotypes or chimeric ITRs can result in directional recombination which may further enhance the production of transcriptionally active intermolecular concatamers. Thus, the 5' and 3' ITRs within a rAAV vector of the invention may be homologous, i.e., from the same serotype, heterologous, i.e., from different serotypes, or chimeric, i.e., an ITR which has ITR sequences from more than one AAV serotype.

### rAAV vectors

Adeno-associated viruses of any serotype are suitable to prepare rAAV, since the various serotypes are functionally and structurally related, even at the genetic level. All AAV serotypes apparently exhibit similar replication properties mediated by homologous rep genes; and all generally bear three related capsid proteins such as those expressed in AAV2. The degree of relatedness is further suggested by heteroduplex analysis which reveals extensive cross-hybridization between serotypes along the length of the genome; and the presence of analogous self-annealing segments at the termini that correspond to ITRs. The similar infectivity patterns also suggest that the replication functions in each serotype are under similar regulatory control. Among the various AAV serotypes, AAV2 is most commonly employed.

An AAV vector of the invention typically comprises a polynucleotide that is heterologous to AAV. The polynucleotide is typically of interest because of a capacity to provide a function to a target cell in the context of gene therapy, such as up- or down-regulation of the expression of a certain phenotype. Such a heterologous polynucleotide or "transgene," generally is of sufficient length to provide the desired function or encoding sequence.

Where transcription of the heterologous polynucleotide is desired in the intended target cell, it can be operably linked to its own or to a heterologous promoter, depending for example on the desired level and/or specificity of transcription within the target cell, as is known in the art. Various types of promoters and enhancers are suitable for use in this context. Constitutive promoters provide an ongoing level of gene transcription, and may be preferred when it is desired that the therapeutic or prophylactic polynucleotide be expressed on an ongoing basis. Inducible promoters generally exhibit low activity in the absence of the inducer, and are up-regulated in the presence of the inducer. They may be preferred when expression is desired only at certain times or at certain locations, or when it is desirable to titrate the level of expression using an inducing agent. Promoters and enhancers may also be tissue-specific: that is, they exhibit their activity only in certain cell types, presumably due to gene regulatory elements found uniquely in those cells.

Illustrative examples of promoters are the SV40 late promoter from simian virus 40, the Baculovirus polyhedron enhancer/promoter element, Herpes Simplex Virus thymidine kinase (HSV tk), the immediate early promoter from cytomegalovirus (CMV) and various retroviral promoters including LTR elements. Inducible promoters include heavy metal ion inducible promoters (such as the mouse mammary tumor virus (mMTV) promoter or various growth hormone promoters), and the promoters from T7 phage which are active in the presence of T7 RNA polymerase. By way of illustration, examples of tissue-specific promoters include various surfactin promoters (for expression in the lung), myosin promoters (for expression in muscle), and albumin promoters (for expression in the liver). A large variety of other promoters are known and generally available in the art, and the sequences of many such promoters are available in sequence databases such as the GenBank database.

Where translation is also desired in the intended target cell, the heterologous polynucleotide will preferably also comprise control elements that facilitate translation (such as a ribosome binding site or "RBS" and a polyadenylation signal). Accordingly, the heterologous polynucleotide generally comprises at least one coding region operatively linked to a suitable promoter, and may also comprise, for example, an operatively linked enhancer, ribosome binding site and poly-A signal. The heterologous polynucleotide may comprise one encoding region, or more than one encoding regions under the control of the same or different promoters. The entire unit, containing a combination of control elements and encoding region, is often referred to as an expression cassette.

The heterologous polynucleotide is integrated by recombinant techniques into or in place of the AAV genomic coding region (i.e., in place of the AAV rep and cap genes), but is generally flanked on either side by AAV inverted terminal repeat (ITR) regions. This means that an ITR appears both upstream and downstream from the coding sequence, either in direct juxtaposition, e.g., (although not necessarily) without any intervening sequence of AAV origin in order to reduce the likelihood of recombination that might regenerate a replication-competent AAV genome. However, a single ITR may be sufficient to carry out the functions normally associated with configurations comprising two ITRs (see, for example, WO 94/13788), and vector constructs with only one ITR can thus be employed in conjunction with the packaging and production methods of the present invention.

The native promoters for rep are self-regulating, and can limit the amount of AAV particles produced. The rep gene can also be operably linked to a heterologous promoter, whether rep is provided as part of the vector construct, or separately. Any heterologous promoter that is not strongly downregulated by rep gene expression is suitable; but inducible promoters may be preferred because constitutive expression of the rep gene can have a negative impact on the host cell. A large variety of inducible promoters are known in the art; including, by way of illustration, heavy metal ion inducible promoters (such as metallothionein promoters); steroid hormone inducible promoters (such as the MMTV promoter or growth hormone promoters); and promoters such as those from T7 phage which are active in the presence of T7 RNA polymerase. One sub-class of inducible promoters are those that are induced by the helper virus that is used to complement the replication and packaging of the rAAV vector. A number of helper-virus-inducible promoters have also been described, including the adenovirus early gene promoter which is inducible by adenovirus E1A protein; the adenovirus major late promoter; the herpesvirus promoter which is inducible by herpesvirus proteins such as VP16 or 1CP4; as well as vaccinia or poxvirus inducible promoters.

Methods for identifying and testing helper-virus-inducible promoters have been described (see, e.g., WO 96/17947). Thus, methods are known in the art to determine whether or not candidate promoters are helper-virus-inducible, and whether or not they will be useful in the generation of high efficiency packaging cells. Briefly, one such method involves replacing the p5 promoter of the AAV rep gene with the putative helper-virus-inducible promoter (either known in the art or identified using well-known techniques such as linkage to promoter-less "reporter" genes). The AAV rep-cap genes (with p5 replaced), e.g., linked to a positive selectable marker such as an antibiotic resistance gene, are then stably integrated into a suitable host cell (such as the HeLa or A549 cells exemplified below). Cells that are able to grow relatively well under selection conditions (e.g., in the presence of the antibiotic) are then tested for their ability to express the rep and cap genes upon addition of a helper virus. As an initial test for rep and/or cap expression, cells can be readily screened using immunofluorescence to detect Rep and/or Cap proteins. Confirmation of packaging capabilities and efficiencies can then be determined by functional tests for replication and packaging of incoming rAAV vectors. Using this methodology, a helper-virus-inducible promoter derived from the mouse metallothionein gene has been identified as a suitable replacement for the p5 promoter, and used for producing high titers of rAAV particles (as described in WO 96/17947).

Removal of one or more AAV genes is in any case desirable, to reduce the likelihood of generating replication-competent AAV ("RCA"). Accordingly, encoding or promoter sequences for rep, cap, or both, may be removed, since the functions provided by these genes can be provided *in trans,* e.g., in a stable line or via co-transfection.

The resultant vector is referred to as being "defective" in these functions. In order to replicate and package the vector, the missing functions are complemented with a packaging gene, or a plurality thereof, which together encode the necessary functions for the various missing rep and/or cap gene products. The packaging genes or gene cassettes are in one embodiment not flanked by AAV ITRs and in one embodiment do not share any substantial homology with the rAAV genome. Thus, in order to minimize homologous recombination during replication between the vector sequence and separately provided packaging genes, it is desirable to avoid overlap of the two polynucleotide sequences. The level of homology and corresponding frequency of recombination increase with increasing length of homologous sequences and with their level of shared identity. The level of homology that will pose a concern in a given system can be determined theoretically and confirmed experimentally, as is known in the art. Typically, however, recombination can be substantially reduced or eliminated if the overlapping sequence is less than about a 25 nucleotide sequence if it is at least 80% identical over its entire length, or less than about a 50 nucleotide sequence if it is at least 70% identical over its entire length. Of course, even lower levels of homology are preferable since they will further reduce the likelihood of recombination. It appears that, even without any overlapping homology, there is some residual frequency of generating RCA. Even further reductions in the frequency of generating RCA (e.g., by nonhomologous recombination) can be obtained by "splitting" the replication and encapsidation functions of AAV, as described by Allen et al., WO 98/27204).

The rAAV vector construct, and the complementary packaging gene constructs can be implemented in this invention in a number of different forms. Viral particles, plasmids, and stably transformed host cells can all be used to introduce such constructs into the packaging cell, either transiently or stably.

In certain embodiments of this invention, the AAV vector and complementary packaging gene(s), if any, are provided in the form of bacterial plasmids, AAV particles, or any combination thereof. In other embodiments, either the AAV vector sequence, the packaging gene(s), or both, are provided in the form of genetically altered (preferably inheritably altered) eukaryotic cells. The development of host cells inheritably altered to express the AAV vector sequence, AAV packaging genes, or both, provides an established source of the material that is expressed at a reliable level.

A variety of different genetically altered cells can thus be used in the context of this invention. By way of illustration, a mammalian host cell may be used with at least one intact copy of a stably integrated rAAV vector. An AAV packaging plasmid comprising at least an AAV rep gene operably linked to a promoter can be used to supply replication functions (as described in U.S. Patent 5,658,776). Alternatively, a stable mammalian cell line with an AAV rep gene operably linked to a promoter can be used to supply replication functions (see, e.g., Trempe et al., WO 95/13392); Burstein et al. (WO 98/23018); and Johnson et al. (U.S. No. 5,656,785). The AAV cap gene, providing the encapsidation proteins as described above, can be provided together with an AAV rep gene or separately (see, e.g., the above-referenced applications and patents as well as Allen et al. (WO 98/27204). Other combinations are possible and included within the scope of this invention.

### Pathways for Delivery

Despite the immense network of the cerebral vasculature, systemic delivery of therapeutics to the central nervous system (CNS) is not effective for greater than 98% of small molecules and for nearly 100% of large molecules (Partridge, 2005). The lack of effectiveness is due to the presence of the blood-brain barrier (BBB), which prevents most foreign substances, even many beneficial therapeutics, from entering the brain from the circulating blood. While certain small molecule, peptide, and protein therapeutics given systemically reach the brain parenchyma by crossing the BBB (Banks, 2008), generally high systemic doses are needed to achieve therapeutic levels, which can lead to adverse effects in the body. Therapeutics can be introduced directly into the CNS by intracerebroventricular or intraparenchymal injections. Intranasal delivery bypasses the BBB and targets therapeutics directly to the CNS utilizing pathways along olfactory and trigeminal nerves innervating the nasal passages (Frey II, 2002; Thorne et al., 2004; Dhanda et al., 2005).

Any route of rAAV administration may be employed so long as that route and the amount administered are prophylactically or therapeutically useful. In one example, routes of administration to the CNS include intrathecal and intracranial. Intracranial administration may be to the cisterna magna or ventricle. The term "cisterna magna" is intended to include access to the space around and below the cerebellum via the opening between the skull and the top of the spine. The term "cerebral ventricle" is intended to include the cavities in the brain that are continuous with the central canal of the spinal cord. Intracranial administration is via injection or infusion and suitable dose ranges for intracranial administration are generally about 10³ to 10¹⁵ infectious units of viral vector per microliter delivered in 1 to 3000 microliters of single injection volume. For instance, viral genomes or infectious units of vector per micro liter would generally contain about 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ viral genomes or infectious units of viral vector delivered in about 10, 50, 100, 200, 500, 1000, or 2000 microliters. It should be understood that the aforementioned dosage is merely an exemplary dosage and those of skill in the art will understand that this dosage may be varied. Effective doses may be extrapolated from dose-responsive curves derived from in vitro or in vivo test systems.

The AAV delivered in the intrathecal methods of treatment of the present invention may be administered through any convenient route commonly used for intrathecal administration. For example, the intrathecal administration may be via a slow infusion of the formulation for about an hour. Intrathecal administration is via injection or infusion and suitable dose ranges for intrathecal administration are generally about 10³ to 10¹⁵ infectious units of viral vector per microliter delivered in, for example, 1, 2, 5, 10, 25, 50, 75or 100 or more milliliters, e.g.,1 to 10,000 milliliters or 0.5 to 15 milliliters, of single injection volume. For instance, viral genomes or infectious units of vector per microliter would generally contain about 10⁴, 10⁵, 10⁶, 10⁷, 10⁸, 10⁹, 10¹⁰, 10¹¹, 10¹², 10¹³, or 10¹⁴ viral genomes or infectious units of viral vector.

The AAV delivered in the intranasal methods of treatment of the present invention may be administered in suitable dose ranges, generally about 10³ to 10¹⁵ infectious units of viral vector per microliter delivered in, for example, 1, 2, 5, 10, 25, 50, 75 or 100 or more milliliters, e.g.,1 to 10,000 milliliters or 0.5 to 15 milliliters. For instance, viral genomes or infectious units of vector per microliter would generally contain about 10⁴, 10⁵, 10⁶, 10⁷, 10⁸, 10⁹, 10¹⁰, 10¹¹, 10¹², 10¹³, 10¹⁴, 10¹⁵, 10¹⁶, or 10¹⁷ viral genomes or infectious units of viral vector, e.g., at least 1.2 x 10¹¹ genomes or infectious units, for instance at least 2 x 10¹¹ up to about 2 x 10¹² genomes or infectious units or about 1 x 10¹³ to about 5 x 10¹⁶ genomes or infectious units. In one embodiment, the AAV employed for intranasal delivery is one that binds to glycans with terminal galactose residues and in one embodiment the dose is 2 to 8 fold higher than w9 x 10¹⁰ to less than 1 x 10¹¹ AAV8 genomes or infectious units of viral vector.

The therapy, if a lysosomal storage enzyme such as IDUA is expressed, results in the normalization of lysosomal storage granules in the neuronal and/or meningeal tissue of the subjects as discussed above. It is contemplated that the deposition of storage granules is ameliorated from neuronal and glial tissue, thereby alleviating the developmental delay and regression seen in individuals suffering with lysosomal storage disease. Other effects of the therapy may include the normalization of lysosomal storage granules in the cerebral meninges near the arachnoid granulation, the presence of which in lysosomal storage disease result in high pressure hydrocephalus. The methods of the invention also may be used in treating spinal cord compression that results from the presence of lysosomal storage granules in the cervical meninges near the cord at C1-C5 or elsewhere in the spinal cord. The methods of the invention also are directed to the treatment of cysts that are caused by the perivascular storage of lysosomal storage granules around the vessels of the brain. In other embodiments, the therapy also may advantageously result in normalization of liver volume and urinary glycosaminoglycan excretion, reduction in spleen size and apnea/hypopnea events, increase in height and growth velocity in prepubertal subjects, increase in shoulder flexion and elbow and knee extension, and reduction in tricuspid regurgitation or pulmonic regurgitation.

The intrathecal administration of the present invention may comprise introducing the composition into the lumbar area. Any such administration may be via a bolus injection. Depending on the severity of the symptoms and the responsiveness of the subject to the therapy, the bolus injection may be administered once per week, once per month, once every 6 months or annually. In other embodiments, the intrathecal administration is achieved by use of an infusion pump. Those of skill in the art are aware of devices that may be used to effect intrathecal administration of a composition. The composition may be intrathecally given, for example, by a single injection, or continuous infusion. It should be understood that the dosage treatment may be in the form of a single dose administration or multiple doses.

As used herein, the term "intrathecal administration" is intended to include delivering a pharmaceutical composition directly into the cerebrospinal fluid of a subject, by techniques including lateral cerebroventricular injection through a burrhole or cistemal or lumbar puncture or the like. The term "lumbar region" is intended to include the area between the third and fourth lumbar (lower back) vertebrae and, more inclusively, the L2-S1 region of the spine.

Administration of a composition in accordance with the present invention to any of the above mentioned sites can be achieved by direct injection of the composition or by the use of infusion pumps. For injection, the composition can be formulated in liquid solutions, e.g., in physiologically compatible buffers such as Hank's solution, Ringer's solution or phosphate buffer. In addition, the enzyme may be formulated in solid form and re-dissolved or suspended immediately prior to use. Lyophilized forms are also included. The injection can be, for example, in the form of a bolus injection or continuous infusion (e.g., using infusion pumps) of the enzyme.

In one embodiment of the invention, the rAAV is administered by lateral cerebroventricular injection into the brain of a subject. The injection can be made, for example, through a burr hole made in the subject's skull. In another embodiment, the enzyme and/or other pharmaceutical formulation is administered through a surgically inserted shunt into the cerebral ventricle of a subject. For example, the injection can be made into the lateral ventricles, which are larger, even though injection into the third and fourth smaller ventricles can also be made. In yet another embodiment, the compositions used in the present invention are administered by injection into the cisterna magna or lumbar area of a subject.

While the exact mechanisms underlying intranasal drug delivery to the CNS are not entirely understood, an accumulating body of evidence demonstrates that pathways involving nerves connecting the nasal passages to the brain and spinal cord are important. In addition, pathways involving the vasculature, cerebrospinal fluid, and lymphatic system have been implicated in the transport of molecules from the nasal cavity to the CNS. It is likely that a combination of these pathways is responsible, although one pathway may predominate, depending on the properties of the therapeutic, the characteristics of the formulation, and the delivery device used.

Therapeutics can rapidly gain access to the CNS following intranasal administration along olfactory nerve pathways leading from the nasal cavity directly to the CNS. Olfactory nerve pathways are a major component of intranasal delivery, evidenced by the fact that fluorescent tracers are associated with olfactory nerves as they traverse the cribriform plate (Jansson et al., 2002), drug concentrations in the olfactory bulbs are generally among the highest CNS concentrations observed (Thorne et al., 2004; Banks et al., 2004; Graff et al., 2005a); Nonaka et al., 2008; Ross et al., 2004; Ross et al., 2008; Thorne et al., 2008), and a strong, positive correlation exists between concentrations in the olfactory epithelium and olfactory bulbs (Dhuria et al., 2009a).

Olfactory pathways arise in the upper portion of the nasal passages, in the olfactory region, where olfactory receptor neurons (ORNs) are interspersed among supporting cells (sustentacular cells), microvillar cells, and basal cells. ORNs mediate the sense of smell by conveying sensory information from the peripheral environment to the CNS (Clerico et al., 2003). Beneath the epithelium, the lamina propria contains mucus secreting Bowman's glands, axons, blood vessels, lymphatic vessels, and connective tissue. The dendrites of ORNs extend into the mucous layer of the olfactory epithelium, while axons of these bipolar neurons extend centrally through the lamina propria and through perforations in the cribriform plate of the ethmoid bone, which separates the nasal and cranial cavities. The axons of ORNs pass through the subarachnoid space containing CSF and terminate on mitral cells in the olfactory bulbs. From there, neural projections extend to multiple brain regions including the olfactory tract, anterior olfactory nucleus, piriform cortex, amygdala, and hypothalamus (Buck, 2000). In addition to ORNs, chemosensory neurons located at the anterior tip of the nasal cavity in the Grueneberg ganglion lead into the olfactory bulbs (Fuss et al., 2005; Koos et al., 2005).

The unique characteristics of the ORNs contribute to a dynamic cellular environment critical for intranasal delivery to the CNS. Due to the direct contact with toxins in the external environment, ORNs regenerate every 3-4 weeks from basal cells residing in the olfactory epithelium (Mackay-Sim, 2003). Special Schwann cell-like cells called olfactory ensheathing cells (OECs) envelope the axons of ORNs and have an important role in axonal regeneration, regrowth, and remyelination (Field et al., 2003; Li et al., 2005a; Li et al., 2005b). The OECs create continuous, fluid-filled perineurial channels that, interestingly, remain open, despite the degeneration and regeneration of ORNs (Williams et al., 2004).

Given the unique environment of the olfactory epithelium, it is possible for intranasally administered therapeutics to reach the CNS via extracellular or intracellular mechanisms of transport along olfactory nerves. Extracellular transport mechanisms involve the rapid movement of molecules between cells in the nasal epithelium, requiring only several minutes to 30 minutes for a drug to reach the olfactory bulbs and other areas of the CNS after intranasal administration (Frey II, 2002; Balin et al., 1986). Transport likely involves bulk flow mechanisms (Thorne et al., 2004; Thorne et al., 2001) within the channels created by the OECs. Drugs may also be propelled within these channels by the structural changes that occur during depolarization and axonal propagation of the action potential in adjacent axons (Luzzati et al., 2004). Intracellular transport mechanisms involve the uptake of molecules into ORNs by passive diffusion, receptor-mediated endocytosis or adsorptive endocytosis, followed by slower axonal transport, taking several hours to days for a drug to appear in the olfactory bulbs and other brain areas (Baker et al., 1986; Broadwell et al., 1985; Kristensson et al., 1971). Intracellular transport in ORNs has been demonstrated for small, lipophilic molecules such as gold particles (de Lorenzo, 1970; Gopinath et al., 1978), aluminum salts (Perl et al., 1987), and for substances with receptors on ORNs such as WGA-HRP (Thorne et al., 1995; Baker et al., 1986; Itaya et al., 1986; Shipley, 1985). Intracellular mechanisms, while important for certain therapeutics, are not likely to be the predominant mode of transport into the CNS. While some large molecules, such as galanin-like peptide (GALP), exhibit saturable transport pathways into the CNS (Nonaka et al., 2008), for other large molecules such as NGF and insulin-like growth factor-I (IGF-I), intranasal delivery into the brain is nonsaturable and not receptor mediated (Thorne et al., 2004; Chen et al., 1998; Zhao et al., 2004),

An often overlooked but important pathway connecting the nasal passages to the CNS involves the trigeminal nerve, which innervates the respiratory and olfactory epithelium of the nasal passages and enters the CNS in the pons (Clerico et al., 2003; Graff et al., 2003). Interestingly, a small portion of the trigeminal nerve also terminates in the olfactory bulbs (Schaefer et al., 2002). The cellular composition of the respiratory region of the nasal passages is different from that of the olfactory region, with ciliated epithelial cells distributed among mucus secreting goblet cells. These cells contribute to mucociliary clearance mechanisms that remove mucus along with foreign substances from the nasal cavity to the nasopharynx. The trigeminal nerve conveys sensory information from the nasal cavity, the oral cavity, the eyelids, and the cornea, to the CNS via the ophthalmic division (V1), the maxillary division (V2), or the mandibular division (V3) of the trigeminal nerve (Clerico et al., 2003; Gray, 1978). Branches from the ophthalmic division of the trigeminal nerve provide innervation to the dorsal nasal mucosa and the anterior portion of the nose, while branches of the maxillary division provide innervation to the lateral walls of the nasal mucosa. The mandibular division of the trigeminal nerve extends to the lower jaw and teeth, with no direct neural inputs to the nasal cavity. The three branches of the trigeminal nerve come together at the trigeminal ganglion and extend centrally to enter the brain at the level of the pons, terminating in the spinal trigeminal nuclei in the brainstem. A unique feature of the trigeminal nerve is that it enters the brain from the respiratory epithelium of the nasal passages at two sites: (1) through the anterior lacerated foramen near the pons and (2) through the cribriform plate near the olfactory bulbs, creating entry points into both caudal and rostral brain areas following intranasal administration. It is also likely that other nerves that innervate the face and head, such as the facial nerve, or other sensory structures in the nasal cavity, such as the Grueneberg ganglion, may provide entry points for intranasally applied therapeutics into the CNS.

Traditionally, the intranasal route of administration has been utilized to deliver drugs to the systemic circulation via absorption into the capillary blood vessels underlying the nasal mucosa. The nasal mucosa is highly vascular, receiving its blood supply from branches of the maxillary, ophthalmic and facial arteries, which arise from the carotid artery (Clerico et al., 2003; Cauna, 1982). The olfactory mucosa receives blood from small branches of the ophthalmic artery, whereas the respiratory mucosa receives blood from a large caliber arterial branch of the maxillary artery (DeSesso, 1993). The relative density of blood vessels is greater in the respiratory mucosa compared to the olfactory mucosa, making the former region an ideal site for absorption into the blood (DeSesso, 1993). The vasculature in the respiratory region contains a mix of continuous and fenestrated endothelia (Grevers et al., 1987; Van Diest et al., 1979), allowing both small and large molecules to enter the systemic circulation following nasal administration.

Delivery to the CNS following absorption into the systemic circulation and subsequent transport across the BBB is possible, especially for small, lipophilic drugs, which more easily enter the blood stream and cross the BBB compared to large, hydrophilic therapeutics such as peptides and proteins.

Increasing evidence is emerging suggesting that mechanisms involving channels associated with blood vessels, or perivascular channels, are involved in intranasal drug delivery to the CNS. Perivascular spaces are bound by the outermost layer of blood vessels and the basement membrane of the surrounding tissue (Pollock et al., 1997). These perivascular spaces act as a lymphatic system for the brain, where neuron-derived substances are cleared from brain interstitial fluid by entering perivascular channels associated with cerebral blood vessels. Perivascular transport is due to bulk flow mechanisms, as opposed to diffusion alone (Cserr et al., 1981; Groothuis et al., 2007), and arterial pulsations are also a driving force for perivascular transport (Rennels et al., 1985; Rennels et al., 1985). Intranasally applied drugs can move into perivascular spaces in the nasal passages or after reaching the brain and the widespread distribution observed within the CNS could be due to perivascular transport mechanisms (Thorne et al., 2004).

Pathways connecting the subarachnoid space containing CSF, perineurial spaces encompassing olfactory nerves, and the nasal lymphatics are important for CSF drainage and these same pathways provide access for intranasally applied therapeutics to the CSF and other areas of the CNS. Several studies document that tracers injected into the CSF in the cerebral ventricles or subarachnoid space drain to the underside of the olfactory bulbs into channels associated with olfactory nerves traversing the cribriform plate and reach the nasal lymphatic system and cervical lymph nodes (Bradbury et al., 1983; Hatterer et al., 2006; Johnston et al., 2004a); Kida et al., 1993; Walter et al., 2006a; Walter et al., 2006b). Drugs can access the CNS via these same pathways after intranasal administration, moving from the nasal passages to the CSF to the brain interstitial spaces and perivascular spaces for distribution throughout the brain. These drainage pathways are significant in a number of animal species (sheep, rabbits, and rats) accounting for approximately 50% of CSF clearance (Bradbury et al., 1981; Boulton et al., 1999; Boulton et al., 1996; Cserr et al., 1992). Pathways between the nasal passages and the CSF are still important and functional in humans, evidenced by the fact that therapeutics are directly delivered to the CSF following intranasal delivery, without entering the blood to an appreciable extent (Born et al., 2002). A number of intranasal studies demonstrate that drugs gain direct access to the CSF from the nasal cavity, followed by subsequent distribution to the brain and spinal cord. Many intranasally applied molecules rapidly enter the CSF, and this transport is dependent on the lipophilicity, molecular weight, and degree of ionization of the molecules (Dhanda et al., 2005; Born et al., 2002; Kumar et al., 1974; Sakane et al., 1995; Sakane et al., 1994; Wang et al., 2007). Assessing distribution into the CSF can provide information on the mechanism of intranasal delivery.

Optimal delivery to the CNS along neural pathways is associated with delivery of the agent to the upper third of the nasal cavity (Hanson et al., 2008). Although a supine position may be employed another position for targeting the olfactory region is with the "praying to Mecca" position, with the head down-and-forward. A supine position with the head angle at 70° or 90° may be suitable for efficient delivery to the CSF using a tube inserted into the nostrils to deliver the drug via intranasal administration (van den Berg et al., (2002)).

For intranasal drug administration nose drops may be administered over a period of 10-20 minutes to alternating nostrils every 1-2 minutes to allow the solution to be absorbed into the nasal epithelium (Thorne et al., 2004; Capsoni et al., 2002; Ross et al., 2004; Ross et al., 2008; Dhuria et al., 2009a; Dhuria et al., 2009b; Francis et al., 2008; Martinez et al., 2008). This noninvasive method does not involve inserting the device into the nostril. Instead, drops are placed at the opening of the nostril, allowing the individual to sniff the drop into the nasal cavity. Other administration methods in anesthetized individual involve sealing the esophagus and inserting a breathing tube into the trachea to prevent the nasal formulation from being swallowed and to eliminate issues related to respiratory distress (Chow et al., 1999; Chow et al., 2001; Fliedner et al., 2006; Dahlin et al., 2001). Flexible tubing can be inserted into the nostrils for localized delivery of a small volume of the drug solution to the respiratory or olfactory epithelia, depending on the length of the tubing (Chow et al., 1999; Van den Berg et al., 2003; van den Berg et al., 2004a; Banks et al., 2004; van den Berg et al., 2002; Vyas et al., 2006a; Charlton et al., 2007a; Gao et al., 2007a).

Nasal delivery devices, such as sprays, nose droppers or needle-less syringes, may be employed to target the agent to different regions of the nasal cavity. OptiMist™ is a breath actuated device that targets liquid or powder nasal formulations to the nasal cavity, including the olfactory region, without deposition in the lungs or esophagus (Djupesland et al., 2006). The ViaNase™ device can also be used to target a nasal spray to the olfactory and respiratory epithelia of the nasal cavity. Nasal drops tend to deposit on the nasal floor and are subjected to rapid mucociliary clearance, while nasal sprays are distributed to the middle meatus of the nasal mucosa (Scheibe et al., 2008).

The immune suppressant or immunotolerizing agent may be administered by any route including parenterally. In one embodiment, the immune suppressant or immunotolerizing agent may be administered by subcutaneous, intramuscular, or intravenous injection, orally, intrathecally, intracranially, or intranasally, or by sustained release, e.g., using a subcutaneous implant. The immune suppressant or immunotolerizing agent may be dissolved or dispersed in a liquid carrier vehicle. For parenteral administration, the active material may be suitably admixed with an acceptable vehicle, e.g., of the vegetable oil variety such as peanut oil, cottonseed oil and the like. Other parenteral vehicles such as organic compositions using solketal, glycerol, formal, and aqueous parenteral formulations may also be used. For parenteral application by injection, compositions may comprise an aqueous solution of a water soluble pharmaceutically acceptable salt of the active acids according to the invention, desirably in a concentration of 0.01-10%, and optionally also a stabilizing agent and/or buffer substances in aqueous solution. Dosage units of the solution may advantageously be enclosed in ampules.

The composition, e.g., rAAV containing composition, immune suppressant containing composition or immunotolerizing composition, may be in the form of an injectable unit dose. Examples of carriers or diluents usable for preparing such injectable doses include diluents such as water, ethyl alcohol, macrogol, propylene glycol, ethoxylated isostearyl alcohol, polyoxyisostearyl alcohol and polyoxyethylene sorbitan fatty acid esters, pH adjusting agents or buffers such as sodium citrate, sodium acetate and sodium phosphate, stabilizers such as sodium pyrosulfite, EDTA, thioglycolic acid and thiolactic acid, isotonic agents such as sodium chloride and glucose, local anesthetics such as procaine hydrochloride and lidocaine hydrochloride. Furthermore, usual solubilizing agents and analgesics may be added. Injections can be prepared by adding such carriers to the enzyme or other active, following procedures well known to those of skill in the art. A thorough discussion of pharmaceutically acceptable excipients is available in REMINGTON'S PHARMACEUTICAL SCIENCES (Mack Pub. Co., N.J. 1991). The pharmaceutically acceptable formulations can easily be suspended in aqueous vehicles and introduced through conventional hypodermic needles or using infusion pumps. Prior to introduction, the formulations can be sterilized with, preferably, gamma radiation or electron beam sterilization.

When the immune suppressant or immunotolerizing agent is administered in the form of a subcutaneous implant, the compound is suspended or dissolved in a slowly dispersed material known to those skilled in the art, or administered in a device which slowly releases the active material through the use of a constant driving force such as an osmotic pump. In such cases, administration over an extended period of time is possible.

The dosage at which the immune suppressant or immunotolerizing agent containing composition is administered may vary within a wide range and will depend on various factors such as the severity of the disease, the age of the patient, etc., and may have to be individually adjusted. A possible range for the amount which may be administered per day is about 0.1 mg to about 2000 mg or about 1 mg to about 2000 mg. The compositions containing the immune suppressant or immunotolerizing agent may suitably be formulated so that they provide doses within these ranges, either as single dosage units or as multiple dosage units. In addition to containing an immune suppressant, the subject formulations may contain one or more rAAV encoding a therapeutic gene product.

Compositions described herein may be employed in combination with another medicament. The compositions can appear in conventional forms, for example, aerosols, solutions, suspensions, or topical applications, or in lyophilized form.

Typical compositions include a rAAV, an immune suppressant, a permeation enhancer, or a combination thereof, and a pharmaceutically acceptable excipient which can be a carrier or a diluent. For example, the active agent(s) may be mixed with a carrier, or diluted by a carrier, or enclosed within a carrier. When the active agent is mixed with a carrier, or when the carrier serves as a diluent, it can be solid, semi-solid, or liquid material that acts as a vehicle, excipient, or medium for the active agent. Some examples of suitable carriers are water, salt solutions, alcohols, polyethylene glycols, polyhydroxyethoxylated castor oil, peanut oil, olive oil, gelatin, lactose, terra alba, sucrose, dextrin, magnesium carbonate, sugar, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose and polyvinylpyrrolidone. Similarly, the carrier or diluent can include any sustained release material known in the art, such as glyceryl monostearate or glyceryl distearate, alone or mixed with a wax.

The formulations can be mixed with auxiliary agents which do not deleteriously react with the active agent(s). Such additives can include wetting agents, emulsifying and suspending agents, salt for influencing osmotic pressure, buffers and/or coloring substances preserving agents, sweetening agents or flavoring agents. The compositions can also be sterilized if desired.

If a liquid carrier is used, the preparation can be in the form of a liquid such as an aqueous liquid suspension or solution. Acceptable solvents or vehicles include sterilized water, Ringer's solution, or an isotonic aqueous saline solution.

The agent(s) may be provided as a powder suitable for reconstitution with an appropriate solution as described above. Examples of these include, freeze dried, rotary dried or spray dried powders, amorphous powders, granules, precipitates, or particulates. The composition can optionally contain stabilizers, pH modifiers, surfactants, bioavailability modifiers and combinations of these. A unit dosage form can be in individual containers or in multi-dose containers.

Compositions contemplated by the present invention may include, for example, micelles or liposomes, or some other encapsulated form, or can be administered in an extended release form to provide a prolonged storage and/or delivery effect, e.g., using biodegradable polymers, e.g., polylactide-polyglycolide. Examples of other biodegradable polymers include poly(orthoesters) and poly(anhydrides).

Polymeric nanoparticles, e.g., comprised of a hydrophobic core of polylactic acid (PLA) and a hydrophilic shell of methoxy-poly(ethylene glycol) (MPEG), may have improved solubility and targeting to the CNS. Regional differences in targeting between the microemulsion and nanoparticle formulations may be due to differences in particle size.

Liposomes are very simple structures consisting of one or more lipid bilayers of amphiphilic lipids, i.e., phospholipids or cholesterol. The lipophilic moiety of the bilayers is turned towards each other and creates an inner hydrophobic environment in the membrane. Liposomes are suitable drug carriers for some lipophilic drugs which can be associated with the non-polar parts of lipid bilayers if they fit in size and geometry. The size of liposomes varies from 20 nm to few µm.

Mixed micelles are efficient detergent structures which are composed of bile salts, phospholipids, tri, di- and monoglycerides, fatty acids, free cholesterol and fat soluble micronutrients. As long-chain phospholipids are known to form bilayers when dispersed in water, the preferred phase of short chain analogues is the spherical micellar phase. A micellar solution is a thermodynamically stable system formed spontaneously in water and organic solvents. The interaction between micelles and hydrophobic/lipophilic drugs leads to the formation of mixed micelles (MM), often called swallen micelles, too. In the human body, they incorporate hydrophobic compounds with low aqueous solubility and act as a reservoir for products of digestion, e.g. monoglycerides.

Lipid microparticles includes lipid nano- and microspheres. Microspheres are generally defined as small spherical particles made of any material which are sized from about 0.2 to 100 µm. Smaller spheres below 200 nm are usually called nanospheres. Lipid microspheres are homogeneous oil/water microemulsions similar to commercially available fat emulsions, and are prepared by an intensive sonication procedure or high pressure emulsifying methods (grinding methods). The natural surfactant lecithin lowers the surface tension of the liquid, thus acting as an emulsifier to form a stable emulsion. The structure and composition of lipid nanospheres is similar to those of lipid microspheres, but with a smaller diameter.

Polymeric nanoparticles serve as carriers for a broad variety of ingredients. The active components may be either dissolved in the polymetric matrix or entrapped or adsorbed onto the particle surface. Polymers suitable for the preparation of organic nanoparticles include cellulose derivatives and polyesters such as poly(lactic acid), poly(glycolic acid) and their copolymer. Due to their small size, their large surface area/volume ratio and the possibility of functionalization of the interface, polymeric nanoparticles are ideal carrier and release systems. If the particle size is below 50 nm, they are no longer recognized as particles by many biological and also synthetic barrier layers, but act similar to molecularly disperse systems.

Thus, the composition of the invention can be formulated to provide quick, sustained, controlled, or delayed release, or any combination thereof, of the active agent after administration to the individual by employing procedures well known in the art. In one embodiment, the enzyme is in an isotonic or hypotonic solution. In one embodiment, for enzymes that are not water soluble, a lipid based delivery vehicle may be employed, e.g., a microemulsion such as that described in WO 2008/049588, or liposomes.

In one embodiment, the preparation can contain an agent, dissolved or suspended in a liquid carrier, such as an aqueous carrier, for aerosol application. The carrier can contain additives such as solubilizing agents, e.g., propylene glycol, surfactants, absorption enhancers such as lecithin (phosphatidylcholine) or cyclodextrin, or preservatives such as parabens. For example, in addition to solubility, efficient delivery to the CNS following intranasal administration may be dependent on membrane permeability. For enzymes where paracellular transport is hindered due to size and polarity, improving membrane permeability may enhance extracellular mechanisms of transport to the CNS along olfactory and trigeminal nerves. One approach to modifying membrane permeability within the nasal epithelium is by using permeation enhancers, such as surfactants, e.g., lauroylcarnitine (LC), bile salts, lipids, cyclodextrins, polymers, or tight junction modifiers.

Generally, the active agents are dispensed in unit dosage form including the active ingredient together with a pharmaceutically acceptable carrier per unit dosage. Usually, dosage forms suitable for nasal administration include from about 125 µg to about 125 mg, e.g., from about 250 µg to about 50 mg, or from about 2.5 mg to about 25 mg, of the compounds admixed with a pharmaceutically acceptable carrier or diluent.

Dosage forms can be administered daily, or more than once a day, such as twice or thrice daily. Alternatively, dosage forms can be administered less frequently than daily, such as every other day, or weekly, if found to be advisable by a prescribing physician.

### Example I

### AAV Vector-Mediated Iduronidase Gene Delivery in a Murine Model of Mucopolysaccharidosis Type I: Comparing Different Routes of Delivery to the CNS

Mucopolysaccharidosis type I (MPS I) is an inherited metabolic disorder caused by deficiency of the lysosomal enzyme alpha-L-iduronidase (IDUA). Systemic and abnormal accumulation of glycosaminoglycans is associated with growth delay, organomegaly, skeletal dysplasia, and cardiopulmonary disease. Individuals with the most severe form of the disease (Hurler syndrome) suffer from neurodegeneration, mental retardation, and early death. The two current treatments for MPS I (hematopoietic stem cell transplantation and enzyme replacement therapy) cannot effectively treat all central nervous system (CNS) manifestations of the disease.

With respect to gene therapy, it was previously demonstrated that intravascular delivery of AAV9 in adult mice does not achieve widespread direct neuronal targeting (see Foust et al, 2009). Previous work also showed that direct injection of AAV8-IDUA into the CNS of adult IDUA-deficient mice resulted in a low frequency or a poor level of transgene expression (see Figure 18). The following examples, which use a pre-clinical model for the treatment of MPS1, surprisingly demonstrate that direct injection of AAV9-IDUA into the CNS of immunocompetent adult IDUA-deficient mice resulted in IDUA enzyme expression and activity that is the same or higher than IDUA enzyme expression and activity in wild-type adult mice (see Figure 15, *infra*).

### Methods

AAV9-IDUA preparation. The AAV-IDUA vector construct (MCI) has been previously described (Wolf et al., 2011) (mCags promoter). AAV-IDUA plasmid DNA was packaged into AAV9 virions at the University of Florida Vector Core, yielding a titer of 3 x 10¹³ vector genomes per milliliter.

ICV infusions. Adult Idua-/- mice were anesthetized using a cocktail of ketamine and xylazine (100 mg ketamine + 10 mg xylazine per kg) and placed on a stereotactic frame. Ten microliters of AAV9-IDUA were infused into the right-side lateral ventricle (stereotactic coordinates AP 0.4, ML 0.8, DV 2.4 mm from bregma) using a Hamilton syringe. The animals were returned to their cages on heating pads for recovery.

Intrathecal infusions. Infusions into young adult mice were carried out by injection of 10 µL AAV vector containing solution between the L5 and L6 vertebrae 20 minutes after intravenous injection of 0.2 mL 25% mannitol.

Immunotolerization. Newborn IDUA deficient mice were injected through the facial temporal vein with 5 µL containing 5.8 µg of recombinant iduronidase protein (Aldurazyme), and then the animals were returned to their cage.

Cyclophosphamide immunosuppression. For immunosuppression, animals were administered cyclophosphamide once per week at a dose of 120 mg/kg starting one day after infusion with AAV9-IDUA vector.

Animals. Animals were anesthetized with ketamine/xylazine (100 mg ketamine + 10 mg xylazine per kg) and transcardially perfused with 70 mL PBS prior to sacrifice. Brains were harvested and microdissected on ice into cerebellum, hippocampus, striatum, cortex, and brainstem/thalamus ("rest"). The samples were frozen on dry ice and then stored at -80°C. Samples were thawed and homogenized in 1 mL of PBS using a motorized pestle and permeabilized with 0.1 % Triton X-100. IDUA activity was determined by fluorometric assay using 4MU-iduronide as the substrate. Activity is expressed in units (percent substrate converted to product per minute) per mg protein as determined by Bradford assay (BioRad).

Tissues. Tissue homogenates were clarified by centrifugation for 3 minutes at 13,000 rpm using an Eppendorf tabletop centrifuge model 5415D (Eppendorf) and incubated overnight with proteinase K, DNase1, and Rnase. GAG concentration was determined using the Blyscan Sulfated Glycosaminoglycan Assay (Accurate Chemical) according to the manufacturer's instructions.

### Results

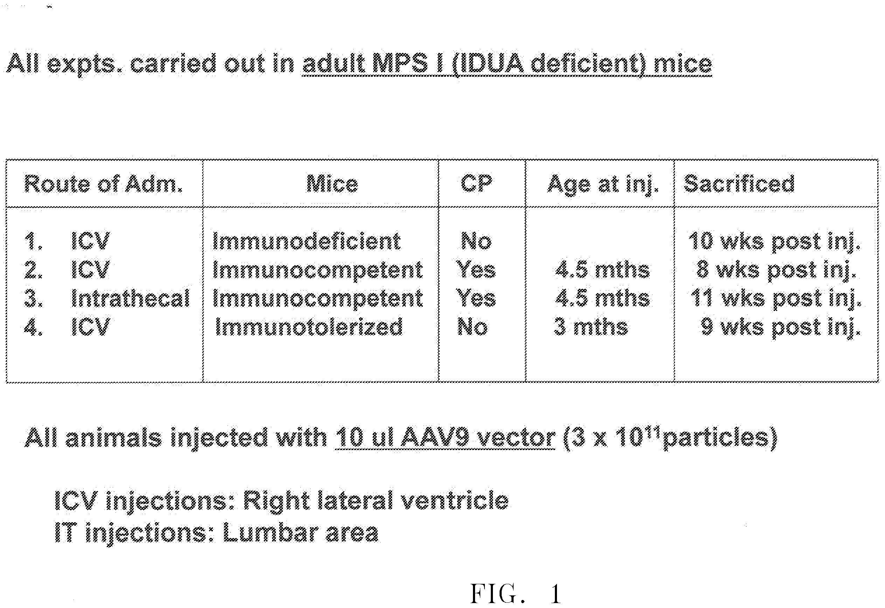

Figure 1 shows the experimental design for iduronidase-deficient mice that were administered AAV either intracerebroventricularly (ICV) or intrathecally (IT). To prevent immune response, animals were either immunosuppressed with cyclophosphamide (CP), immunotolerized at birth by intravenous administration of human iduonidase protein (aldurazyme), or the injections were carried out in NOD-SCID immunodeficient mice that were also iduronidase deficient. Animals were sacrificed at the indicated time post-treatment, the brains were microdissected and extracts assayed for iduronidase activity.

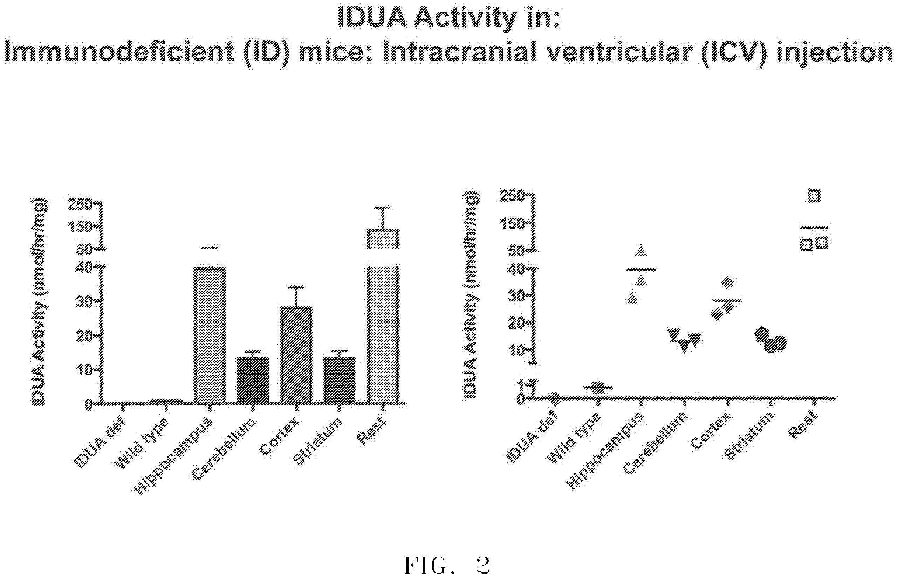

Figure 2 illustrates data for immunodeficient, IDUA deficient animals injected ICV with AAV-IDUA vector. Those animals exhibited high levels of IDUA expression (10 to 100 times wild type) in all areas of the brain, with the highest level observed in the brain stem and thalamus ("rest").

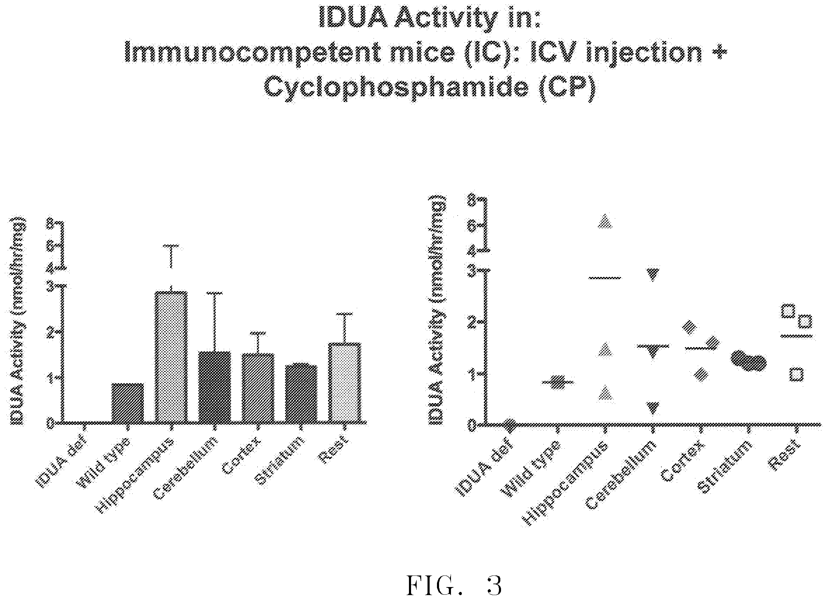

Immunosuppressed animals administered AAV vector by ICV route had a relatively lower level of enzyme in the brain compared to the immunodeficent animals (Figure 3). Note that immunosuppression may have been compromised in these animals because CP was withdrawn 2 weeks before sacrifice due to poor health.

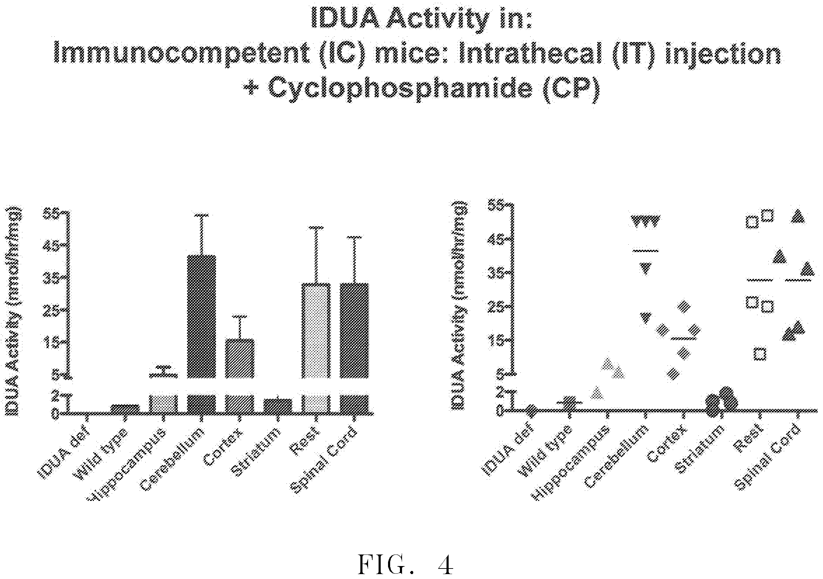

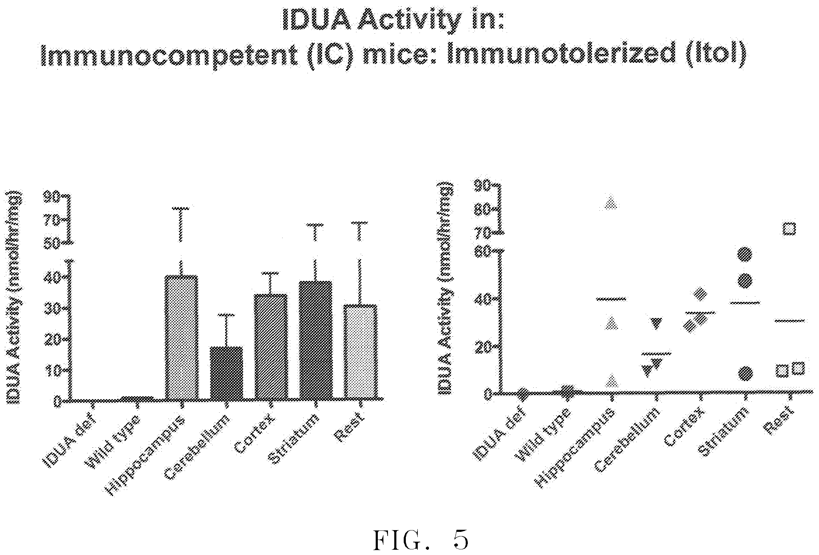

Figure 4 shows data for immunosuppressed animals administered AAV vector by the IT route. Immunotolerized animals administered AAV vector ICV exhibited widespread IDUA activity in all parts of the brain (Figure 5), similar to that observed in the immunodeficient animals, indicating the effectiveness of the immunotolerization procedure.

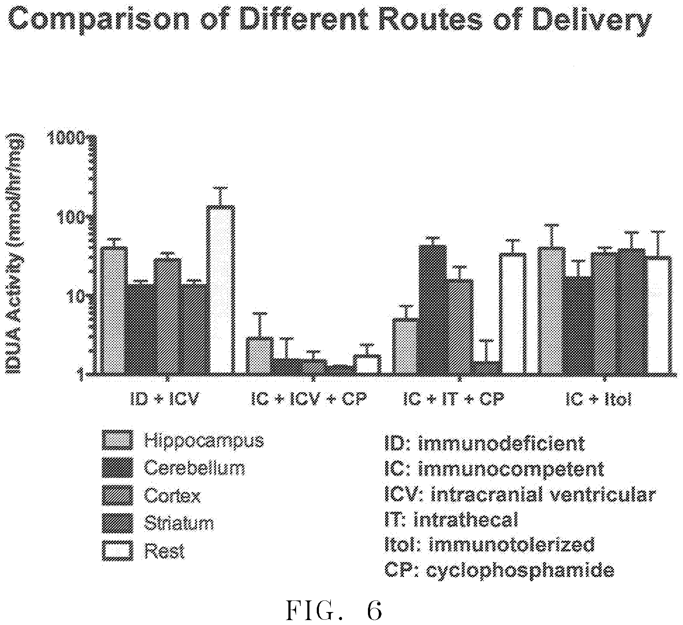

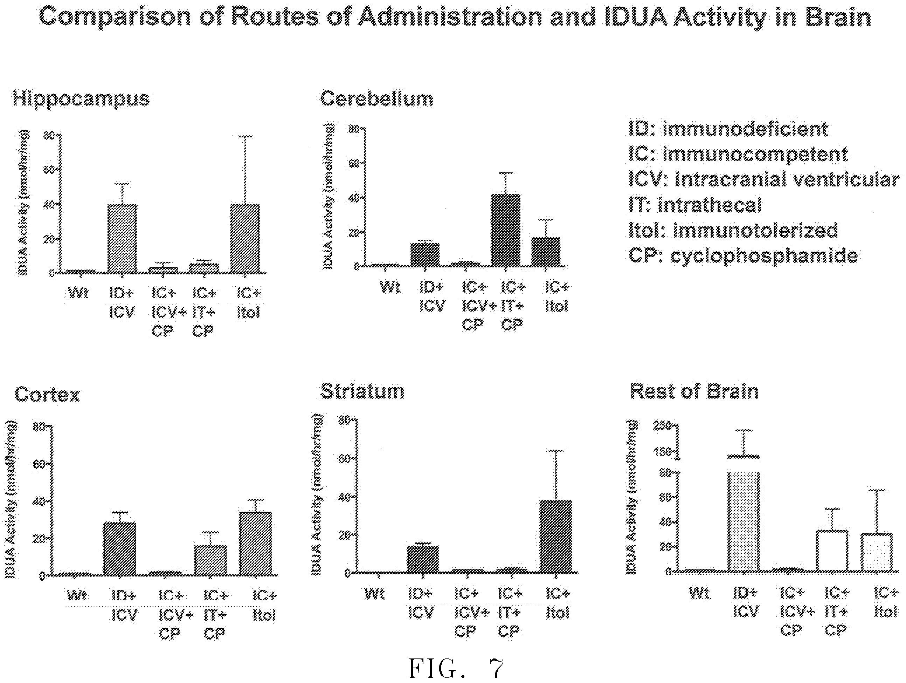

Figure 6 is a compilation of all mean levels of IDUA activity for side-by-side comparison, and Figure 7 is data grouped according the area of the brain.

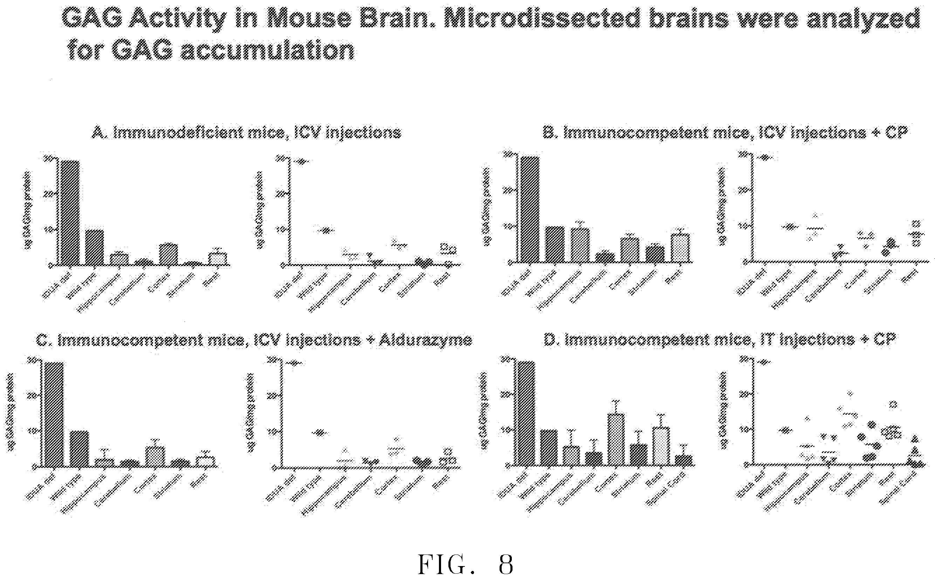

GAG storage material was assayed in the different sections of the brain for all four of the test groups. For each group, the mean of each portion of the brain is shown on the left, the values for each of the individual animals is shown on the right (Figure 8). IDUA deficient animals (far left) contained high levels of GAG compared to wild type animals (magenta bar). GAG levels were at wild-type or lower than wild type for all portions of the brain in all groups of AAV-treated animals. GAG levels were slightly although not significantly higher than wild-type in cortex and brainstem of animals administered AAV9-IDUA intrathecally.

### Conclusions

The results show high and widespread distribution of IDUA in the brain regardless of the route of delivery (ICV or IT) although IDUA expression in striatum and hippocampus was lower in animals injected IT versus ICV. There appears to be an immune response since immune deficient mice have higher levels of expression than immunocompetent mice. With regard to ICV injection, when CP was withdrawn early, IDUA expression is lower. In addition, immunotolerization was effective in restoring high levels of enzyme activity. Further, GAG levels were restored to normal in all treated experimental groups of mice.

### Example II

### Methods

AAV9-IDUA Preparation. AAV-IDUA plasmid was packaged into AAV9 virions at either the University of Florida vector core, or the University of Pennsylvania vector core, yielding a titer of 1-3 x 10¹³ vector genomes per milliliter.

ICV infusions. See Example I.

Intrathecal infusions. See Example I.

Immunotolerization. As in Example I except: for multiple tolerizations, newborn IDUA deficient mice were injected with the first dose of Aldurazyme in the facial temporal vein, followed by 6 weekly injections administered intraperitoneally.

Cyclophosphamide immunosuppression. See Example I.

Animals. Animals were anesthetized with ketamine/xylazine (100 mg ketamine + 10 mg xylazine per kg) and transcardially perfused with 70 mL PBS prior to sacrifice. Brains were harvested and microdissected on ice into cerebellum, hippocampus, striatum, cortex, and brainstem/thalamus ("rest"). The samples were frozen on dry ice and then stored at -80°C.

Tissue IDUA activity. Tissue samples were thawed and homogenized in saline in a tissue homogenizer. Tissue homogenates were clarified by centrifugation at 15,000 rpm in a benchtop Eppendorf centrifuge at 4°C for 15 minutes. Tissue lysates (supernatant) were collected and analyzed for IDUA activity and GAG storage levels.

Tissue GAG levels. Tissue lysates were incubated overnight with Proteinase K, RNase and DNase. GAG levels were analyzed using the Blyscan Sulfated Glycosaminoglycan Assay according to the manufacturer's instructions.

IDUA Vector copies. Tissue homogenates were used for DNA isolation and subsequent QPCR, as described in Wolf et al. (2011).

### Results

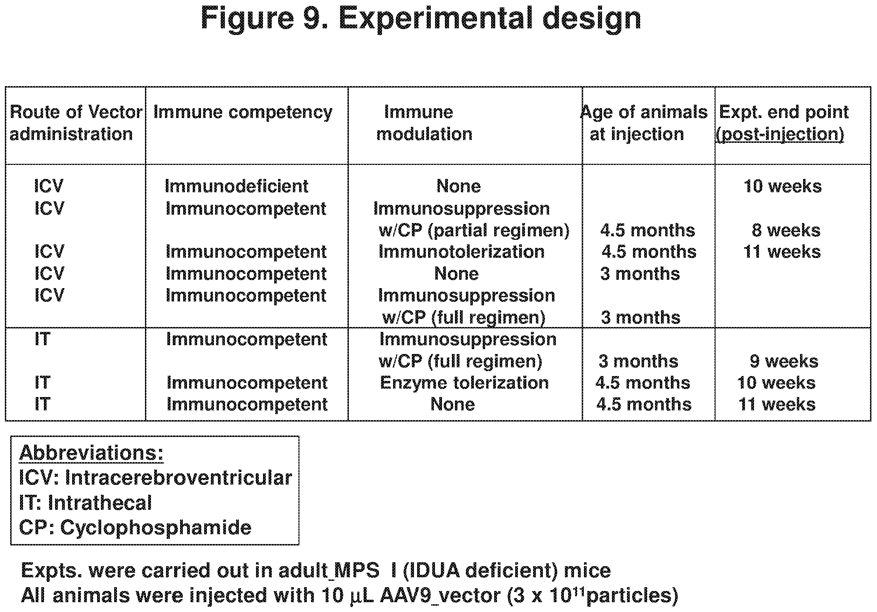

Figure 9 illustrates the experimental design and groups. Animals were administered AAV9-IDUA vector either by intracerebroventricular (ICV) or intrathecal (IT) infusion. Vector administration was carried out in NOD-SCID immunodeficient (ID) mice that were also IDUA deficient, or in IDUA deficient mice that were either immunosuppressed with cyclophosphamide (CP), or immunotolerized at birth by a single or multiple injections of human iduronidase protein (Aldurazyme). The times of treatment with vector and sacrifice are as indicated in Figure 9. All vector administrations were carried out in adult animals ranging in age from 3-4.5 months. Animals were injected with 10 µL of vector at a dose of 3 x 10¹¹ vector genomes per 10 microliters.

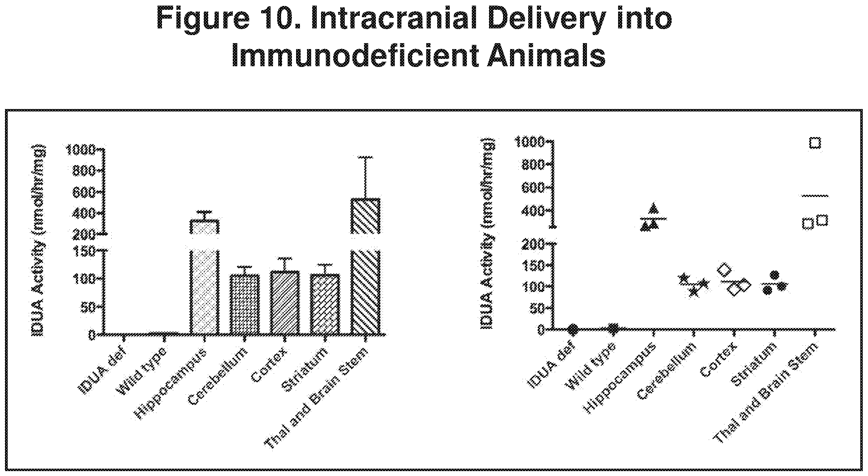

Figure 10 shows IDUA enzyme activities in intracranially infused, immunodeficient, IDUA deficient mice. High levels of enzyme activity were seen in all areas of the brain, ranging from 30- to 300-fold higher than wild type levels. Highest enzyme expressions were seen in thalamus and brain stem, and in the hippocampus.

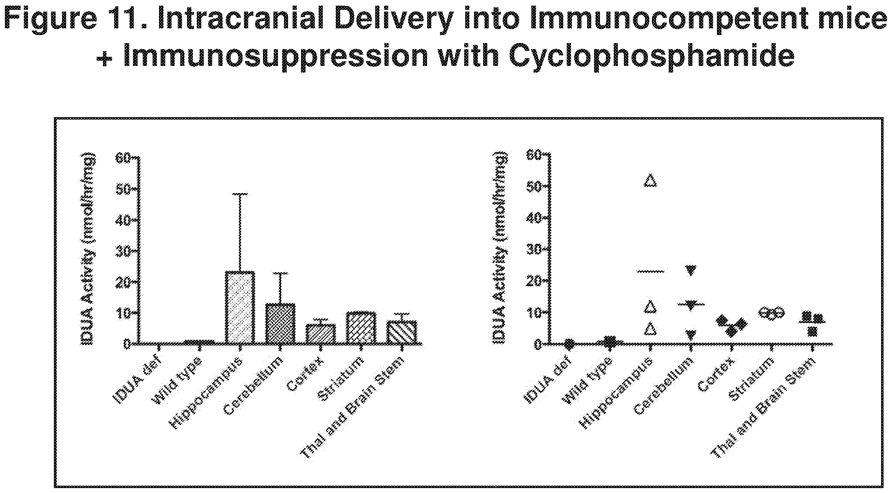

Animals that were injected intracranially and immunosuppressed with cyclophosphamide (CP) demonstrated significantly lower levels of enzyme activity than other groups (Figure 11). However, CP administration in this case had to be withdrawn 2 weeks prior to sacrifice due to poor health of the animals.

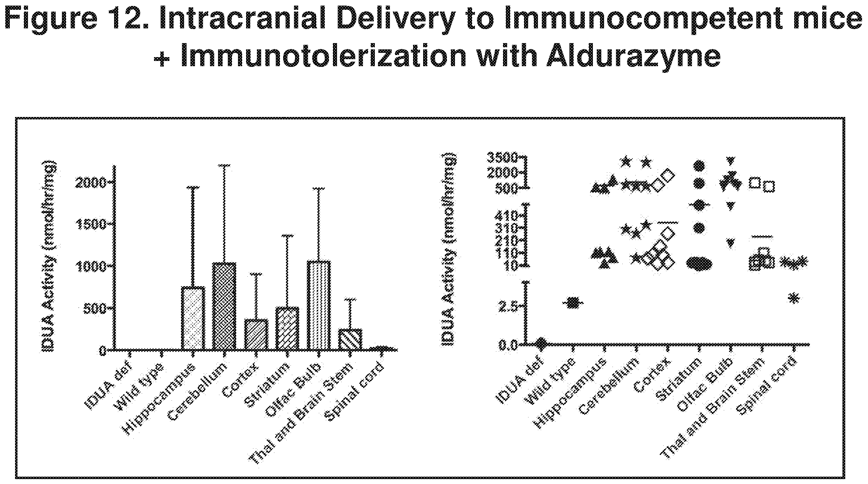

IDUA enzyme levels in animals tolerized at birth with IDUA protein (Aldurazyme) and administered vector intracranially are depicted in Figure 12. All animals showed high enzyme levels in all parts of the brain that ranged from 10- to 1000-fold higher than wild type levels, similar to levels achieved in immunodeficient animals, indicating the effectiveness of the immunotolerization procedure.

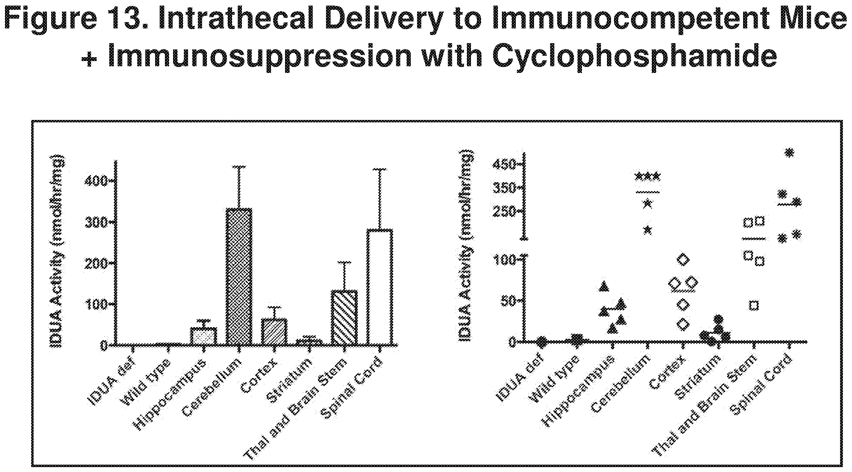

Figure 13 depicts IDUA enzyme levels in mice that were injected intrathecally and administered CP on a weekly basis. Elevated levels of IDUA were observed in all parts of the brain, especially in the cerebellum and the spinal cord. Levels of enzyme were the lowest in the striatum and hippocampus with activities at wild type levels.

IDUA deficient mice were tolerized with Aldurazyme as described, and injected with vector intrathecally (Figure 14). There was widespread IDUA enzyme activity in all parts of the brain, with highest levels of activity in the brain stem and thalamus, olfactory bulb, spinal cord and the cerebellum. Similar to the data in Figure 13, the lowest levels of enzyme activity were seen in the striatum, cortex and hippocampus.

Control immunocompetent IDUA deficient animals were infused with vector intrathecally, without immunosuppression or immunotolerization (Figure 15). The results indicate that although enzyme activities were at wild type levels or slightly higher, they are significantly lower than what was observed in animals that underwent immunomodulation. The decreases in enzyme levels were especially significant in the cerebellum, olfactory bulb and thalamus and brain stem, areas that expressed the highest levels of enzyme in immunomodulated animals.

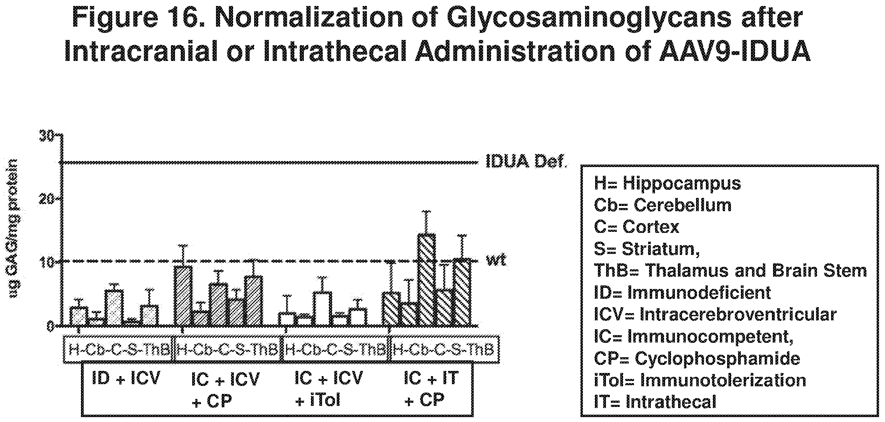

Animals were assayed for GAG storage material, as shown in Figure 16. All groups demonstrated clearance of GAG storage, with GAG levels similar to that observed in wild type animals. Animals that were immunosuppressed and injected with AAV9-IDUA vector intrathecally had GAG levels in the cortex that were slightly higher than wild type, but still much lower than untreated IDUA deficient mice.

The presence of AAV9-IDUA vector in animals that were immunotolerized and injected with vector either intracranially or intrathecally was evaluated by QPCR, as illustrated in Figure 16. IDUA copies per cell were higher in animals infused intracranially in comparison with animals infused intrathecally, which is consistent with the higher level of enzyme activity seen in animals injected intracranially.

### Conclusions

High, widespread, and therapeutic levels of IDUA were observed in all areas of the brain after intracerebroventricular and intrathecal routes of AAV9-IDUA administration in adult mice. Enzyme activities were restored to wild type levels or slightly higher in immunocompetent IDUA deficient animals infused with AAV-IDUA intrathecally. Significantly higher levels of IDUA enzyme were observed for both routes of vector injection in animals immunotolerized starting at birth by administration of IDUA protein.

### Example III

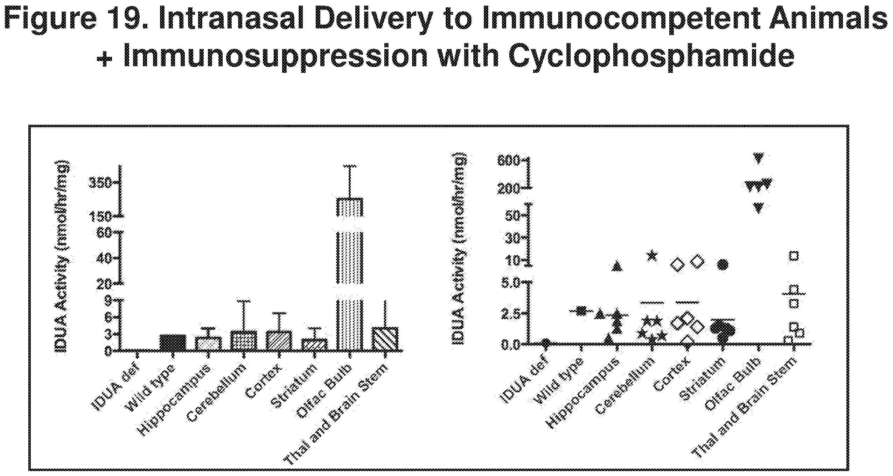

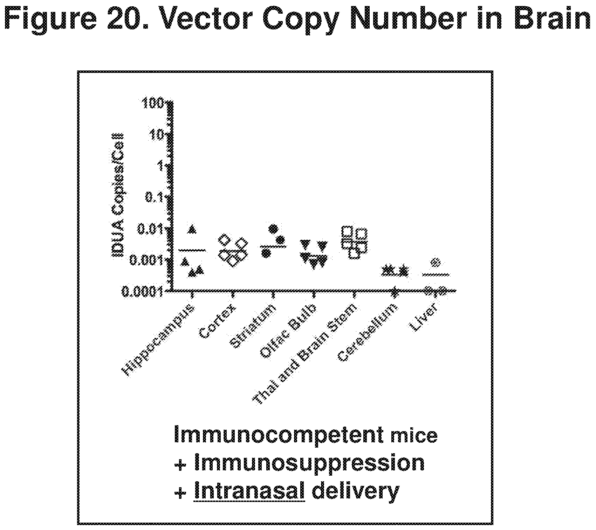

Adult immunocompetent IDUA deficient mice (12 weeks old) were anesthetized with ketamine/xylazine, followed by intranasal infusion of AAV9-IDUA vector. Vector was administered by applying eight 3 µL drops with a micropipette to the intranasal cavity, alternating between nostrils, at 2 minute intervals between each application. A total of 2.4-7 x 10¹¹ vector genomes was administered to each adult animal, depending on source of vector. In order to suppress the mouse immune response to human IDUA produced by the AAV9-IDUA vector, animals were immunosuppressed with 120 mg/kg cyclophosphamide administered weekly, starting the day after vector administration. However, immunosuppression in human subjects is optional and the skilled artisan, in accordance with good/standard medical practice, would know when to employ it. Mice were sacrificed at 12 weeks post vector infusion, animals were assayed for IDUA enzyme expression and vector copies in the brain (Figures 19 and 20).

### Example IV

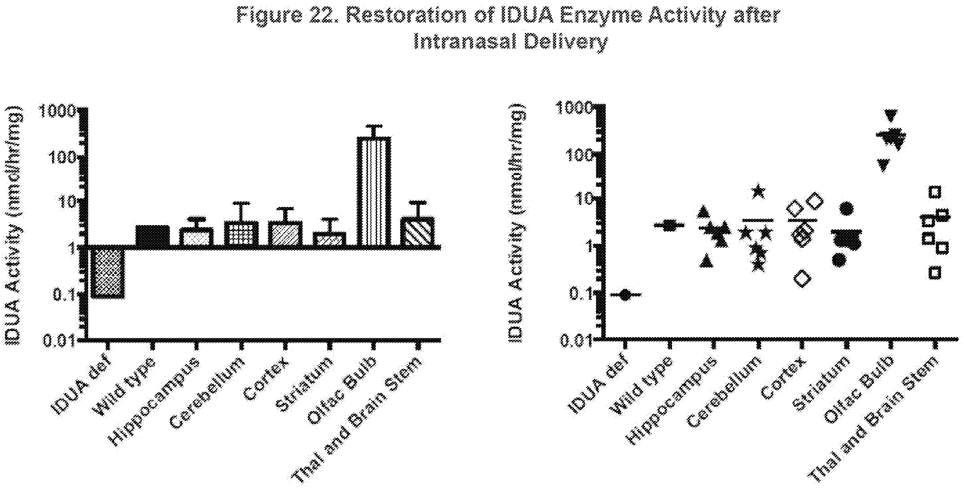

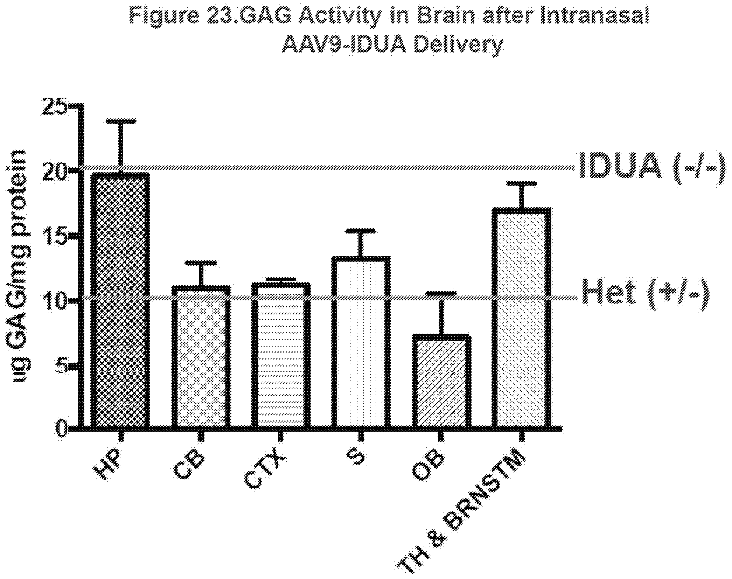

Iduronidase-deficient mice, a model for human mucopolysaccharidosis type I (MPS I), were administered approximately 10¹¹ vector copies of AAV9-IDUA intranasally. Four weeks later the animals were sacrificed and the brain microdissected and extracted for iduronidase enzyme assay. As shown in Figure 22 (means +/- s.d. on the left, individual animals on the right), a high level of IDUA enzyme activity (nearly 100 times greater than wild-type) was observed in the olfactory bulb, with wild-type levels of enzyme observed in all other areas of the brain. Figure 23 shows GAG activity.

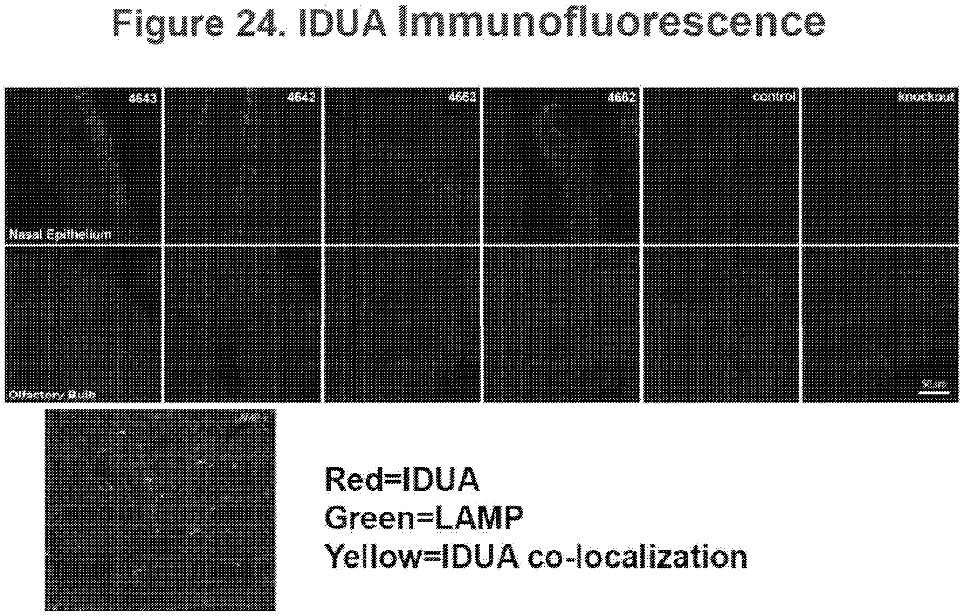

Similarly treated animals were sacrificed and tissue sections were stained for the presence of human IDUA protein using an anti-IDUA antibody. As shown in Figure 24, there was robust staining of IDUA protein observed in the nasal epithelium and in the olfactory bulb of the four intranasal vector-treated animals on the left, while there was no staining observed in control unadministered normal or IDUA-deficient animals on the right. There was no staining observed in other parts of the brains (hippocampus, cerebellum, cortex, striatum, thalamus and brain stem) of AAV-MCI treated animals, demonstrating that transduction was limited to the olfactory bulb and nasal epithelium.

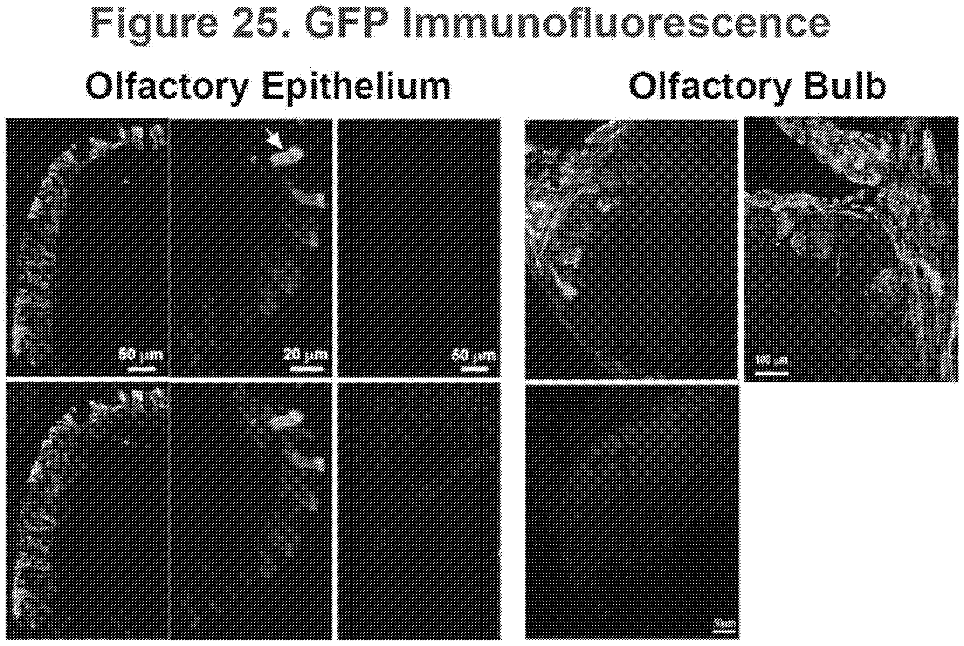

Animals were treated intranasally with approximately 10¹¹ vector genomes of AAV9-GFP vector as a reporter system to identify the location of transduced cell). The animals were sacrificed two weeks later, tissues were collected and sections stained for GFP expression using an anti-GFP antibody (green) along with DAPI staining (blue) to identify cellular nuclei (Figure 25). There was robust staining of GFP protein observed in both the olfactory epithelium (left two panels) and in the olfactory bulb (top two panels) of the treated animals in comparison with untreated controls (right panels for olfactory epithelium and bottom panel for olfactory bulb). There was no GFP staining observed in any other parts of the brain. These results are consistent with the results of IDUA staining, demonstrating that AAV mediated gene transfer and expression was limited to the nasal epithelium and the olfactory bulb after intranasal administration of AAV9-MCI vector. These results implicate diffusion of IDUA expressed at high levels in the forebrain as the mechanism by which wild-type levels of IDUA are achieved in all areas of the brain after intranasal administration of AAV9-MCI vector. This approach for achieving high level therapeutic protein expression in the forebrain by non-invasive intranasal AAV vector administration with subsequent diffusion throughout the brain is applicable not only to the treatment of MPS and related metabolic diseases, but to the treatment of other more common neurologic disorders such as Parkinson's disease and Alzheimer's disease.

### Example V

Mucopolysaccharidosis type II (MPS II; Hunter Syndrome) is an X-linked recessive inherited lysosomal storage disease caused by deficiency of iduronate-2-sulfatase (IDS) and subsequent accumulation of glycosaminoglycans (GAGs) dermatan and heparan sulphate. Affected individuals exhibit a range in severity of manifestations physically, neurologically, and shortened life expectancy. For example, affected individuals exhibit a range in severity of manifestations such as organomegaly, skeletal dysplasias, cardiopulmonary obstruction, neurocognitive deficit, and shortened life expectancy. There is no cure for MPS II at the moment. Current standard of care is enzyme replacement therapy (ELAPSRASE; idursulfase), which is used to manage disease progression. However, enzyme replacement therapy (ERT) does not result in neurologic improvement. As hematopoetic stem cell transplantation (HSCT) has not shown neurologic benefit for MPS II, there is currently no clinical recourse for patients exhibiting neurologic manifestations of this disease, and new therapies are desperately needed.

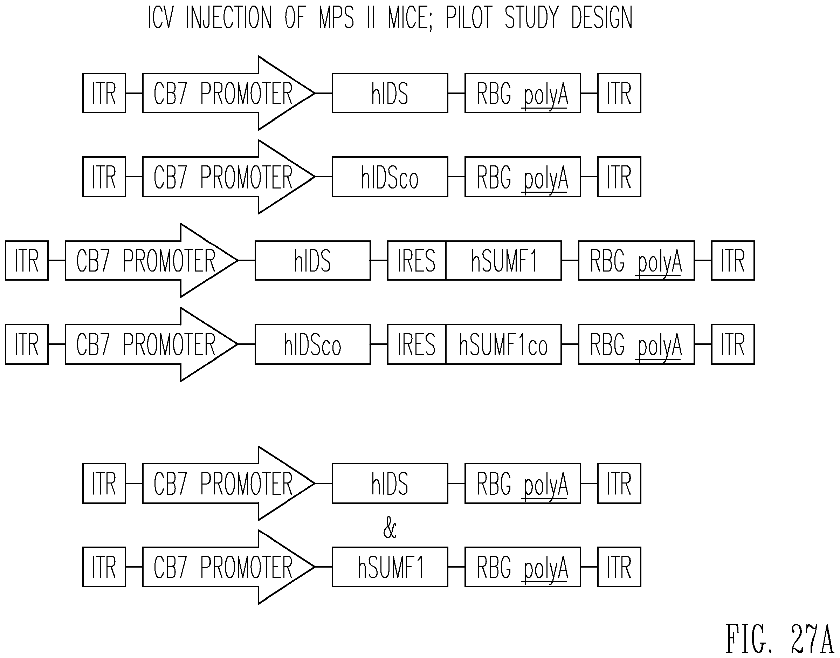

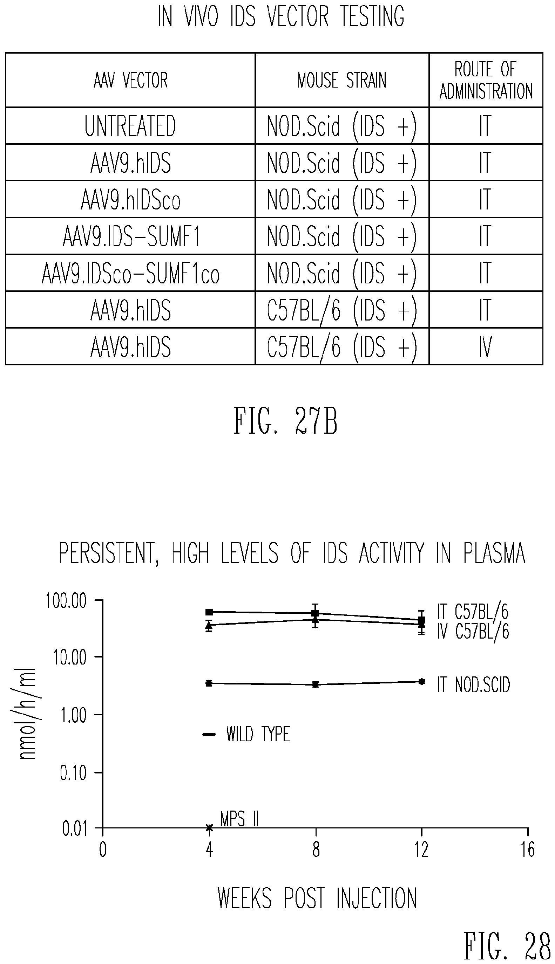

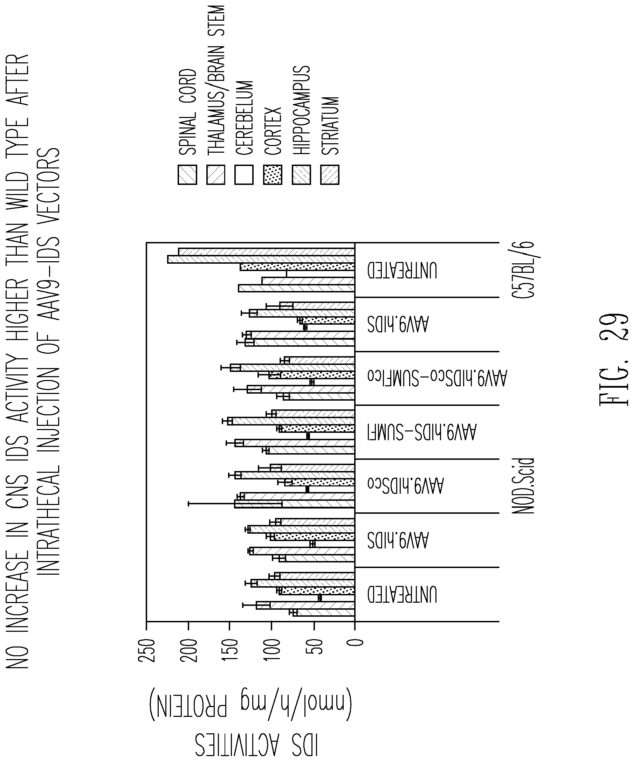

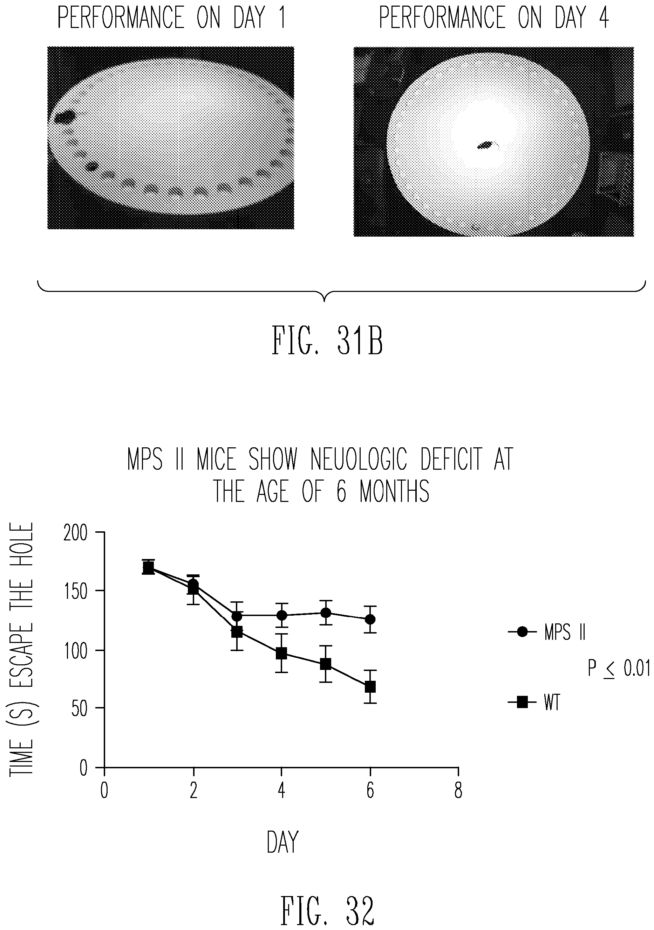

AAV9 vectors are developed for delivery of the human IDS coding sequence (AAV9-hIDS) into the central nervous system of MPS II mice to restore IDS levels in the brain and prevent the emergence of neurocognitive deficits in the treated animals (Figure 27A). In particular, a series of vectors were generated that encode human IDS with or without the human sulfatase modifying factor-1 (SUMF-1), required for activation of the sulfatase active site. Three routes of administration were used in these experiments: Intrathecal (IT) (Figures 28-29), Intracerebroventricular (ICV) (Figures 30A-D) and Intravenous (IV) (Figures 28-29). No significant difference in the enzyme level was found between mice that were treated with AAV9 vector transducing hIDS alone and mice that were treated with AAV9 vector encoding human IDS and SUMF-1, regardless of the route of administration. IT-administrated NOD.SCID (IDS Y+) and C57BL/6 (IDS Y+) did not show elevated IDS activity in the brain and spinal cord when compared to untreated animals, while plasma showed ten-fold higher (NOD.SCID) and 150-fold higher (C57BL/6) levels than untreated animals. IDS-deficient mice intravenously administered AAV9-hIDS exhibited IDS activities in all organs that were comparable to wild type. Moreover, the plasma of IV injected animals showed enzyme activity that was 100-fold higher than wild type. IDS-deficient mice administered AAV9-hIDUA ICV showed IDS activities comparable to wild type in most areas of the brain and peripheral tissues, while some portions of the brain showed two- to four-fold higher activity than wild type. Furthermore, IDS activity in plasma was 200-fold higher than wild type. Surprisingly, IDS enzyme activity in the plasma of all treated animals showed persistence for at least 12 weeks post injection; therefore, IDS enzyme was not immunogenic at least on the C57BL/6 murine background. Additional neurobehavioral testing was conducted using the Barnes maze to differentiate neurocognitive deficits of untreated MPS II animals from that of wild type littermates. It was found that the learning capability of affected animals is distinctively slower than that observed in littermates. Thus, Barnes maze is used to address the benefit of these therapies in the MPS II murine model (Figures 31A-B and 32). These results indicate potential of therapeutic benefit of AAV9 mediated human IDS gene transfer to the CNS to prevent neurologic deficiency in MPS II.

In summary, intracerebroventricular (ICV) injection of AAV9-hIDS resulted in systemic correction of IDS enzyme deficiency, including wild-type levels of IDS in the brain. Co-delivery of hIDS with hSUMF-1 did not increase IDS activity in tissues. hIDS expression was non-immunogenic in WT and MPS II C57BL/6 mice.

### Example VI

Mucopolysaccharidosis type I (MPS I) is an inherited autosomal recessive metabolic disease caused by deficiency of α-L-iduronidase (IDUA), resulting in accumulation of heparin and dermatan sulfate glycosaminoglycans (GAGs). Individuals with the most severe form of the disease (Hurler syndrome) suffer from neurodegeneration, mental retardation, and death by age 10. Current treatments for this disease include allogeneic hematopoietic stem cell transplantation (HSCT) and enzyme replacement therapy (ERT). However, these treatments are insufficiently effective in addressing CNS manifestations of the disease.

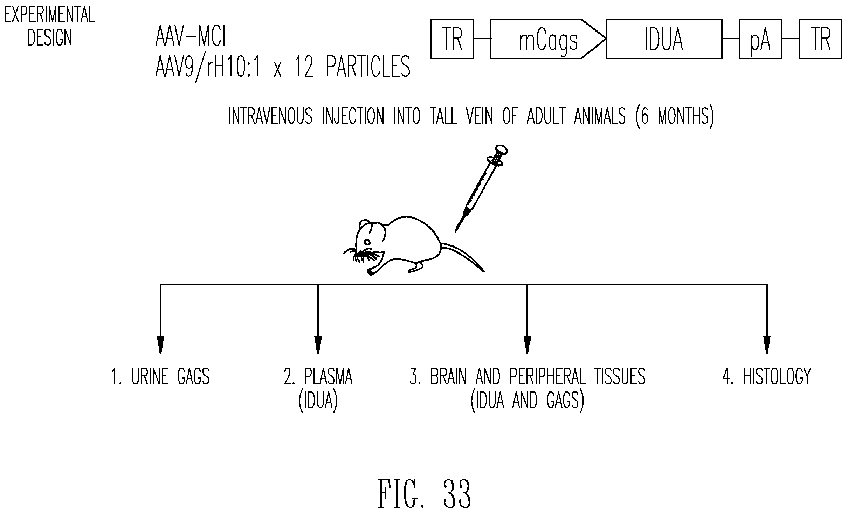

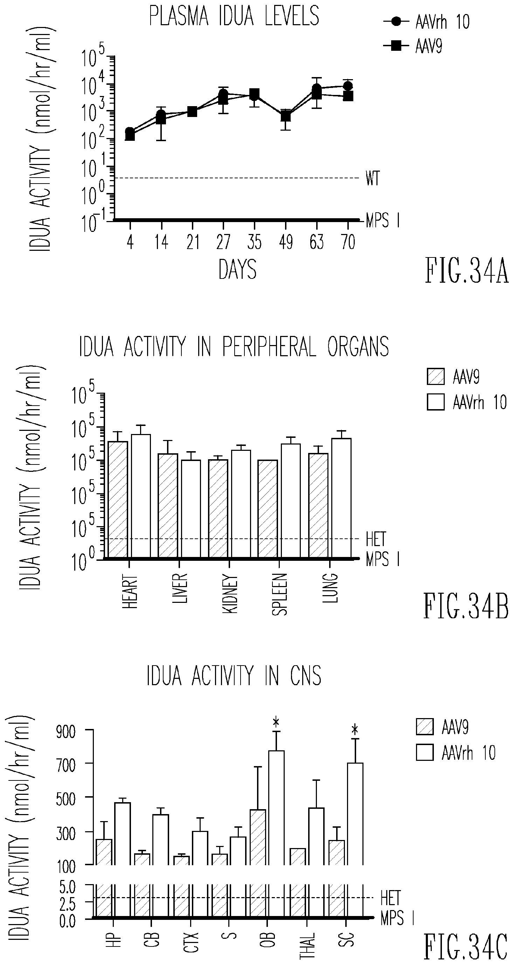

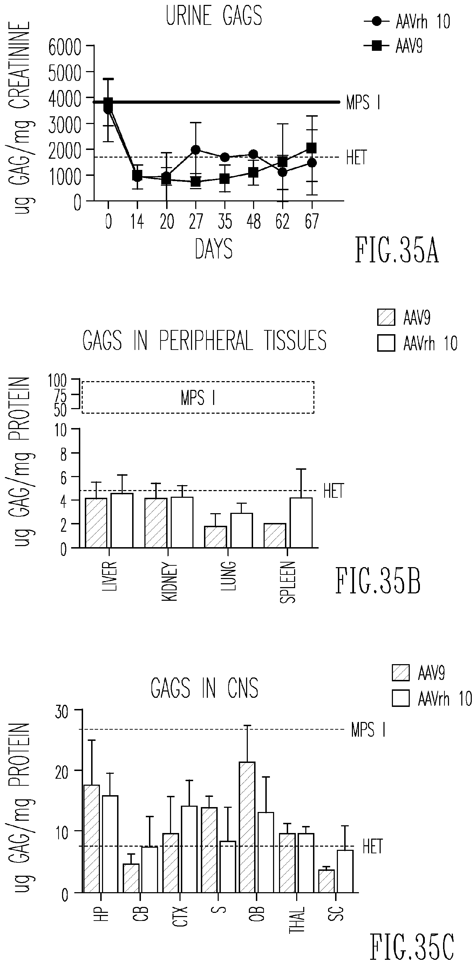

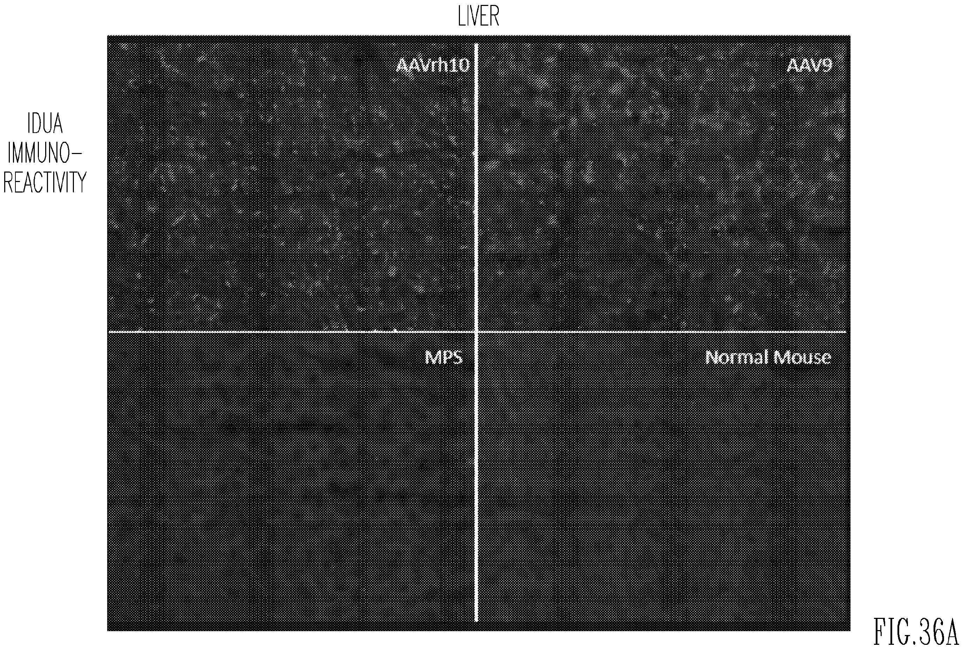

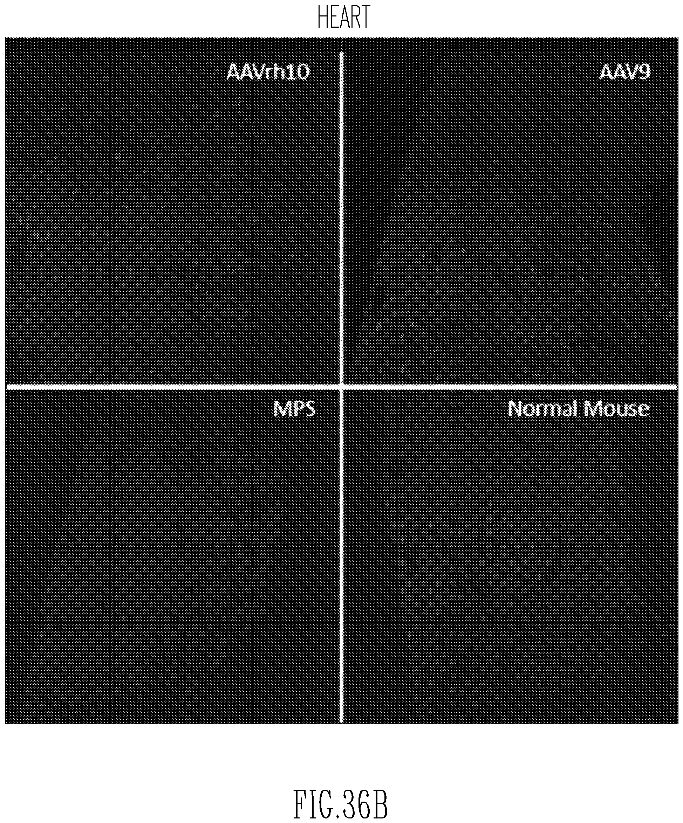

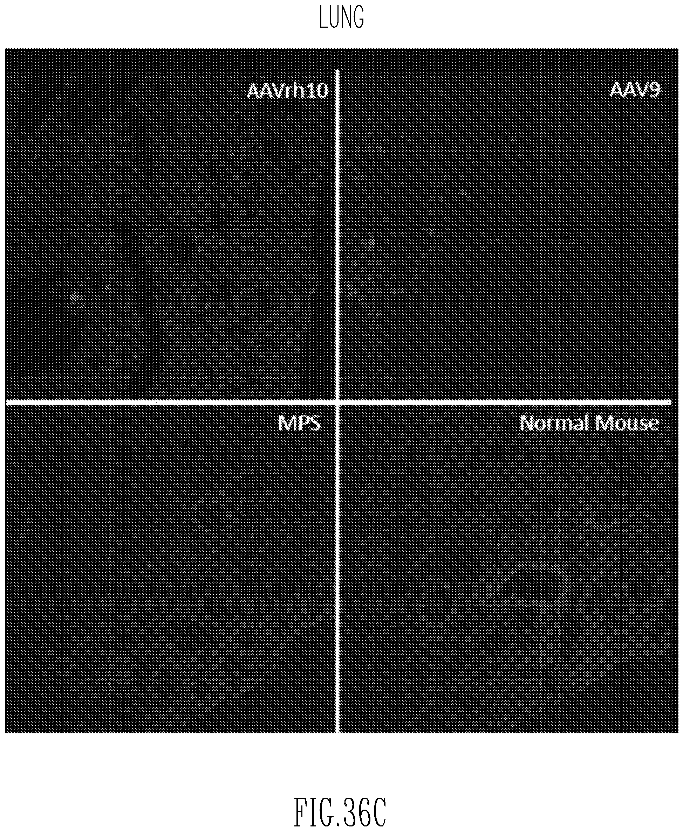

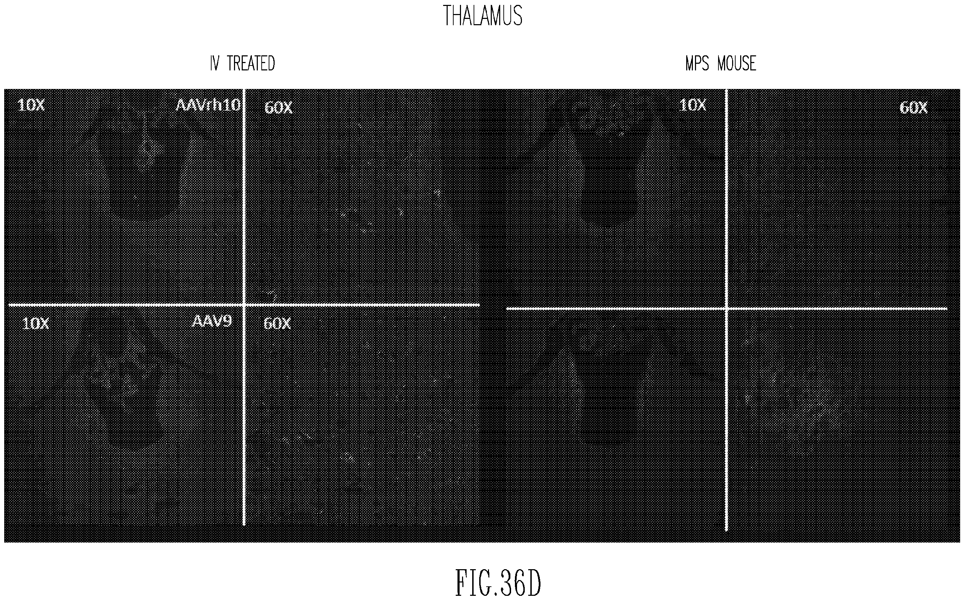

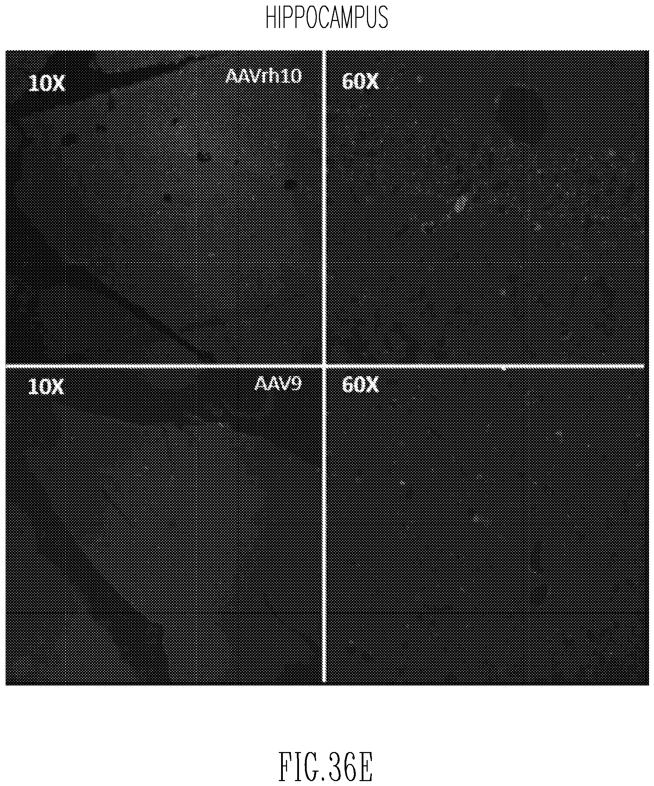

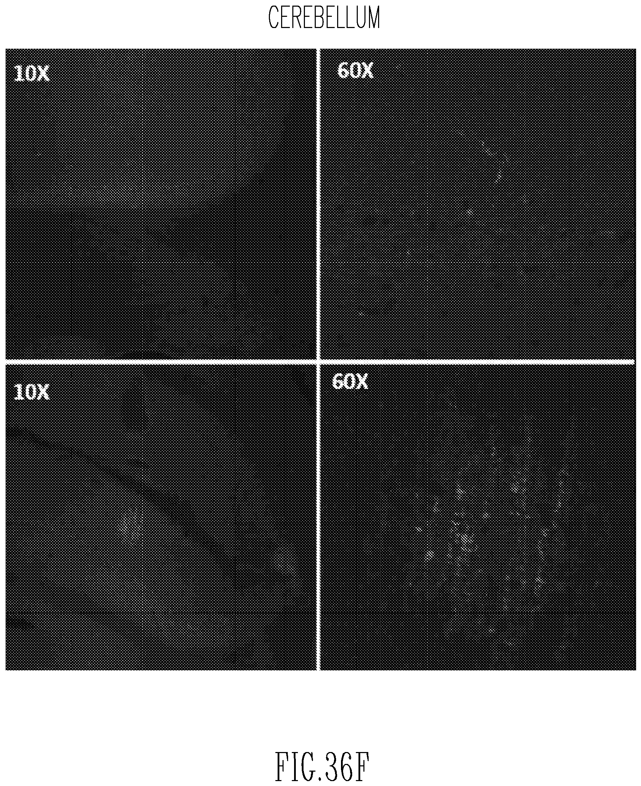

The goal is to improve therapy for severe MPS I by supplementing current ERT and HSCT with IDUA gene transfer to the CNS, thereby preventing neurological manifestations of the disease. In this study, the ability of intravenously administered AAV serotypes 9 and rh10 (AAV9 and AAVrh10) to cross the blood brain barrier for delivery and expression of the IDUA gene in the CNS was tested (Figure 33). 4-5 month old adult MPS I animals were infused intravenously via the tail vein with either an AAV9 or AAVrh10 vector encoding the human IDUA gene. Blood and urine samples were collected on a weekly basis until the animals were sacrificed at 10 weeks post-injection. Plasma IDUA activities in treated animals were close to 1000-fold higher than that of heterozygote controls at 3 weeks post-injection (Figure 34). Brains, spinal cords, and peripheral organs were analyzed for IDUA activity, clearance of GAG accumulation, and IDUA immunofluorescence of tissue sections (Figures 34-36). Treated animals demonstrated widespread restoration of IDUA enzyme activity in all organs including the CNS. These data demonstrate the effectiveness of systemic AAV9 and AAVrh10 vector infusion in counteracting CNS manifestations of MPS I.

### Example VII

Mucopolysaccharidosis type I (MPS I) is a progressive, multisystemic, inherited metabolic disease caused by deficiency of α-L-iduronidase (IDUA). The most severe form of this disease (Hurler syndrome) results in death by age 10. Current treatments for this disease are ineffective in treating CNS disease due to the inability of lysosomal enzymes to traverse the blood-brain barrier. The goal is to supplement current therapy, and treat CNS manifestations of the disease, by AAV-mediated gene delivery and expression of IDUA.

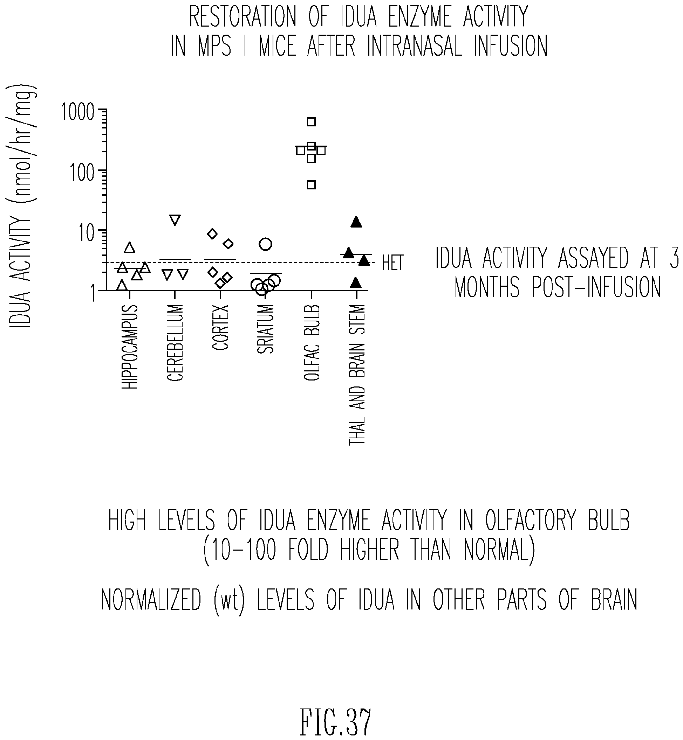

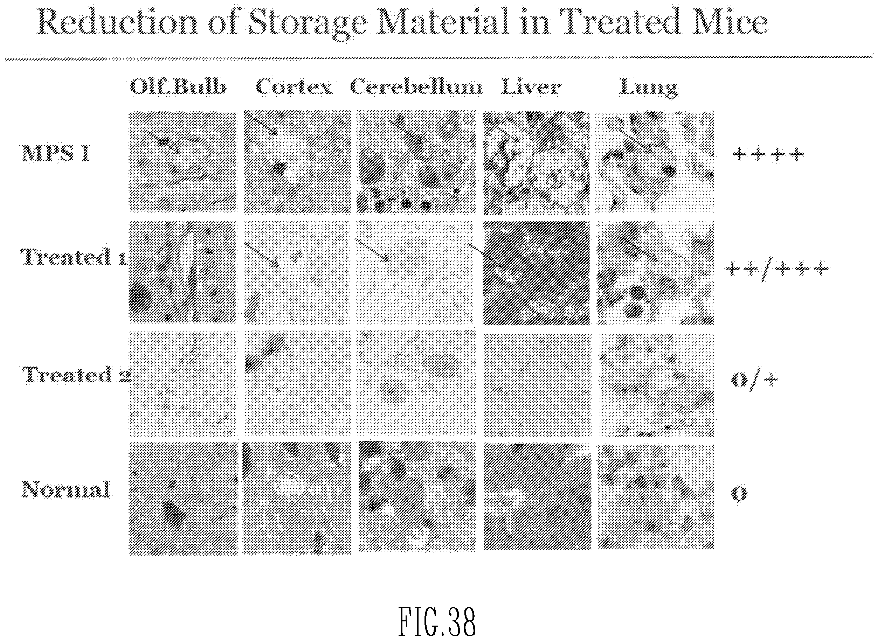

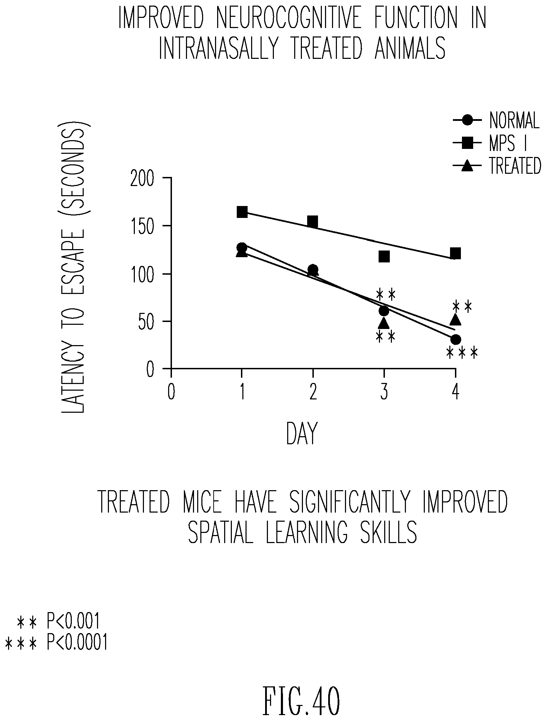

A non-invasive and effective approach to the treatment of CNS disease was taken by intranasal administration of an IDUA-encoding AAV9 vector. Adult IDUA-deficient mice were immunotolerized at birth with human iduronidase, to prevent anti-IDUA immune response, and at 3 months of age were infused intranasally with a CAGS (CMV enhancer/β-actin promoter/globin intron) regulated AAV9-IDUA vector. Animals sacrificed 3 months post-infusion exhibited IDUA enzyme activity levels that were 100-fold that of wild type in the olfactory bulb, with wild type levels of enzyme restored in all other parts of the brain (Figure 37). Intranasal treatment with AAV9-IDUA also resulted in reduction of tissue GAG storage materials in all parts of the brain (Figure 38). Neurocognitive testing using the Barnes maze demonstrated that treated IDUA-deficient mice were not different from normal control animals, while untreated IDUA-deficient mice exhibited a significant learning deficit (Figure 40). Unaffected heterozygote animals exhibited improved performance in this test while MPS I mice displayed a deficit in locating the escape. Remarkably, MPS I mice treated intranasally with AAV9-IDUA exhibited behavior similar to the heterozygote controls, demonstrating prevention of the neurocognitive deficit seen in the untreated MPS I animals (Figure 40D).

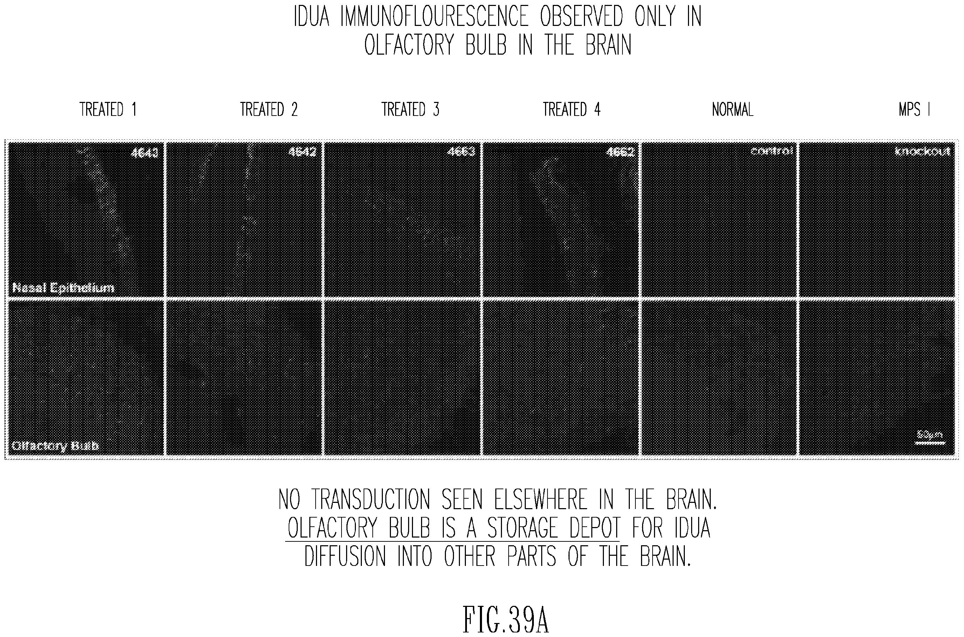

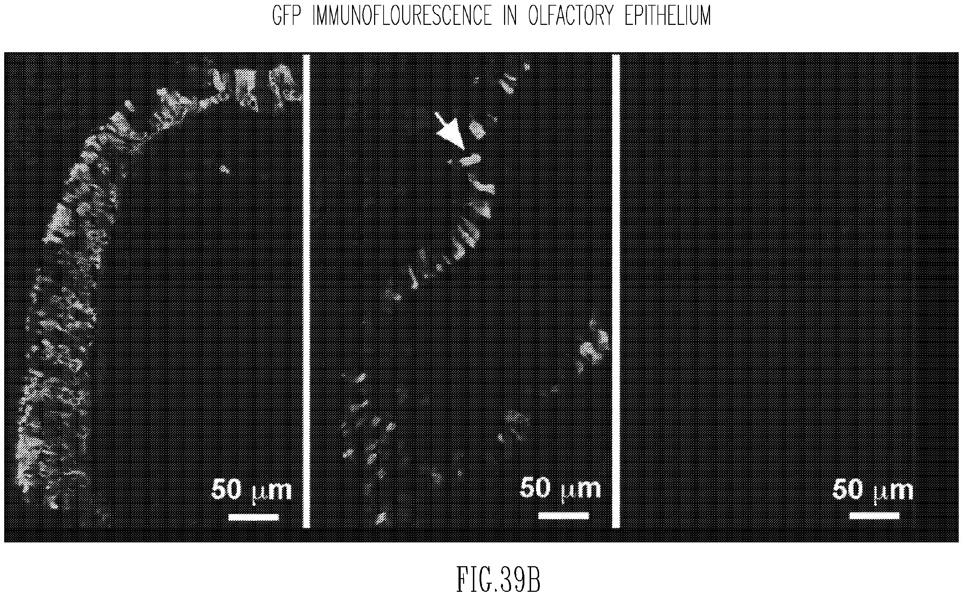

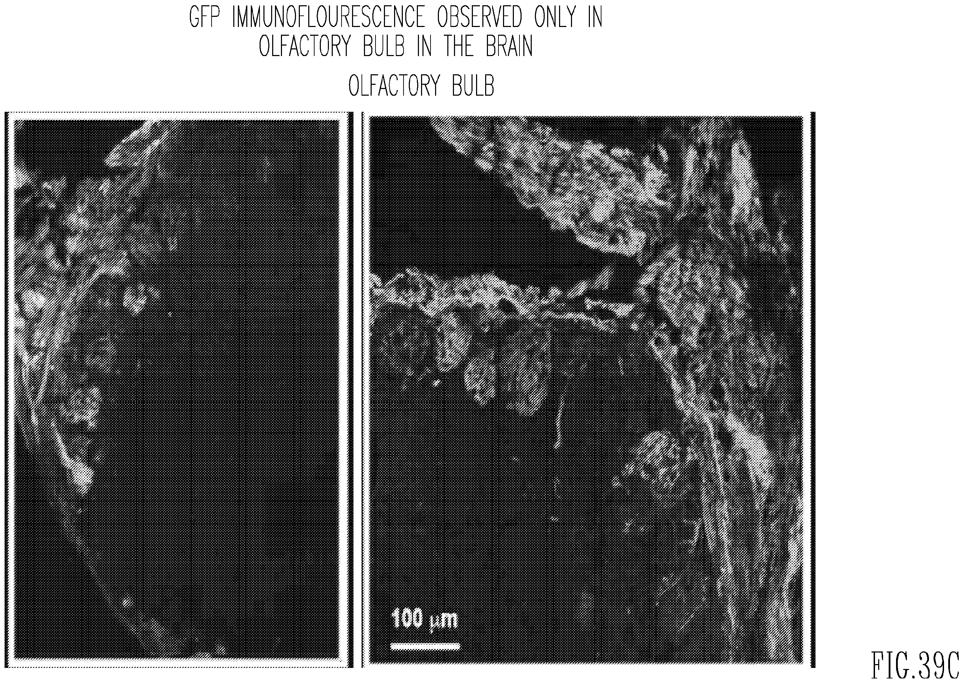

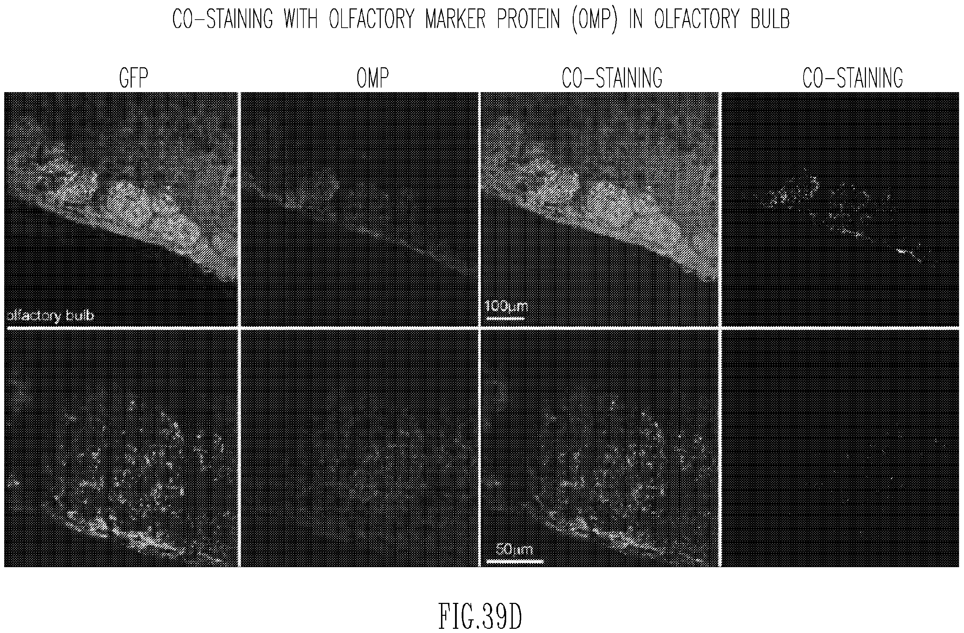

There was strong IDUA immunofluorescence staining observed in tissue sections of the nasal epithelium and olfactory bulb, but no staining was observed in other portions of the brain (Figures 39A-B). This indicates that the widespread distribution of IDUA enzyme most likely was the result of enzyme diffusion from sites of transduction and IDUA expression in the olfactory bulb and the nasal epithelium into deeper areas of the brain. In order to increase access, delivery and vector distribution throughout the brain, IDUA-deficient animals were pretreated with intranasal infusions of an absorption enhancer. At different time points following pretreatment, animals were infused intranasally with AAV9 or AAVrh10 vector encoding IDUA. Animals were sacrificed at 2 months post-infusion, brains microdissected, and assayed for IDUA enzyme activity, clearance of glycosaminoglycans, and immunofluorescence staining for IDUA and GFP. This novel, non-invasive strategy for intranasal AAV9-IDUA administration could potentially be used to treat CNS manifestations of MPS I and other lysosomal diseases.

### Example VIII

Mucopolysaccharidosis type I (MPS I) is an autosomal recessive lysosomal storage disease caused by deficiency of alpha-L-iduronidase (IDUA), resulting in accumulation of glycosaminoglycans. Manifestations of the disease include multi-systemic disorders, and in the severe form of the disease (Hurler syndrome), death by age ten. Currently used treatments, such as enzyme replacement therapy and allogeneic hematopoietic stem cell transplantation, appear to be inefficient for CNS treatment. In this study we have used intrathecal delivery of an adeno-associated virus serotype 9 vector transducing the IDUA gene (AAV9-IDUA) to the CNS in a knock-out mouse model of MPS I. The purpose of this study was to assess the ability of the AAV-mediated gene therapy to prevent the pathological neurochemical changes associated with the MPS I disease.

### Methods

C57BL/6 knock-out mice deficient for IDUA were used as a well-established model of Hurler syndrome. AAV9-IDUA vector was delivered intrathecally to MPS I mice at 12 weeks of age. Prior to AAV administration, the mice were injected with mannitol to open the blood-brain barrier and immunotolerized with laronidase to prevent anti-IDUA immune response. *In vivo* ¹H MR spectra were acquired from the hippocampus and cerebellum of AAV9-IDUA gene treated MPS I mice (MPS I treated, N = 11), untreated MPS I mice (MPS I, N = 12) and heterozygote littermates (control, N = 12) at 9 months of age. ¹H MRS data were acquired at 9.4T using FASTMAP shimming and ultra-short TE STEAM (TE = 2 ms) localization sequence combined with VAPOR water suppression. Metabolites were quantified using LCModel with the spectrum of fast relaxing macromolecules included in the basis set. Spontaneously breathing animals were anesthetized with 1.0 - 1.5% isoflurane.

### Results

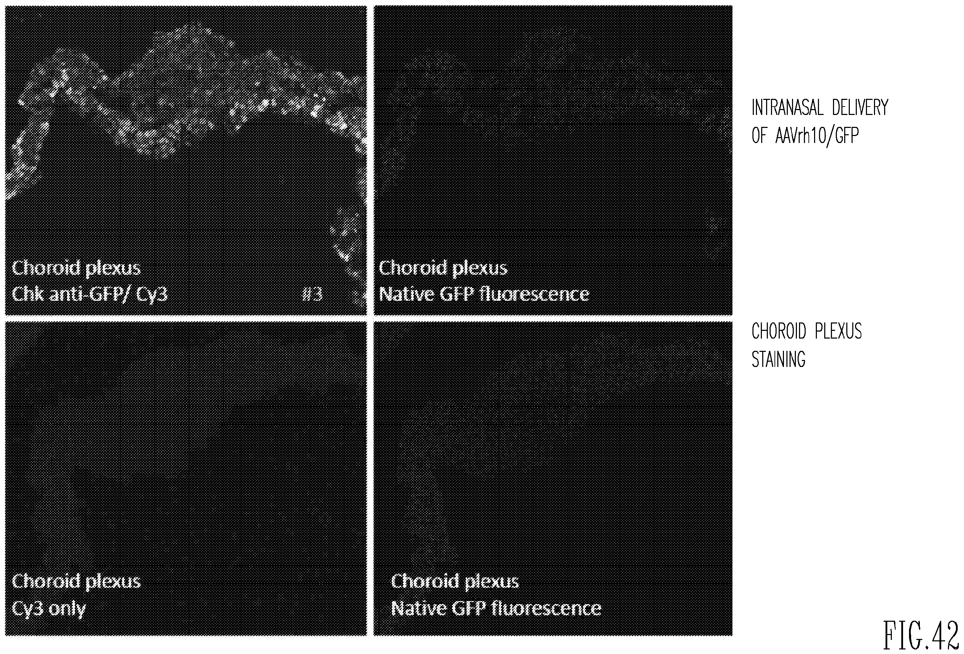

The spectral quality consistently accomplished in this study enabled reliable quantification of fifteen brain metabolites. Small but significant increases in ascorbate (Asc, +0.6 µmol/g, p = 0.003) and N-acetylaspartylglutamate (NAAG, +0.3 mol/g, p = 0.015) concentrations were observed in the hippocampus of untreated MPS I mice relative to controls. In addition, a trend of increased glutathione level (GSH, +0.2 µmol/g, p = 0.054) has been observed. Differences between cerebellar neurochemical profiles of untreated MPS I mice and controls include an increase in NAAG (0.25 µmol/g, p = 0.026) and a decrease in phosphoethanolamine (PE, -0.44 µmol/g, p = 0.04). Neurochemical profiles of MPS I mice treated with AAV9-IDUA showed remarkable similarity to those of control mice (Figures 42A-B). In the hippocampus of treated MPS I mice, the levels of Asc, NAAG and GSH were normalized; only lactate (Lac) showed a small difference relative to control. In the cerebellum of treated MPS I mice, PE but not NAAG level was normalized. Small, but significant differences between treated and control mice were observed for Asc, Lac taurine (Tau) and total creatine (Cr+PCr). Except Asc, changes in metabolite concentrations in treated MPS I mice were always opposite to those observed in the untreated group. In addition, for a number of metabolites that did not show significant changes between untreated MPS I mice and controls (e.g. glucose, glutamate, NAA) it appears that metabolite levels found in treated MPS I mice were closer to controls than to untreated MPS I mice.

### Discussion

Significantly increased concentrations of Asc and a trend for increased GSH in the hippocampus of untreated MPS I mice indicate a protective response against the oxidative stress reported in lysosomal diseases. Whereas decreased PE in the cerebellum and increased NAAG in both brain regions of untreated MPS I may indicate demyelination. A similar pattern of decreased PE and increased NAAG was observed in iron deficiency model where altered myelination was confirmed. The comparison of hippocampal and cerebellar neurochemical profiles of treated MPS I mice against those of untreated MPS I and control mice clearly demonstrates that direct transfer of the missing IDUA gene to the CNS using intrathecal delivery of AAV9 (at 12 weeks of age) prevented neurochemical alternations (at 9 months of age) associated with the neurodegenerative processes in this MPS I mouse model. These neurochemical results are in agreement with similar gene therapy approaches tested in the mouse model of MPS I.

Gene therapy based on direct AAV9-IDUA delivery to the CNS indicates that the oxidative stress and demyelination associate with this mouse model of MPS I can be prevented.

### References

Al-Ghananeem et al., AAPS Pharm. Sci. Tech., 3:E5 (2002).
Bagger et al., Eur. J. Pharm. Sci., 21:235-242 (2004b).
Bagger et al., Int. J. Pharm., 269:311-322 (2004a).
Baker et al., Exp. Brain Res., 63:461 (1986).
Balin et al., J. Comp. Neurol., 251:260-280 (1986).
Banks et al., J. Drug Target, 17:91-97 (2009).
Banks et al., J. Pharmacol. Exp. Ther., 309:469 (2004).
Banks, Biopolymers, 90:589 (2008).
Barakat et al., J. Pharm. Pharmacol., 58:63 (2006).
Barbier et al., Mol. Genet. Metab., 110:303 (2013).
Baumgartner et al., Neuron., 58:639 (2008).
Benedict et al., Neuroendocrinology, 86:136 (2007b).
Benedict et al., Neuropsychopharmacology, 32:239 (2007a).
Benedict et al., Psychoneuroendocrinology, 29:1326 (2004).
Bjoraker et al., J. Dev. Behav. Ped., 27:290 (2006).
Blits et al., J. Neuros. Methods, 185:257 (2010).
Born et al., Nat. Neurosci., 5:514 (2002).
Boulton et al., Am. J. Physiol., 276:R818 (1999).
Boulton et al., Neuropathol. Appl. Neurobiol., 22:325 (1996).
Bradbury et al., Am. J. Physiol., 240:F329 (1981).
Bradbury et al., J. Physiol., 339:519 (1983).
Brady, Ann. Rev. Med., 57:283 (2006).
Broadwell et al., J. Comp. Neurol., 242:632 (1985).
Broekman et al., Neuroscience, 138:501 (2006).
Buck, In: Kandel ER, Schwartz JH, Jessell TM, editors. Principles of neural science. 4th edition. New York: McGraw-Hill Companies. pp. 625-652 (2000).
Buxer et al., J. Neurochem., 56:1012 (1991).
Cai et al., Sichuan Da Xue Xue Bao Yi Xue Ban, 39:438 (2008).
Campos et al., Meta. Brain Dis., 27:121 (2012).
Capsoni et al., Proc. Natl. Acad. Sci. USA, 99:12432 (2002).
Carare et al., Neuropathol. Appl. Neurobiol., 34:131 (2008).
Cauna, In: Proctor DF, Andersen I, editors. Amsterdam: Elsevier Biomedical Press. pp. 45-69 (1982).
Charlton et al., Int. J. Pharm., 338:94 (2007b).
Charlton et al., J. Drug Target, 15:370 (2007a).
Charlton et al., Pharm. Res., 25:1531 (2008).
Chen et al., J. Alzheimers Dis., 1:35 (1998).
Chen et al., J. Pharm. Sci., 95:1364 (2006).
Chow et al., J. Pharm. Sci., 88:754 (1999).
Chow et al., J. Pharm. Sci., 90:1729 (2001).
Clerico et al., In: Doty RL, editor. Handbook of olfaction and gustation. 2nd edition. New York: Marcel
Dekker, Inc. pp. 1-16 (2003).\Campos et al., Meta. Brain Dis., 27:121 (2012).
Costantino et al., Int. J. Pharm., 337:1 (2007).
Cserr et al., Am. J. Physiol., 240:F319 (1981).
Cserr et al., Brain Pathol., 2:269 (1992).
Dahlin et al., Eur. J. Pharm. Sci., 14:75 (2001).
Danhof et al., American Association of Pharmaceutical Scientists Annual Meeting, Atlanta, GA (2008).
Danielyan et al., Eur. J. Cell. Biol., 88:315 (2009).
Davis et al., Clin. Pharmacokinet., 42:1107 (2003).
de Lorenzo, In: Wolstenholme GEW, Knight J, editors. Taste and smell in vertebrates. London: Churchill. pp. 151-175 (1970).
De Rosa et al., Proc. Natl. Acad. Sci. USA, 102:3811 (2005).
DeSesso, Qual. Assur., 2:213 (1993).
Desmanis et al., Ann. Neurol., 56:68 (2004).
deSouza et al., Eur. Neuropsychopharmacol., 19:53 (2009).
Dhanda et al., Drug Del. Tech., 5:64 (2005).
Dhuria et al., J. Pharm. Sci., 98:2501 (2009b).
Dhuria et al., J. Pharmaceutical Sciences, 99:1654 (2010).
Dhuria et al., J. Pharmacol. Exp. Ther., 328:312 (2009a).
Diano et al., J. Clin. Invest., 118:26 (2008).
Dickson et al., Mol. Gen. Metab., 91:61 (2007).
Djupesland et al., Laryngoscope, 116:466 (2006).
Domes et al., Biol. Psychiatry, 61:731 (2007b).
Domes et al., Biol. Psychiatry, 62:1187 (2007a).
Dufes et al., Int. J. Pharm., 255:87 (2003).
Einer-Jensen et al., Exp. Brain Res., 130:216 (2000b).
Einer-Jensen et al., Pharmacol. Toxicol., 87:276 (2000a).
Einer-Jensen et al., Reproduction, 129:9 (2005).
Ellinwood et al., Mol. Genet. Metab., 91:239 (2007).
Fehm et al., J. Clin. Endocrinol. Metab., 86:1144 (2001).
Field et al., J. Neurocytol., 32:317 (2003).
Fliedner et al., Endocrinology., 17:2088 (2006).
Foust et al., Nat. Biotech., 27:5 (2009)).
Francis et al., Brain, 131:3311 (2008).
Fratantoni et al., Science, 162:570 (1968).
Frey et al., Drug Delivery, 4:87 (1997).
Frey II, Drug Del. Tech., 2:46 (2002).
Fuss et al., Eur. J. Neurosci., 22:2649 (2005).
Gao et al., Biomaterials, 27:3482 (2006).
Gao et al., Int. J. Pharm., 340:207 (2007a).
Gao et al., J. Control Release, 121:156 (2007b).
Gopinath et al., Current Ther. Res., 23:596 (1978).
Gozes et al., Curr. Alzheimer Res., 4:507 (2007).
Graff et al., Pharm. Res., 20:1225 (2003).
Graff et al., Pharm. Res., 22:235 (2005a).
Graff et al., Pharm. Res., 22:86 (2005b).
Gray, 15th revised edition (Classic Collectors edition). New York: Bounty Books (1978).
Grevers et al., Arch. Otorhinolaryngol., 244:55 (1987).
Groothuis et al., J. Cereb. Blood Flow Metab., 27:43 (2007).
Gross et al., J. Anat., 135:83 (1982).
Guastella et al., Biol. Psychiatry, 63:3 (2008).
Hadaczek et al., Mol. Ther., 14:69 (2006).
Hallschmid et al., Requl. Pept., 149:79 (2008).
Han et al., J. Mol. Med., 85:75 (2007).
Hanson et al., BMC Neurosci., 9:S5 (2008).
Hanson et al., Drug Del. Tech., 4:66 (2004).
Hanson et al., San Diego, CA: Society for Neuroscience (2007).
Hanson et al., In: EPO, editor. Biopharm and HealthPartners Research Foundation (2008).
Hartung et al., J. Am. Soc. Gene Ther., 9:866 (2004).
Hartung et al., Mol. Thera., 9:869 (2004).
Hashizume et al., Neuro. Oncol., 10:112 (2008).
Hatterer et al., Blood, 107:806 (2006).
Herati et al., J. Gene Med., 10:972 (2008).
Hess et al., Exp. Neuro., 186:134 (2004).
Horvat et al., Eur. J. Pharm. Biopharm., 72:252 (2009).
Hussar et al., Chem. Senses, 27:7 (2002).
Ilium, J. Control Release, 87:187 (2003).
Ilium, J. Pharm. Pharmacol., 56:3 (2004).
Itaya et al., Brain Res., 398:397 (1986).
Jansson et al., J. Drug Target, 10:379 (2002).
Jogani et al., Alzheimer Dis. Assoc. Disord., 22:116 (2008).
Johnston et al., Cerebrospinal Fluid Res., 1:2 (2004).
Kakkis et al., Mol. Gen. Met., 83:163 (2004).
Kandimalla et al., J. Pharm. Sci., 94:613 (2005b).
Kandimalla et al., Pharm. Res., 22:1121 (2005a).
Kida et al., Neuropathol. Appl. Neurobiol., 19:480 (1993).
Kirsch et al., J. Neurosci., 25:11489 (2005).
Klein et al., J. Am. Soc. Gene Ther., 13:517 (2006).
Koos et al., Neuroreport, 16:1929 (2005).
Kosfeld et al., Nature, 435:673 (2005).
Kristensson et al., Acta Neuropathol (Berl), 19:145 (1971).
Krivit, Springer Seminars in Immunopathology, 26:119 (2004).
Kumar et al., Curr. Sci., 43:435 (1974).
Kumar et al., Int. J. Pharm., 358:285 (2008).
Li et al., Chin. J. Physiol., 48:7 (2005c).
Li et al., Glia, 52:245 (2005a).
Li et al., J. Neurocytol., 34:343 (2005b).
Loftus et al., Neuroscience, 139:1061 (2006).
Luzzati et al., J. Mol. Biol., 343:199 (2004).
Mackay-Sim, In: Doty RL, editor. Handbook of olfaction and gustation. 2nd edition. New York: Marcel Dekker, Inc. pp. 93-113 (2003).
Martinez et al., Neuroscience, 157:908 (2008).
Minn et al., J. Drug Target, 10:285 (2002).
Miragall et al., J. Comp. Neurol., 341:433 (1994).
Muenzer, J. Pediatrics, 144:S27 (2004).
Munoz-Rojas et al., Am. J. Med. Gen., 146A:2538 (2008).
Neufeld and Muenzer, In A. L. B. C.R. Scriver, W.S. Sly, et al (ed.), McGraw Hill, NY, pg. 3421 (2001).
Nonaka et al., J. Pharmacol. Exp. Ther., 325:513 (2008).
Ohlfest et al., Blood, 105:2691 (2005).
Orchard et al., J. Pediatrics, 151:340 (2007).
Owens et al., Diabet. Med., 20:886 (2003).
Pan et al., Brain Res., 1188:241 (2008).
Pardridge, NeuroRx, 2:3 (2005).
Parker et al., Psychoneuroendocrinology, 30:924 (2005).
Pastores, Exp. Opin. Biol. Ther., 8:1003 (2008).
Pereira et al., Clin. Chim. Acta:lnt. J. Clin. Chem., 387:75 (2008).
Perl et al., Lancet, 1:1028 (1987).
Peters et al., Bone Marrow Transpl., 31:229 (2003).
Pollock et al., J. Anat., 191:337 (1997).
Raghavan et al., J. Laryngol. Otol., 114:456 (2000).
Rao et al., Ped. Res., 73:31 (2013).
Reger et al., J. Alzheimers Dis., 13:323 (2008a).
Reger et al., Neurobiol. Aging, 27:451 (2006).
Reger et al., Neurology, 70:440 (2008b).
Rennels et al., Adv. Neurol., 52:431 (1990).
Rennels et al., Brain Res., 326:47 (1985).
Reolon et al., Brain Res., 1076:225 (2006).
Reolon et al., Cell. Mol. Neurobiol., 29:443 (2009).
Rimmele et al., J. Neurosci., 29:38 (2009).
Ross et al., J. Neuroimmunol., 151:66 (2004).
Ross et al., Neurosci. Lett., 439: 30 (2008).
Sakane et al., J. Pharm. Pharmacol., 46:378 (1994).
Sakane et al., J. Pharm. Pharmacol., 47:379 (1995).
Sarkar, Pharm. Res., 9:1 (1992).
Schaefer et al., J. Comp. Neurol., 444:221 (2002).
Scheibe et al., Arch. Otolaryngol. Head Neck Surg., 134:643 (2008).
Schley et al., J. Theor. Biol., 238:962 (2006).
Schulz et al., Endocrinology, 145:2696 (2004).
Schuster et al., Frontiers in Neuroanatomy, 8:42 (2014).
Scott et al., Am. J. Hum. Genet., 53:973 (1993).
Shimizu et al., Int. J. Obes. (Lond), 29:858 (2005).
Shipley, Brain Res. Bull., 15:129 (1985).
Skipor et al., Reprod. Biol., 3:143 (2003).
Steen et al., J. Alzheimers Dis., 7:63 (2005).
Stefanczyk-Krzymowska et al., Exp. Physiol., 85:801 (2000).
Takano et al., J. Histochem. Cytochem., 53:611 (2005).
Thorne et al., Brain Res., 692:278 (1995).
Thorne et al., Clin. Pharmacokinet., 40:907 (2001).
Thorne et al., Neuroscience, 127:481 (2004).
Thorne et al., Neuroscience, 152:785 (2008).
Thorne, RG. 2002. The nasal pathways for drug delivery to the central nervous system: Studies with protein tracers and therapeutics. Doctoral Dissertation, University of Minnesota.
Tkac et al., Mag. Res. In Med., 41:649 (1999).
Tkac et al., Mag. Res. In Med., 50:24 (2003).
Unger et al., J. Neuropath. Exp. Neuro., 52:460 (1993).
van den Berg et al., Eur. J. Pharm. Biopharm., 58:131 (2004b).
Van den Berg et al., J. Drug Target, 11:325 (2003).
van den Berg et al., J. Neurosci. Methods, 116:99 (2002).
van den Berg et al., Pharm. Res., 21:799 (2004a).
Van Diest et al., J. Anat., 128:293 (1979).
Vyas et al., AAPS PharmSciTech., 7:E1 (2006c).
Vyas et al., Crit. Rev. Ther. Drug Carrier Syst., 23:319 (2006b).
Vyas et al., J. Drug Target, 13:317 (2005).
Vyas et al., J. Pharm. Sci., 95:570 (2006a).
Walter et al., Arch. Histol. Cytol., 69:37 (2006a).
Walter et al., Neuropathol. Appl. Neurobiol., 32:388 (2006b).
Wang et al., Cancer Chemother. Pharmacol., 57:97 (2006a).
Wang et al., Eur. J. Pharm. Biopharm., 70:735 (2008).
Wang et al., Int. J. Pharm., 317:40 (2006b).
Wang et al., Int. J. Pharm., 341:20 (2007).
Watson et al., Gene Therapy, 16 February 2006, doi:10.1038/sj.gt.3302735.
Weller et al., Neurol. Res., 25:611 (2003).
Westin et al., Eur. J. Pharm. Sci., 24:565 (2005).
Westin et al., Pharm. Res., 23:565 (2006).
Williams et al., J. Comp. Neurol., 470:50 (2004).
Wioland et al., J. Histochem. Cytochem., 48:1215 (2000).
Wolf et al., Neurobio. Dis., 43:123 (2011).
Wolf et al., Neurology, 62:1503 (2004)
Xu et al., J. Clin. Invest., 118:272 (2008).
Yamada et al., Am. J. Physiol., 261:H1197 (1991).
Yang et al., J. Pharm. Sci., 94:1577 (2005).
Zhang et al., Acta Neuropathol. (Berl), 83:233 (1992).
Zhang et al., Acta Pharmacol. Sin., 25:522 (2004a).
Zhang et al., Int. J. Pharm., 275:85 (2004b).
Zhang et al., J. Drug Target, 14:281 (2006).
Zhao et al., Acta Pharmacol. Sin., 28:273 (2007).
Zhao et al., Chin. Med. Sci. J., 19:257 (2004).
Zheng et al., Mol. Genet. Metab., 79:233 (2003).
Ziegler and Shapiro, In J. Donders and S. Hunter (ed.), Cambridge University Press, p. 427 (2007).

## Claims

1. A composition comprising an effective amount of a recombinant adeno-associated virus (rAAV) vector comprising an open reading frame encoding iduronate-2-sulfatase, for use in a method to enhance neurocognition in a mammal having a mucopolysaccharidosis type II (MPSII) disorder relative to a mammal with MPSII that is not administered the rAAV, wherein the rAAV is rAAV9 or rAAVrh10.

2. The composition for use according to claim 1 wherein the mammal is not treated with an immunosuppressant.

3. The composition for use according to claim 1 or 2 wherein the mammal is treated with an immunosuppressant.

4. The composition for use according to claim 3 wherein the immune suppressant comprises a glucocorticoid, cytostatic agents including an alkylating agent, an anti-metabolite, a cytotoxic antibiotic, an antibody, an agent active on immunophilin, cyclophosphamide, a nitrogen mustard, nitrosourea, platinum compound, methotrexate, azathioprine, mercaptopurine, fluorouracil, dactinomycin, an anthracycline, mitomycin C, bleomycin, mithramycin, IL-2 receptor- (CD25-) or CD3-directed antibodies, anti-IL-2 antibodies, ciclosporin, tacrolimus, sirolimus, IFN-β, IFN-γ, an opioid, or TNF-α (tumor necrosis factor-alpha) binding agent.

5. The composition for use according to claim 4 wherein the rAAV and the immune suppressant are co-administered or the immune suppressant is administered after the rAAV.

6. The composition for use according to any one of claims 1 to 5 wherein the mammal is not immunotolerized prior to administration of rAAV.

7. The composition for use according to any one of claims 1 to 5 wherein the mammal is immunotolerized prior to administration of rAAV.

8. The composition for use according to claim 1 wherein the mammal is a human or an immunocompetent adult.

9. The composition for use according to any one of claims 1 to 8 wherein the rAAV vector is a rAAV9 vector.

10. The composition for use according to any one of claims 1 to 9 wherein multiple doses are administered or wherein the composition is administered weekly.

11. The composition for use according to any one of claims 1 to 10 wherein the amount inhibits growth delay, inhibits hepatospenomegaly, inhibits cardiopulmonary disease, or inhibits skeletal dysplasia, or any combination thereof.

12. The composition for use according to any one of claims 1 to 11 wherein the rAAV is rAAVrh10.

## Patentansprüche

1. Zusammensetzung, die eine wirksame Menge eines rekombinanten Adeno-assoziierten Virus (rAAV)-Vektors umfasst, der einen offenen Leserahmen umfasst, der für Iduronat-2-Sulfatase codiert, zur Verwendung in einem Verfahren, um eine Neurokognition bei einem Säugetier, das eine Mukopolysaccharidose Typ II (MPSII) Störung aufweist, relativ zu einem Säugetier mit MPSII, dem nicht das rAAV verabreicht wird, zu verbessern, wobei das rAAV rAAV9 oder rAAVrh10 ist.

2. Zusammensetzung zur Verwendung nach Anspruch 1, wobei das Säugetier nicht mit einem Immunsuppressivum behandelt wird.

3. Zusammensetzung zur Verwendung nach Anspruch 1 oder 2, wobei das Säugetier mit einem Immunsuppressivum behandelt wird.

4. Zusammensetzung zur Verwendung nach Anspruch 3, wobei das Immunsuppressivum Folgendes umfasst: ein Glukokortikoid, Zytostatika, einschließlich eines Alkylierungsmittels, ein Antimetabolit, ein zytotoxisches Antibiotikum, einen Antikörper, ein gegen Immunophilin aktives Mittel, Cyclophosphamid, einen Stickstoffsenf, Nitrosoharnstoff, Platinverbindung, Methotrexat, Azathioprin, Mercaptopurin, Fluorouracil, Dactinomycin, ein Anthracyclin, Mitomycin C, Bleomycin, Mithramycin, IL-2-Rezeptor-(CD25-) oder CD3-gerichtete Antikörper, Anti-IL-2-Antikörper, Ciclosporin, Tacrolimus, Sirolimus, IFN-ß, IFN-γ, ein Opioid oder TNF-α (Tumornekrosefaktor-alpha) bindendes Mittel.

5. Zusammensetzung zur Verwendung nach Anspruch 4, wobei das rAAV und das Immunsuppressivum gemeinsam verabreicht werden oder das Immunsuppressivum nach dem rAAV verabreicht wird.

6. Zusammensetzung zur Verwendung nach einem der Ansprüche 1 bis 5, wobei das Säugetier vor der Verabreichung von rAAV nicht immuntolerant ist.

7. Zusammensetzung zur Verwendung nach einem der Ansprüche 1 bis 5, wobei das Säugetier vor der Verabreichung von rAAV immuntolerant ist.

8. Zusammensetzung zur Verwendung nach Anspruch 1, wobei das Säugetier ein Mensch oder ein immunkompetenter Erwachsener ist.

9. Zusammensetzung zur Verwendung nach einem der Ansprüche 1 bis 8, wobei der rAAV-Vektor ein rAAV9-Vektor ist.

10. Zusammensetzung zur Verwendung nach einem der Ansprüche 1 bis 9, wobei mehrere Dosen verabreicht werden oder wobei die Zusammensetzung wöchentlich verabreicht wird.

11. Zusammensetzung zur Verwendung nach einem der Ansprüche 1 bis 10, wobei die Menge eine Wachstumsverzögerung hemmt, eine Hepatosplenomegalie hemmt, eine kardiopulmonale Erkrankung hemmt oder eine Skelettdysplasie hemmt, oder eine beliebige Kombination davon.

12. Zusammensetzung zur Verwendung nach einem der Ansprüche 1 bis 11, wobei das rAAV rAAVrh10 ist.

## Revendications

1. Composition comprenant une quantité efficace d'un vecteur de virus adéno-associé recombinant (rAAV) comprenant un cadre de lecture ouvert codant l'iduronate-2-sulfatase, à utiliser dans un procédé pour améliorer la neurocognition chez un mammifère atteint d'un trouble de mucopolysaccharidose de type II (MPSII) concernant un mammifère atteint de MPSII à qui le rAAV n'est pas administré, le rAAV étant rAAV9 ou rAAVrh10.

2. Composition destinée à être utilisée selon la revendication 1, dans laquelle le mammifère n'est pas traité avec un immunosuppresseur.

3. Composition destinée à être utilisée selon la revendication 1 ou 2, dans laquelle le mammifère est traité avec un immunosuppresseur.

4. Composition destinée à être utilisée selon la revendication 3, dans laquelle le suppresseur immunitaire comprend un glucocorticoïde, des agents cytostatiques comportant un agent alkylant, un anti-métabolite, un antibiotique cytotoxique, un anticorps, un agent actif sur l'immunophiline, le cyclophosphamide, une moutarde azotée, la nitrosourée, un composé de platine, le méthotrexate, l'azathioprine, la mercaptopurine, le fluorouracile, la dactinomycine, l'anthracycline, la mitomycine C, la bléomycine, la mithramycine, des anticorps dirigés contre les récepteurs de I'IL-2 (CD25-) ou CD3, des anticorps anti-IL-2, la ciclosporine, le tacrolimus, le sirolimus, I'IFN-β, I'IFN-γ, un opioïde ou un agent de liaison au TNF-α (facteur de nécrose tumorale alpha).

5. Composition destinée à être utilisée selon la revendication 4, dans laquelle le rAAV et le suppresseur immunitaire sont co-administrés ou le suppresseur immunitaire est administré après le rAAV.

6. Composition destinée à être utilisée selon l'une quelconque des revendications 1 à 5, dans laquelle le mammifère n'est pas immunotolérisé avant l'administration de rAAV.

7. Composition destinée à être utilisée selon l'une quelconque des revendications 1 à 5, dans laquelle le mammifère est immunotolérisé avant l'administration de rAAV.

8. Composition destinée à être utilisée selon la revendication 1, dans laquelle le mammifère est un être humain ou un adulte immunocompétent.

9. Composition destinée à être utilisée selon l'une quelconque des revendications 1 à 8, dans laquelle le vecteur rAAV est un vecteur rAAV9.

10. Composition destinée à être utilisée selon l'une quelconque des revendications 1 à 9, dans laquelle des doses multiples sont administrées ou la composition est administrée de façon hebdomadaire.

11. Composition destinée à être utilisée selon l'une quelconque des revendications 1 à 10, dans laquelle la quantité inhibe un retard de croissance, inhibe l'hépatospénomégalie, inhibe une maladie cardiopulmonaire ou inhibe la dysplasie squelettique, ou toute combinaison de celles-ci.

12. Composition destinée à être utilisée selon l'une quelconque des revendications 1 à 11, dans laquelle le rAAV est rAAVrh10.
